# EUROPÄISCHE PATENTSCHRIFT

(11) **EP 2 435 428 B1**
(45) Veröffentlichungstag und Bekanntmachung des Hinweises auf die Patenterteilung: **31.07.2013**
(21) Anmeldenummer: 10721991.7
(22) Anmeldetag: 14.05.2010
(51) Int. Cl.: C07D 413/06, C07D 417/06, C07D 211/20, C07D 211/22, C07D 211/24, C07D 211/26, C07D 211/34, C07D 401/06

(54) **SUBSTITUIERTE PIPERIDINE**
SUBSTITUTED PIPERIDINES
PIPÉRIDINES SUBSTITUÉES

(30) Priorität: 27.05.2009 DE 102009022892
(43) Veröffentlichungstag der Anmeldung: 04.04.2012
(73) Patentinhaber: Bayer Intellectual Property GmbH, 40789 Monheim (DE)
(72) Erfinder: HEIMBACH, Dirk, 40549 Düsseldorf (DE); RÖHRIG, Susanne, 40724 Hilden (DE); CANCHO GRANDE, Yolanda, 51373 Leverkusen (DE); RESTER, Ulrich, 42115 Wuppertal (DE); BENDER, Eckhard, 40764 Langenfeld (DE); ZIMMERMANN, Katja, 40489 Düsseldorf (DE); ZUBOV, Dmitry, 42857 Remscheid (DE); BUCHMÜLLER, Anja, 45259 Essen (DE); VON DEGENFELD, Georges, 51373 Leverkusen (DE); GERDES, Christoph, 51063 Köln (DE); GNOTH, Mark, Jean, 40822 Mettmann (DE); GERICKE, Kersten, Matthias, 42115 Wuppertal (DE); JESKE, Mario, 42699 Solingen (DE)
(74) Vertreter: BIP Patents
(86) Internationale Anmeldenummer: PCT/EP2010/002967
(87) Internationale Veröffentlichungsnummer: WO 2010/136128

(56) Entgegenhaltungen:
- MCATEE J.J. ET AL.: "Development of potent and selective small-molecule human Urotensin-II antagonists" BIOORGANIC AND MEDICINAL CHEMISTRY LETTERS, Bd. 18, 2008, Seiten 3500-3503, XP002595937

## Beschreibung

Die Erfindung betrifft neue substituierte Piperidine, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Herz-Kreislauf-Erkrankungen und von Tumorerkrankungen.

Thrombozyten (Blutplättchen) sind ein wesentlicher Faktor sowohl in der physiologischen Blutstillung (Hämostase) als auch bei thromboembolischen Erkrankungen. Insbesondere im arteriellen System kommt Thrombozyten eine zentrale Bedeutung in der komplexen Interaktion zwischen Blutkomponenten und Gefäßwand zu. Unerwünschte Thrombozytenaktivierung kann durch Bildung plättchenreicher Thromben zu thromboembolischen Erkrankungen und thrombotischen Komplikationen mit lebensbedrohlichen Zuständen führen.

Einer der potentesten Plättchenaktivatoren ist die Blutgerinnungsprotease Thrombin, die an verletzten Blutgefäßwänden gebildet wird und neben der Fibrinbildung zur Aktivierung von Thrombozyten, Endothelzellen und mesenchymalen Zellen führt (Vu TKH, Hung DT, Wheaton VI, Coughlin SR, Cell 1991, 64, 1057-1068). An Thrombozyten *in vitro* und in Tiermodellen hemmen Thrombin-Inhibitoren die Plättchenaggregation bzw. die Bildung plättchenreicher Thromben. Beim Menschen können arterielle Thrombosen erfolgreich mit Inhibitoren der Thrombozytenfunktion sowie Thrombin-Inhibitoren verhindert oder behandelt werden (Bhatt DL, Topol EJ, Nat. Rev. Drug Discov. 2003, 2, 15-28). Deshalb besteht eine hohe Wahrscheinlichkeit, dass Antagonisten der Thrombinwirkung auf Blutplättchen die Bildung von Thromben und das Auftreten von klinischen Folgen wie Herzinfarkt und Schlaganfall vermindern. Weitere zelluläre Thrombinwirkungen, z.B. auf Gefäßendothel- und -glattmuskelzellen, Leukozyten und Fibroblasten, sind möglicherweise für entzündliche und proliferative Erkrankungen verantwortlich.

Die zellulären Effekte von Thrombin werden zumindest teilweise über eine Familie G-Proteingekoppelter Rezeptoren (Protease Activated Receptors, PARs) vermittelt, deren Prototyp der PAR-1-Rezeptor darstellt. PAR-1 wird durch Bindung von Thrombin und proteolytische Spaltung seines extrazellulär liegenden N-Terminus aktiviert. Durch die Proteolyse wird ein neuer N-Terminus mit der Aminosäurensequenz SFLLRN... freigelegt, der als Agonist ("Tethered Ligand") zur intramolekularen Rezeptoraktivierung und Übertragung intrazellulärer Signale führt. Von der Tethered-Ligand Sequenz abgeleitete Peptide können als Agonisten des Rezeptors eingesetzt werden und führen auf Thrombozyten zur Aktivierung und Aggregation. Andere Proteasen sind ebenfalls in der Lage, PAR-1 zu aktivieren, hierunter z.B. Plasmin, Faktor VIIa, Faktor Xa, Trypsin, aktiviertes Protein C (aPC), Tryptase, Cathepsin G, Proteinase 3, Granzyme A, Elastase und Matrixmetalloprotease 1 (MMP-1).

Im Gegensatz zur Inhibition der Proteaseaktivität von Thrombin mit direkten Thrombin-Inhibitoren sollte eine Blockade des PAR-1 zur Hemmung der Thrombozytenaktivierung ohne Verminderung der Gerinnungsfähigkeit des Blutes (Antikoagulation) führen.

Antikörper und andere selektive PAR-1-Antagonisten hemmen die Thrombin-induzierte Aggregation von Thrombozyten *in vitro* bei niedrigen bis mittleren Thrombinkonzentrationen (Kahn ML, Nakanishi-Matsui M, Shapiro MJ, Ishihara H, Coughlin SR, J. Clin. Invest. 1999, 103, 879-887). Ein weiterer Thrombinrezeptor mit möglicher Bedeutung für die Pathophysiologie thrombotischer Prozesse, PAR-4, wurde auf humanen und tierischen Thrombozyten identifiziert. In experimentellen Thrombosen an Tieren mit einem dem Menschen vergleichbaren PAR-Expressionsmuster reduzieren PAR-1-Antagonisten die Bildung plättchenreicher Thromben (Derian CK, Damiano BP, Addo MF, Darrow AL, D'Andrea MR, Nedelman M, Zhang H-C, Maryanoff BE, Andrade-Gordon P, J. Pharmacol. Exp. Ther. 2003, 304, 855-861).

In den letzten Jahren wurde eine Vielzahl von Substanzen auf ihre plättchenfunktionshemmende Wirkung geprüft, in der Praxis haben sich aber nur wenige Plättchenfunktionshemmer bewährt. Es besteht daher ein Bedarf an Pharmazeutika, die spezifisch eine gesteigerte Plättchenreaktion hemmen ohne das Blutungsrisiko erheblich zu erhöhen und damit das Risiko von thromboembolischen Komplikationen vermindern.

Effekte von Thrombin, die über den Rezeptor PAR-1 vermittelt werden, haben Auswirkungen auf den Krankheitsverlauf während und nach operativer koronarer Bypassanlage (CABG) sowie anderer Operationen und insbesondere Operationen mit extrakorporalem Kreislauf (z. B. Herz-Lungenmaschine). Im Verlauf der Operation kann es aufgrund der prä- oder intraoperativen Medikation mit gerinnungshemmenden und/oder plättchenhemmenden Substanzen zu Blutungskomplikationen kommen. Aus diesem Grunde muss beispielsweise eine Medikation mit Clopidogrel mehrere Tage vor einer CABG pausiert werden. Außerdem kann es wie erwähnt (z.B. aufgrund des ausgedehnten Kontaktes zwischen Blut und künstlichen Oberflächen bei Einsatz einer extrakorporalen Zirkulation oder bei Bluttransfusionen) zur Ausbildung einer disseminierten intravaskulären Gerinnung oder Verbrauchskoagulopathie (DIC) kommen, die sekundär zu Blutungskomplikationen führen kann. Im weiteren Verlauf kommt es häufig zur Restenose der angelegten venösen oder arteriellen Bypässe (bis hin zum Verschluß) aufgrund von Thrombose, Intimafibrose, Arteriosklerose, Angina pectoris, Myokardinfarkt, Herzinsuffizienz, Arrhythmien, transitorische ischämische Attacke (TIA) und/oder Schlaganfall.

Der Rezeptor PAR-1 wird im Menschen auch auf anderen Zellen exprimiert, hierunter z.B. Endothelzellen, glatte Gefäßmuskelzellen und Tumorzellen. Bösartige Tumorerkrankungen (Krebs) haben eine hohe Inzidenz und sind im Allgemeinen mit einer hohen Sterblichkeit verbunden. Gegenwärtige Therapien erreichen nur in einem Bruchteil der Patienten eine Vollremission und sind typischerweise mit schweren Nebenwirkungen verbunden. Daher besteht ein hoher Bedarf an effektiveren und sichereren Therapien. Der PAR-1-Rezeptor trägt zur Entstehung, dem Wachstum, der Invasivität und der Metastasierung von Krebs bei. Außerdem vermittelt auf Endothelzellen exprimiertes PAR-1 Signale, die in Gefäßwachstum münden ("Angiogenese"), ein Vorgang, der zur Ermöglichung von Tumorwachstum über ca. 1 mm³ hinaus unerläßlich ist. Angiogenese trägt auch zur Enstehung oder Verschlimmerung anderer Erkrankungen bei, hierunter z.B. hämatopoetische Krebserkrankungen, die zur Erblindung führende Makuladegeneration und diabetische Retinopathie, entzündliche Erkrankungen wie rheumatoide Arthritis und Colitis.

Sepsis (oder Septikämie) ist eine häufige Erkrankung mit hoher Letalität. Anfängliche Symptome der Sepsis sind typischerweise unspezifisch (z.B. Fieber, reduziertes Allgemeinbefinden), im weiteren Verlauf kann es jedoch zur generalisierten Aktivierung des Gerinnungssystems kommen ("Disseminated Intravascular coagulation" oder "Verbrauchskoagulopathie" (DIC)) mit Mikrothrombosierung in verschiedenen Organen und sekundärer Blutungskomplikationen. DIC kann auch unabhängig von einer Sepsis auftreten, z.B. im Rahmen von Operationen oder bei Tumorerkrankungen.

Die Therapie der Sepsis besteht einerseits in der konsequenten Beseitigung der infektiösen Ursache, z.B. durch operative Herdsanierung und Antibiose. Andererseits besteht sie in der temporären intensivmedizinischen Unterstützung der beeinträchtigten Organsysteme. Therapien der verschiedenen Stadien dieser Erkrankung sind z.B. in folgender Publikation beschrieben (Dellinger et al., Crit. Care Med. 2004, 32, 858-873). Für die DIC existieren keine erwiesenermaßen effektive Therapien.

Eine Aufgabe der vorliegenden Erfindung ist es daher, neue PAR-1-Antagonisten zur Behandlung von Erkrankungen, wie z. B. Herz-Kreislauf-Erkrankungen und thromboembolischen Erkrankungen, sowie Tumorerkrankungen bei Menschen und Tieren zur Verfügung zu stellen.

WO 2006/002349, WO 2006/002350, WO 2007/089683 und WO 2007/101270 beschreiben strukturell ähnliche Piperidine als 11-β HSD1 Inhibitoren zur Behandlung von unter anderem Diabetes, thromboembolischen Erkrankungen und Schlaganfall.

J. J. McAtee et al., Bioorg. Med. Chem. Lett., 18, 2008, 3500-3503 beschreiben substituierte Piperidine als humane Urotensin-II Antagonisten.

Gegenstand der Erfindung sind Verbindungen der Formel 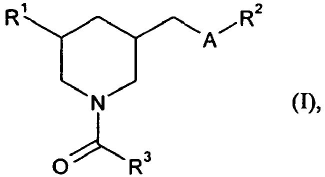 in welcher
- A: für ein Sauerstoffatom, ein Schwefelatom, -NR⁴-, -S(=O)- oder -S(=O)₂- steht,
wobei
R⁴ für Wasserstoff oder C₁-C₃-Alkyl steht,
oder
R² und R⁴ bilden zusammen mit dem Stickstoffatom an das sie gebunden sind einen 5- oder 6-gliedrigen Heterocyclus,
worin der Heterocyclus substituiert sein kann mit 1 bis 3 Substituenten, unabhängig voneinander ausgewählt aus der Gruppe bestehend aus Halogen, Cyano, Hydroxy, Amino, C₁-C₄-Alkyl, C₁-C₄-Alkoxy und C₁-C₄-Alkylamino,
oder
- A: für eine Gruppe der Formel 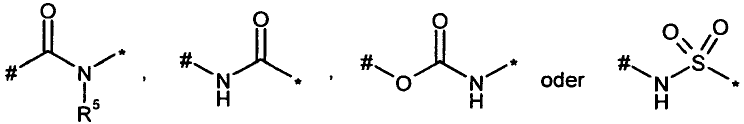 steht,
# die Anknüpfstelle an den Piperidinring ist,
* die Anknüpfstelle an R² ist,
und
R⁵ für Wasserstoff oder C₁-C₃-Alkyl steht,
- R¹: für Phenyl steht,
wobei Phenyl substituiert sein kann mit 1 bis 3 Substituenten, unabhängig voneinander ausgewählt aus der Gruppe bestehend aus Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Methylsulfonyl, C₁-C4₋Alkyl, C₁-C₄-Alkoxy und C₁-C₄-Alkoxycarbonyl,
- R²: für C₁-C₆-Alkyl, C₃-C₆-Cycloalkyl, 4- bis 6-gliedriges Heterocyclyl, Phenyl oder 5- oder 6-gliedriges Heteroaryl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₁-C₆-Alkylamino und Phenyl,
worin Phenyl substituiert sein kann mit 1 bis 3 Substituenten, unabhängig voneinander ausgewählt aus der Gruppe bestehend aus Halogen und Trifluormethyl,
und
wobei C₁-C₆-Alkyl substituiert sein kann mit einem Substituenten ausgewählt aus der Gruppe bestehend aus C₁-C₄-Alkoxy, C₁-C₄-Alkylsulfonyl, C₃-C₆-Cycloalkyl und Phenyl,
worin Cycloalkyl und Phenyl substituiert sein können mit 1 bis 3 Substituenten, unabhängig voneinander ausgewählt aus der Gruppe bestehend aus Halogen, Cyano, Monofluormethyl, Difluormethyl, Trifluormethyl, Monofluormethoxy, Difluormethoxy, Trifluormethoxy, C₁-C₄-Alkyl und C₁-C₄-Alkoxy,
- R³: für C₁-C₆-Alkyl, C₁-C₆-Alkoxy, C₁-C₆-Alkylamino, C₃-C₇-Cycloalkyl, 4- bis 7-gliedriges Heterocyclyl, Phenyl, 5- oder 6-gliedriges Heteroaryl, C₃-C₇-Cycloalkyloxy, C₃-C₇-Cycloalkylamino, 4- bis 7-gliedriges Heterocyclylamino, Phenylamino oder 5- oder 6-gliedriges Heteroarylamino steht,
wobei Alkyl, C₂-C₆-Alkoxy und Alkylamino substituiert sein können mit einem Substituenten ausgewählt aus der Gruppe bestehend aus Halogen, Hydroxy, Amino, Cyano, C₁-C₄-Alkoxy, C₁-C₄-Alkoxycarbonyl, C₃-C₇-Cycloalkyl, 4- bis 6-gliedriges Heterocyclyl, Phenyl und 5- oder 6-gliedriges Heteroaryl,
und
wobei Cycloalkyl, Heterocyclyl, Phenyl, Heteroaryl, Cycloalkyloxy, Cycloalkylamino, Heterocyclylamino, Phenylamino und Heteroarylamino substituiert sein können mit 1 bis 3 Substituenten, unabhängig voneinander ausgewählt aus der Gruppe bestehend aus Halogen, Cyano, Oxo, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxycarbonyl, Aminocarbonyl, C₁-C₄-Alkyl, C₁-C₄-Alkoxy, C₁-C₆-Alkylamino, C₁-C₄-Alkoxycarbonyl, C₁-C₄-Alkylaminocarbonyl und Cyclopropyl,
worin Alkyl substituiert sein kann mit einem Substituenten Hydroxy,
und ihre Salze, ihre Solvate und die Solvate ihrer Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin, N-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkoxycarbonyl Alkylaminocarbonyl und Alkylsulfonyl stehen für einen linearen oder verzweigten Alkylrest mit 1 bis 6 Kohlenstoffatomen, beispielhaft und vorzugsweise für Methyl, Ethyl, n-Propyl, iso-Propyl, n-Butyl, tert-Butyl, n-Pentyl und n-Hexyl.

Alkoxy steht beispielhaft und vorzugsweise für Methoxy, Ethoxy, n-Propoxy, iso-Propoxy, n-Butoxy, tert-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, tert-Butylamino, *N,N*-Dimethylamino, *N,N*-Diethylamino, *N*-Ethyl-*N-*methylamino, *N*-Methyl-*N*-n-propylamino, *N*-Iso-Propyl-*N*-n-propylamino und *N*-tert-Butyl-*N-*methylamino. C₁-C₄-Alkylamino steht beispielsweise für einen Monoalkylaminorest mit 1 bis 4 Kohlenstoffatomen oder für einen Dialkylaminorest mit jeweils 1 bis 4 Kohlenstoffatomen pro Alkylsubstituent.

Alkoxycarbonyl steht beispielhaft und vorzugsweise für Methoxycarbonyl, Ethoxycarbonyl, n-Propoxycarbonyl, iso-Propoxycarbonyl, n-Butoxycarbonyl und tert-Butoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, N,N-Dimethylaminocarbonyl, *N,N*-Diethylaminocarbonyl, *N*-Ethyl-*N*-methylaminocarbonyl, *N-*Methyl-N-n-propylaminocarbonyl, *N*-iso-Propyl-*N*-n-propylaminocarbonyl und *N*-tert-Butyl-*N-*methylaminocarbonyl. C₁-C₄-Alkylaminocarbonyl steht beispielsweise für einen Monoalkylaminocarbonylrest mit 1 bis 4 Kohlenstoffatomen oder für einen Dialkylaminocarbonylrest mit jeweils 1 bis 4 Kohlenstoffatomen pro Alkylsubstituent.

Alkylsulfonyl steht beispielhaft und vorzugsweise für Methylsulfonyl, Ethylsulfonyl, n-Propylsulfonyl, iso-Propylsulfonyl, n-Butylsulfonyl und tert-Butylsulfonyl.

Cycloalkyl steht für eine monocyclische Cycloalkylgruppe mit in der Regel 3 bis 7, bevorzugt 5 oder 6 Kohlenstoffatomen, beispielhaft und vorzugsweise für Cycloalkyl seien genannt Cyclopropyl, Cyclobutyl, Cyclopentyl, Cyclohexyl und Cycloheptyl.

Cycloalkyloxy steht für eine monocyclische Cycloalkyloxygruppe mit in der Regel 3 bis 7, bevorzugt 5 oder 6 Kohlenstoffatomen, beispielhaft und vorzugsweise für Cycloalkyloxy seien genannt Cyclopropyloxy, Cyclobutyloxy, Cyclopentyloxy und Cyclohexyloxy.

Cycloalkylamino steht für eine monocyclische Cycloalkylaminogruppe mit in der Regel 3 bis 7, bevorzugt 3 oder 4 Kohlenstoffatomen, beispielhaft und vorzugsweise für Cycloalkylamino seien genannt Cyclopropylamino, Cyclobutylamino, Cyclopentylamino und Cyclohexylamino.

Heterocyclyl steht für einen monocyclischen oder bicyclischen, heterocyclischen Rest mit 4 bis 7 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, beispielhaft und vorzugsweise für Oxetanyl, Azetidinyl, Pyrrolidin-2-yl, Pyrrolidin-3-yl, Pyrrolinyl, Tetrahydrofuranyl, Tetrahydrothienyl, Pyranyl, Piperidin-1-yl, Piperidin-2-yl, Piperidin-3-yl, Piperidin-4-yl, 1,2,5,6-Tetrahydropyridin-3-yl, 1,2,5,6-Tetrahydropyridin-4-yl, Thiopyranyl, Morpholin-1-yl, Morpholin-2-yl, Morpholin-3-yl, Piperazin-1-yl, Piperazin-2-yl, Thiomorpholin-2-yl, Thiomorpholin-3-yl, Thiomorpholin-4-yl, 1-Oxidothiomorpholin-4-yl, 1,1-Dioxidothiomorpholin-4-yl.

Heterocyclylamino steht für einen monocyclischen oder bicyclischen, heterocyclischen Heterocyclylamino-Rest mit 4 bis 7 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, beispielhaft und vorzugsweise für Oxetanylamino, Azetidinylamino, Pyrrolidin-2-yl-amino, Pyrrolidin-3-yl-amino, Tetrahydrofuranylamino, Tetrahydrothienylamino, Pyranylamino, Piperidin-2-yl-amino, Piperidin-3-yl-amino, Piperidin-4-yl-amino, 1,2,5,6-Tetrahydropyridin-3-yl-amino, 1,2,5,6-Tetrahydropyridin-4-yl-amino, Thiopyranylamino, Morpholin-2-yl-amino, Morpholin-3-yl-amino, Piperazin-2-yl-amino, Thiomorpholin-2-yl-amino, Thiomorpholin-3-yl-amino.

Heteroaryl steht für einen aromatischen, monocyclischen Rest mit in der Regel 5 oder 6 Ringatomen und bis zu 4 Heteroatomen aus der Reihe S, O und N, wobei ein Stickstoffatom auch ein N-Oxid bilden kann, beispielhaft und vorzugsweise für Thienyl, Furyl, Pyrrolyl, Thiazolyl, Oxazolyl, Isoxazolyl, Oxadiazolyl, Pyrazolyl, Imidazolyl, Triazolyl, Pyridyl, Pyrimidyl, Pyridazinyl, Pyrazinyl.

Heteroarylamino steht für einen aromatischen, monocyclischen Heteroarylamino-Rest mit in der Regel 5 oder 6 Ringatomen und bis zu 4 Heteroatomen aus der Reihe S, O und N, wobei ein Stickstoffatom auch ein N-Oxid bilden kann, beispielhaft und vorzugsweise für Thienylamino, Furylamino, Pyrrolylamino, Thiazolylamino, Oxazolylamino, Isoxazolylamino, Oxadiazolylamino, Pyrazolylamino, Imidazolylamino, Pyridylamino, Pyrimidylamino, Pyridazinylamino, Pyrazinylamino.

Halogen steht für Fluor, Chlor, Brom und Jod, vorzugsweise für Fluor und Chlor.

In der Formel der Gruppe, die für A stehen kann, steht der Endpunkt der Linie, neben der ein # oder ein * steht, nicht für ein Kohlenstoffatom beziehungsweise eine CH₂-Gruppe, sondern ist Bestandteil der Bindung zu dem Atom, an das A gebunden ist.

Bevorzugt sind Verbindungen der Formel (I), in welcher
- A: für ein Sauerstoffatom, ein Schwefelatom, -NR⁴-, -S(=O)- oder -S(=O)₂- steht,
wobei
R⁴ für Wasserstoff oder C₁-C₃-Alkyl steht,
oder
R² und R⁴ bilden zusammen mit dem Stickstoffatom an das sie gebunden sind einen 5- oder 6-gliedrigen Heterocyclus,
worin der Heterocyclus substituiert sein kann mit 1 bis 3 Substituenten, unabhängig voneinander ausgewählt aus der Gruppe bestehend aus Halogen, Cyano, Hydroxy, Amino, C₁-C₄-Alkyl, C₁-C₄-Alkoxy und C₁-C₄-Alkylamino,
oder
- A: für eine Gruppe der Formel 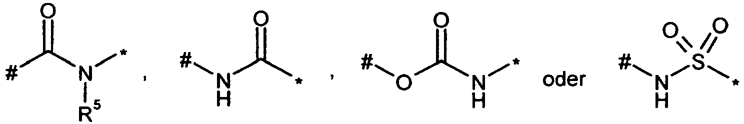 steht,
wobei
# die Anknüpfstelle an den Piperidinring ist,
* die Anknüpfstelle an R² ist,
und
R⁵ für Wasserstoff oder C₁-C₃-Alkyl steht,
- R¹: für Phenyl steht,
wobei Phenyl substituiert ist mit 1 bis 3 Substituenten, unabhängig voneinander ausgewählt aus der Gruppe bestehend aus Trifluormethyl, Trifluormethoxy, C₁-C₄-Alkyl, C₁-C₄-Alkoxy und C₁-C₄-Alkoxycarbonyl,
- R²: für C₁-C₆-Alkyl, C₃-C₆-Cycloalkyl, 4- bis 6-gliedriges Heterocyclyl, Phenyl oder 5- oder 6-gliedriges Heteroaryl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Hydroxy, Amino, Trifluormethyl, Difluormethoxy, Trifluormethoxy, Methyl, Ethyl, Methoxy, Ethoxy und Phenyl,
worin Phenyl substituiert sein kann mit 1 bis 3 Substituenten, unabhängig voneinander ausgewählt aus der Gruppe bestehend aus Halogen und Trifluormethyl,
und
wobei C₁-C₆-Alkyl substituiert sein kann mit einem Substituenten ausgewählt aus der Gruppe bestehend aus Methoxy, Ethoxy, Methylsulfonyl und Phenyl,
worin Phenyl substituiert sein kann mit 1 bis 3 Substituenten, unabhängig voneinander ausgewählt aus der Gruppe bestehend aus Halogen, Cyano, Trifluormethyl, Trifluormethoxy, Methyl, Ethyl, Methoxy und Ethoxy,
- R³: für C₃-C₇-Cycloalkyl, 4- bis 7-gliedriges Heterocyclyl, Phenyl, 5- oder 6-gliedriges Heteroaryl, C₃-C₇-Cycloalkyloxy, C₃-C₇-Cycloalkylamino, 4- bis 7-gliedriges Heterocyclylamino, Phenylamino oder 5- oder 6-gliedriges Heteroarylamino steht,
wobei Cycloalkyl, Heterocyclyl, Phenyl, Heteroaryl, Cycloalkyloxy, Cycloalkylamino, Heterocyclylamino, Phenylamino und Heteroarylamino substituiert sein können mit 1 bis 3 Substituenten, unabhängig voneinander ausgewählt aus der Gruppe bestehend aus Halogen, Cyano, Oxo, Hydroxy, Amino, Trifluormethyl, Difluormethoxy, Trifluormethoxy, Hydroxycarbonyl, Aminocarbonyl, Methyl, Ethyl, Methoxy, Ethoxy, Dimethylamino, Methoxycarbonyl, Ethoxycarbonyl, Dimethylaminocarbonyl und Cyclopropyl,
worin Methyl und Ethyl substituiert sein können mit einem Substituenten Hydroxy,
und ihre Salze, ihre Solvate und die Solvate ihrer Salze.

Bevorzugt sind auch Verbindungen der Formel (I), in welcher
- A: für ein Sauerstoffatom, ein Schwefelatom, -NR⁴-, -S(=O)- oder -S(=O)₂- steht,
wobei
R⁴ für Wasserstoff, Methyl oder Ethyl steht,
oder
R² und R⁴ bilden zusammen mit dem Stickstoffatom an das sie gebunden sind ein 3-Hydroxypyrrolidin-1-yl,
oder
- A: für eine Gruppe der Formel 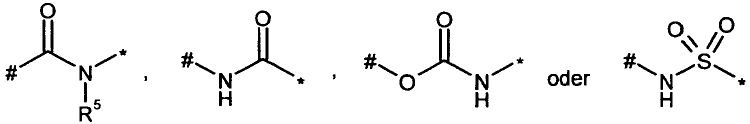 steht,
wobei
# die Anknüpfstelle an den Piperidinring ist,
* die Anknüpfstelle an R² ist,
und
R⁵ für Wasserstoff, Methyl oder Ethyl steht,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl und Methoxy,
- R²: für Methyl, Ethyl, Propyl, Isopropyl, 2-Methylprop-1-yl, tert-Butyl, Cyclopropyl, Cyclobutyl, Cyclopentyl, Cyclohexyl, Tetrahydro-2H-pyran-4-yl oder Phenyl steht,
wobei Phenyl substituiert sein kann mit 1 bis 2 Substituenten, unabhängig voneinander ausgewählt aus der Gruppe bestehend aus Halogen, Cyano, Methyl, Ethyl, Methoxy und Ethoxy,
und
wobei Methyl und Ethyl substituiert sein können mit einem Substituenten ausgewählt aus der Gruppe bestehend aus Methoxy, Methylsulfonyl und Phenyl,
- R³: for Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
und ihre Salze, ihre Solvate und die Solvate ihrer Salze.

Bevorzugt sind auch Verbindungen der Formel (I), in welcher
- A: für ein Sauerstoffatom, ein Schwefelatom, -NR⁴-, -S(=O)- oder -S(=O)₂- steht,
wobei
R⁴ für Wasserstoff, Methyl oder Ethyl steht,
oder
R² und R⁴ bilden zusammen mit dem Stickstoffatom an das sie gebunden sind ein 3-Hydroxypyrrolidin-1-yl,
oder
- A: für eine Gruppe der Formel 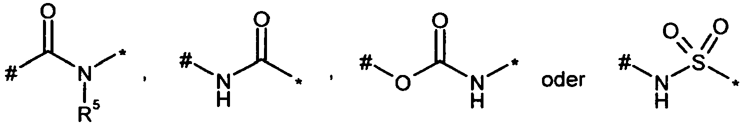 steht,
wobei
# die Anknüpfstelle an den Piperidinring ist,
* die Anknüpfstelle an R² ist,
und
R⁵ für Wasserstoff, Methyl oder Ethyl steht,
- R¹: für Phenyl steht,
wobei Phenyl substituiert ist mit Trifluormethyl,
- R²: für Methyl, Ethyl, Propyl, Isopropyl, 2-Methylprop-1-yl, tert-Butyl, Cyclopropyl, Cyclobutyl, Cyclopentyl, Cyclohexyl, Tetrahydro-2H-pyran-4-yl oder Phenyl steht,
wobei Phenyl substituiert sein kann mit 1 bis 2 Substituenten, unabhängig voneinander ausgewählt aus der Gruppe bestehend aus Halogen, Methyl und Methoxy,
und
wobei Methyl und Ethyl substituiert sein können mit einem Substituenten ausgewählt aus der Gruppe bestehend aus Methoxy, Methylsulfonyl und Phenyl,
- R³: für Morpholin-4-yl steht,
und ihre Salze, ihre Solvate und die Solvate ihrer Salze.

Bevorzugt sind auch Verbindungen der Formel (I), in welcher
- A: für ein Sauerstoffatom, ein Schwefelatom, -S(=O)- oder -S(=O)₂- steht,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl und Methoxy,
- R²: für Methyl, Ethyl, Propyl, Isopropyl, 2-Methylprop-1-yl, tert-Butyl, Cyclopropyl, Cyclobutyl, Cyclopentyl, Cyclohexyl, Tetrahydro-2H-pyran-4-yl oder Phenyl steht,
wobei Phenyl substituiert sein kann mit 1 bis 2 Substituenten, unabhängig voneinander ausgewählt aus der Gruppe bestehend aus Halogen, Cyano, Methyl, Ethyl, Methoxy und Ethoxy,
und
wobei Methyl und Ethyl substituiert sein können mit einem Substituenten ausgewählt aus der Gruppe bestehend aus Methoxy, Methylsulfonyl und Phenyl,
- R³: für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
und ihre Salze, ihre Solvate und die Solvate ihrer Salze.

Bevorzugt sind auch Verbindungen der Formel (I), in welcher
- A: für ein Sauerstoffatom, ein Schwefelatom, -S(=O)- oder -S(=O)₂- steht,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl und Methoxy,
- R²: für Phenyl steht,
wobei Phenyl substituiert sein kann mit 1 bis 2 Substituenten, unabhängig voneinander ausgewählt aus der Gruppe bestehend aus Halogen, Methyl und Methoxy,
- R³: für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
und ihre Salze, ihre Solvate und die Solvate ihrer Salze.

Bevorzugt sind auch Verbindungen der Formel (I), in welcher
- A: für eine Gruppe der Formel 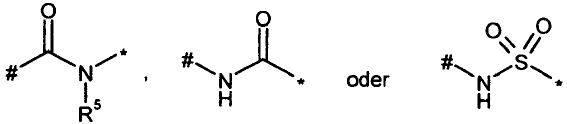 steht,
wobei
# die Anknüpfstelle an den Piperidinring ist,
* die Anknüpfstelle an R² ist,
und
- R⁵: für Wasserstoff, Methyl oder Ethyl steht,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl und Methoxy,
- R²: für Methyl, Ethyl, Propyl, Isopropyl, 2-Methylprop-1-yl, tert-Butyl, Cyclopropyl, Cyclobutyl, Cyclopentyl, Cyclohexyl, Tetrahydro-2H-pyran-4-yl oder Phenyl steht,
wobei Phenyl substituiert sein kann mit 1 bis 2 Substituenten, unabhängig voneinander ausgewählt aus der Gruppe bestehend aus Halogen, Cyano, Methyl, Ethyl, Methoxy und Ethoxy,
und
wobei Methyl und Ethyl substituiert sein können mit einem Substituenten ausgewählt aus der Gruppe bestehend aus Methoxy, Methylsulfonyl und Phenyl,
- R³: für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
und ihre Salze, ihre Solvate und die Solvate ihrer Salze.

Bevorzugt sind auch Verbindungen der Formel (I), in welcher
- A: für eine Gruppe der Formel 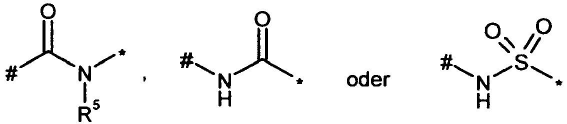 steht,
wobei
# die Anknüpfstelle an den Piperidinring ist,
* die Anknüpfstelle an R² ist,
und
- R⁵: für Wasserstoff, Methyl oder Ethyl steht,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl und Methoxy,
- R²: für Methyl, Ethyl, Isopropyl, tert-Butyl, Cyclopropyl, Cyclobutyl, Cyclopentyl oder Cyclohexyl steht,
wobei Methyl und Ethyl substituiert sein können mit einem Substituenten ausgewählt aus der Gruppe bestehend aus Methoxy und Methylsulfonyl,
- R³: für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
und ihre Salze, ihre Solvate und die Solvate ihrer Salze.

Bevorzugt sind auch Verbindungen der Formel (I), in welcher die Substituenten -R¹ und -CH₂-AR² in cis-Position zueinander stehen.

Bevorzugt sind auch Verbindungen der Formel (I), in welcher das Kohlenstoffatom, an das R¹ gebunden ist, eine S-Konfiguration hat und das Kohlenstoffatom, an das -CH₂-A-R² gebunden ist, ebenfalls eine S-Konfiguration hat.

Bevorzugt sind auch Verbindungen der Formel (I), in welcher
- A: für ein Sauerstoffatom, ein Schwefelatom, -NR⁴-, -S(=O)- oder -S(=O)₂- steht,
wobei
R⁴ für Wasserstoff, Methyl oder Ethyl steht,
oder
R² und R⁴ bilden zusammen mit dem Stickstoffatom an das sie gebunden sind ein 3-Hydroxypyrrolidin-1-yl.

Bevorzugt sind auch Verbindungen der Formel (I), in welcher
- A: für ein Sauerstoffatom, ein Schwefelatom, -S(=O)- oder -S(=O)₂- steht.

Bevorzugt sind auch Verbindungen der Formel (I), in welcher
- A: für ein Sauerstoffatom oder ein Schwefelatom steht.

Bevorzugt sind auch Verbindungen der Formel (I), in welcher
- A: für -S(=O)- oder -S(=O)₂- steht.

Bevorzugt sind auch Verbindungen der Formel (I), in welcher
- A: für S(=O)- steht.

Bevorzugt sind auch Verbindungen der Formel (I), in welcher
- A: für -S(=O)₂- steht.

Bevorzugt sind auch Verbindungen der Formel (I), in welcher
- A: für eine Gruppe der Formel 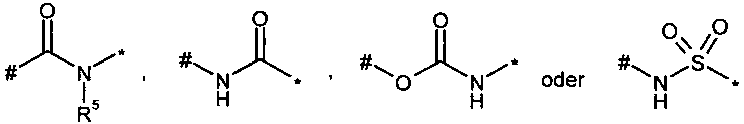 steht,
wobei
# die Anknüpfstelle an den Piperidinring ist,
* die Anknüpfstelle an R² ist,
und
R⁵ für Wasserstoff, Methyl oder Ethyl steht.

Bevorzugt sind auch Verbindungen der Formel (I), in welcher
- A: für eine Gruppe der Formel 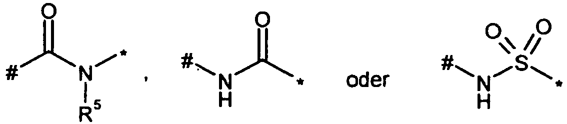 steht,
wobei
# die Anknüpfstelle an den Piperidinring ist,
* die Anknüpfstelle an R² ist,
und
R⁵ für Wasserstoff, Methyl oder Ethyl steht.

Bevorzugt sind auch Verbindungen der Formel (I), in welcher
- A: für eine Gruppe der Formel 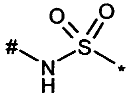 steht,
wobei
# die Anknüpfstelle an den Piperidinring ist,
* die Anknüpfstelle an R² ist.

Bevorzugt sind auch Verbindungen der Formel (I), in welcher R¹ für Phenyl steht, wobei Phenyl substituiert ist mit einem Substituenten in para-Position zur Verknüpfungsstelle an den Piperidinring, ausgewählt aus der Gruppe bestehend aus Trifluormethyl, Trifluormethoxy und Ethyl.

Bevorzugt sind auch Verbindungen der Formel (I), in welcher R¹ für Phenyl steht, wobei Phenyl substituiert ist mit einem Substituenten Trifluormethyl in para-Position zur Verknüpfungsstelle an den Piperidinring.

Bevorzugt sind auch Verbindungen der Formel (I), in welcher
- R²: für Methyl, Ethyl, Isopropyl, tert-Butyl, Cyclopropyl, Cyclobutyl, Cyclopentyl oder Cyclohexyl steht,
wobei Methyl und Ethyl substituiert sein können mit einem Substituenten ausgewählt aus der Gruppe bestehend aus Methoxy und Methylsulfonyl.

Bevorzugt sind auch Verbindungen der Formel (I), in welcher
- R²: für Phenyl steht,
wobei Phenyl substituiert sein kann mit 1 bis 2 Substituenten, unabhängig voneinander ausgewählt aus der Gruppe bestehend aus Halogen, Methyl und Methoxy.

Bevorzugt sind auch Verbindungen der Formel (I), in welcher R³ für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht.

Bevorzugt sind auch Verbindungen der Formel (I), in welcher R³ für Morpholin-4-yl steht.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei entweder
[A] Verbindungen der Formel 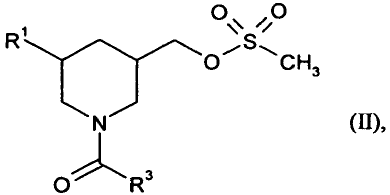 in welcher
   R¹ und R³ die oben angegebene Bedeutung aufweisen,
   mit Verbindungen der Fonnel

   H—_{A}—_{R}² (III),

   in welcher
   R² die oben angegebene Bedeutung aufweist,
   - A: für ein Sauerstoffatom, ein Schwefelatom oder -NR⁴- steht, und
   R⁴ die oben angegebene Bedeutung aufweist,
   zu Verbindungen der Formel 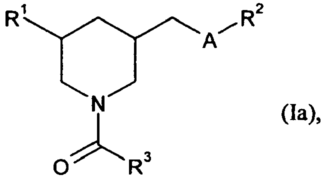 in welcher
   - A: für ein Sauerstoffatom, ein Schwefelatom oder -NR⁴- steht, und
   - R¹, R², R³ und R⁴: die oben angegebene Bedeutung aufweisen,
   umgesetzt werden
   oder
[B] Verbindungen der Formel (I) in welcher A für ein Schwefelatom steht und R¹, R² und R³ die oben angegebene Bedeutung aufweisen, mit einem Oxidationsmittel
   zu Verbindungen der Formel 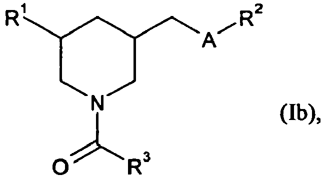 in welcher
   - A: für -S(=O)- oder -S(=O)₂- steht, und
   - R¹, R² und R³: die oben angegebene Bedeutung aufweisen,
   umgesetzt werden
   oder
[C] Verbindungen der Formel 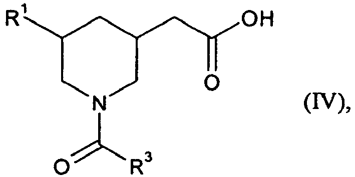 in welcher
   R¹ und R³ die oben angegebene Bedeutung aufweisen,
   mit Verbindungen der Formel 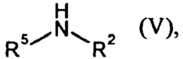 in welcher
   R² und R⁵ die oben angegebene Bedeutung aufweisen,
   zu Verbindungen der Formel 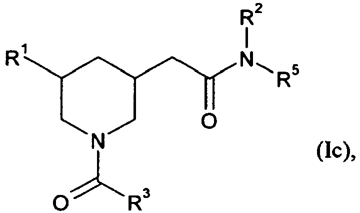 in welcher
   R¹, R², R³ und R⁵ die oben angegebene Bedeutung aufweisen,
   umgesetzt werden
   oder
[D] Verbindungen der Formel 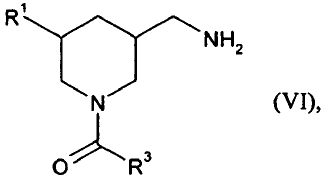 in welcher
   R¹ und R³ die oben angegebene Bedeutung aufweisen,
   mit Verbindungen der Formel 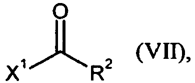 in welcher
   R² die oben angegebene Bedeutung aufweist, und
   X¹ für Halogen, bevorzugt Brom oder Chlor, oder Hydroxy steht,
   zu Verbindungen der Formel 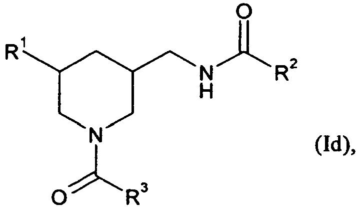 in welcher
   R¹, R² und R³ die oben angegebene Bedeutung aufweisen,
   umgesetzt werden
   oder
[E] Verbindungen der Formel (VI) mit Verbindungen der Formel 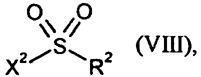 in welcher
   R² die oben angegebene Bedeutung aufweist, und
   X² für Halogen, bevorzugt Brom oder Chlor, steht,
   zu Verbindungen der Formel 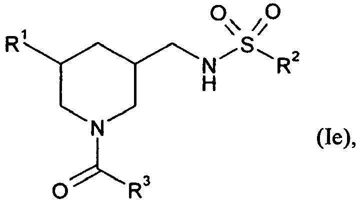 in welcher
   R¹, R² und R³ die oben angegebene Bedeutung aufweisen,
   umgesetzt werden
   oder
[F] Verbindungen der Formel 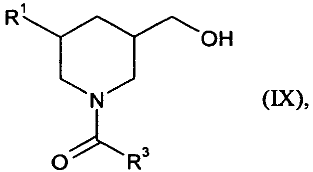 in welcher
   R¹ und R³ die oben angegebene Bedeutung aufweisen,
   mit Verbindungen der Formel 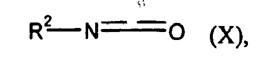 in welcher
   R² die oben angegebene Bedeutung aufweist,
   zu Verbindungen der Formel 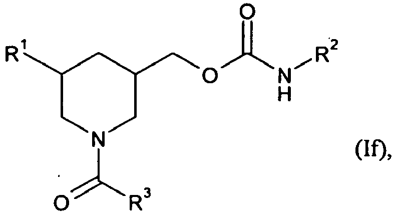 in welcher
   R¹, R² und R³ die oben angegebene Bedeutung aufweisen,
   umgesetzt werden.

Die Verbindungen der Formeln (Ia), (Ib), (Ic), (Id), (Ie) und (If) sind jeweils eine Teilmenge der Verbindungen der Formel (I).

Die Umsetzung nach Verfahren [A] erfolgt im Allgemeinen in inerten Lösungsmitteln, gegebenenfalls in Gegenwart einer Base, gegebenenfalls in einer Mikrowelle, gegebenenfalls in Gegenwart von Molekularsieb, bevorzugt in einem Temperaturbereich von 0°C bis 150°C bei Normaldruck.

Inerte Lösungsmittel sind beispielsweise Halogenkohlenwasserstoffe wie Methylenchlorid, Trichlormethan oder 1,2-Dichlorethan, Kohlenwasserstoffe wie Benzol oder Toluol, Ether wie Diethylether, Dioxan, Tetrahydrofuran oder 1,2-Dimethoxyethan, oder andere Lösemittel wie Dimethylformamid, Dimethylacetamid oder Dimethylsulfoxid, bevorzugt ist Dimethylformamid.

Alternativ können Alkohole wie Methanol oder Ethanol als Lösungsmittel eingesetzt werden, wobei in diesen Fällen das Lösungsmittel auch gleichzeitig das Reagenz darstellt.

Basen sind beispielsweise Natrium- oder Kaliummethanolat, oder Natrium- oder Kaliumethanolat oder Kalium-tert.-butylat, oder Amide wie Natriumamid, Lithium-bis-(trimethylsilyl)amid oder Lithiumdiisopropylamid, oder metallorganische Verbindungen wie Butyllithium oder Phenyllithium, oder Alkalihydroxide wie Natrium- oder Kaliumhydroxid, oder andere Basen wie Natriumhydrid oder Kaliumhydrid, bevorzugt ist Natriumhydrid.

Die Verbindungen der Formel (III) sind bekannt oder lassen sich nach bekannten Verfahren aus den entsprechenden Ausgangsverbindungen synthetisieren.

Die Umsetzung nach Verfahren [B] erfolgt im Allgemeinen in inerten Lösungsmitteln, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan oder 1,2-Dichlorethan, oder Alkohole wie Methanol oder Ethanol, oder andere Lösungsmittel wie Essigsäure, oder Gemische der Lösungsmittel oder Gemische der Lösungsmittel mit Wasser, bevorzugt ist Methylenchlorid.

Oxidationsmittel sind beispielsweise *meta*-Chlorperoxybenzoesäure Oxon, Peroxyessigsäure, Wasserstoffperoxid, Wasserstoffperoxid-Harnstoff-Komplex oder Kaliumpermanganat, bevorzugt ist meta-Chlorperoxybenzoesäure.

Die Umsetzung nach Verfahren [C] erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N,*'-Dipropyl-, *N,N*'-Diisopropyl-, *N,N*'-Dicyclohexylcarbodiimid, *N*-(3-Dimethylaminoisopropyl)-*N*'-ethylcarbodiimid-hydrochlorid (EDC), *N*-Cyclohexylearbodiimid-*N*'-propyloxymethyl-Polystyrol (PS-Carbodiimid) oder Carbonylverbindungen wie Carbonyldiimidazol, oder 1,2-*O*xazoliumverbindungen wie 2-Ethyl-5-phenyl-1,2-oxazolium-3-sulfat oder *2-tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoro-borat (TPTU) oder *O*-(7-Azabenzotriazol- -yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder Benzotriazol-1-yloxy-tris(pyrrolidino)-phosphonium-Hexafluorophosphat (PYBOP), oder N-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU in Gegenwart von Diisopropylethylamin durchgeführt.

Die Verbindungen der Formel (V) sind bekannt oder lassen sich nach bekannten Verfahren aus den entsprechenden Ausgangsverbindungen synthetisieren.

Die Umsetzung nach Verfahren [D] erfolgt, wenn X¹ für Halogen steht, im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Methylenchlorid.

Basen sind beispielsweise Triethylamin, Diisopropylethylamin oder *N*-Methylmorpholin, bevorzugt ist Triethylamin oder Diisopropylethylamin.

Die Umsetzung nach Verfahren [D] erfolgt, wenn X¹ für Hydroxy steht, im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N*,'-Dipropyl-, *N,N*'-Diisopropyl-, *N,N*'-Dicyclohexylcarbodiimid, *N*-(3-Di-methylaminoisopropyl)-*N*'-ethylcarbodiimid-hydrochlorid (EDC), *N*-Cyclohexylcarbodiimid-*N*'-propyloxymethyl-Polystyrol (PS-Carbodümid) oder Carbonylverbindungen wie Carbonyldiimidazol, oder 1,2-Oxazoliumverbindungen wie 2-Ethyl-5-phenyl-1,2-oxazolium-3-sulfat oder 2-*tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N;N'*-tetra-methyluronium-hexafluorophosphat (HBTU), 2-(2-Oxo-1-(2H)-pyridyl)-1,1,3,3-tetramethyluroniumtetrafluoro-borat (TPTU) oder *O*-(7-Azabenzotriazol-1-yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder Benzotriazol-1-yloxy-tris(pyrrolidino)-phosphonium-Hexafluorophosphat (PYBOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, *N*-Methylpiperidin, 4-Dimethylaminopyridin oder Diisopropylethylamin.

Vorzugsweise wird die Kondensation mit HATU in Gegenwart von Diisopropylethylamin oder mit PYBOP in Gegenwart von Diisopropylethylamin durchgeführt.

Die Verbindungen der Formel (VII) sind bekannt oder lassen sich nach bekannten Verfahren aus den entsprechenden Ausgangsverbindungen synthetisieren.

Die Umsetzung nach Verfahren [E] erfolgt im Allgemeinen in inerten Lösungsmitteln, gegebenenfalls in Gegenwart einer Base, bevorzugt in einem Temperaturbereich von 0°C bis 50°C bei Normaldruck.

Inerte Lösungsmittel sind beispielsweise Tetrahydrofuran, Methylenchlorid, Pyridin, Dioxan oder Dimethylformamid, bevorzugt ist Methylenchlorid.

Basen sind beispielsweise Triethylamin, Diisopropylethylamin oder *N*-Methylmorpholin, bevorzugt ist Triethylamin oder Diisopropylethylamin.

Die Verbindungen der Formel (VIII) sind bekannt oder lassen sich nach bekannten Verfahren aus den entsprechenden Ausgangsverbindungen synthetisieren.

Die Umsetzung nach Verfahren [F] erfolgt im Allgemeinen in inerten Lösungsmitteln, in Gegenwart einer Säure, bevorzugt in einem Temperaturbereich von 0°C bis 50°C bei Normaldruck.

Inerte Lösungsmittel sind beispielsweise Tetrahydrofuran, Methylenchlorid, Dioxan oder Dimethylformamid, bevorzugt ist Methylenchlorid.

Säuren sind beispielsweise Chlorwasserstoff in Dioxan, Bromwasserstoff in Dioxan oder konzentrierte Schwefelsäure in Dioxan, bevorzugt ist Chlorwasserstoff in Dioxan.

Die Verbindungen der Formel (X) sind bekannt oder lassen sich nach bekannten Verfahren aus den entsprechenden Ausgangsverbindungen synthetisieren.

Die Verbindungen der Formel (II) sind bekannt oder können hergestellt werden, indem Verbindungen der Formel 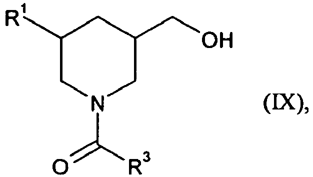 in welcher
R¹ und R³ die oben angegebene Bedeutung aufweisen,
mit Methansulfonsäurechlorid umgesetzt werden.

Die Umsetzung erfolgt im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan, 1,2-Dimethoxyethan oder Dimethylformamid, bevorzugt ist Methylenchlorid oder Tetrahydrofuran.

Basen sind beispielsweise Triethylamin, Diisopropylethylamin, N-Methylmorpholin oder Natriumhydrid, bevorzugt ist Triethylamin oder Natriumhydrid.

Die Verbindungen der Formel (IX) sind bekannt oder können hergestellt werden, indem Verbindungen der Formel 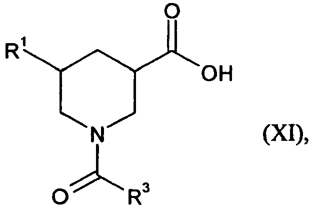 in welcher
R¹ und R³ die oben angegebene Bedeutung aufweisen, mit einem Reduktionsmittel umgesetzt werden.

Die Umsetzung erfolgt im Allgemeinen in inerten Lösungsmitteln, bevorzugt in einem Temperaturbereich von -30°C bis 80°C bei Normaldruck.

Inerte Lösungsmittel sind beispielsweise Ether wie Diethylether, Tetrahydrofuran, Dioxan oder 1,2-Dimethoxyethan, bevorzugt ist Tetrahydrofuran.

Reduktionsmittel sind beispielsweise Lithiumaluminiumhydrid, Natriumborhydrid in Verbindung mit Bortrifluorid-Diethyletherat, Lithiumborhydrid, Boran-THF-Komplex, Boran-Dimethylsulfid-Komplex, bevorzugt ist Natriumborhydrid in Verbindung mit Bortrifluorid-Diethyletherat.

Die Verbindungen der Formel (XI) sind bekannt oder können hergestellt werden, indem Verbindungen der Formel 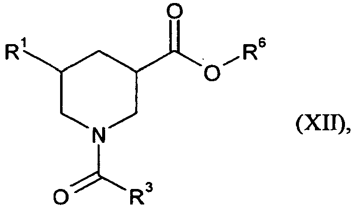 in welcher
R¹ und R³ die oben angegebene Bedeutung aufweisen, und
R⁶ für Methyl oder Ethyl steht,
mit einer Base umgesetzt werden.

Die Umsetzung erfolgt im Allgemeinen in inerten Lösungsmitteln, in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan, Tetrachlormethan oder 1,2-Dichlorethan, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt ist Lithiumhydroxid.

Die Verbindungen der Formel (XII) sind bekannt oder können hergestellt werden, indem Verbindungen der Formel 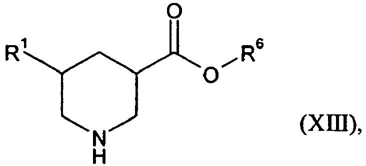 in welcher
R¹ und R⁶ die oben angegebene Bedeutung aufweisen,
mit Verbindungen der Formel 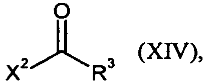 in welcher
R³ die oben angegebene Bedeutung aufweist, und
X² für Halogen, bevorzugt Brom oder Chlor, oder Hydroxy steht,
umgesetzt werden.

Die Umsetzung erfolgt, wenn X² für Halogen steht, im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Tetrahydrofuran.

Basen sind beispielsweise Triethylamin, Diisopropylethylamin oder N-Methylmorpholin, bevorzugt ist Triethylamin oder Diisopropylethylamin.

Die Umsetzung erfolgt, wenn X² für Hydroxy steht, im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N,'*-Dipropyl-, *N,N'*-Diisopropyl-, *N,N'*-Dicyclohexylcarbodiimid, *N-(3-Di*methylaminoisopropyl)-*N*'-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoro-borat (TPTU) oder *O*-(7-Azabenzotriazol-1-yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (XIV) sind bekannt oder lassen sich nach bekannten Verfahren aus den entsprechenden Ausgangsverbindungen synthetisieren.

Die Verbindungen der Formel (XIII) sind bekannt oder können hergestellt werden, indem Verbindungen der Formel 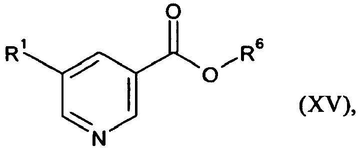 in welcher
R¹ und R⁶ die oben angegebene Bedeutung aufweisen,
hydriert werden.

Die Hydrierung erfolgt im Allgemeinen mit einem Reduktionsmittel in inerten Lösungsmitteln, gegebenenfalls unter Zusatz von Säure wie Mineralsäuren und Carbonsäuren, bevorzugt Essigsäure, bevorzugt in einem Temperaturbereich von Raumtemperatur bis zum Rückfluss der Lösungsmittel und in einem Druckbereich von Normaldruck bis 100 bar, bevorzugt bei 50-80 bar.

Reduktionsmittel sind beispielsweise Wasserstoff mit Palladium auf Aktivkohle, mit Rhodium auf Aktivkohle, mit Ruthenium auf Aktivkohle oder daraus gemischte Katalysatorn, oder Wasserstoff mit Palladium auf Aluminiumoxid oder mit Rhodium auf Aluminiumoxid, bevorzugt ist Wasserstoff mit Palladium auf Aktivkohle oder mit Rhodium auf Aktivkohle.

Inerte Lösungsmittel sind beispielsweise Alkohole wie Methanol, Ethanol, n-Propanol, isoPropanol, n-Butanol oder tert-Butanol, bevorzugt ist Methanol oder Ethanol.

Die Verbindungen der Formel (XV) sind bekannt oder können hergestellt werden, indem Verbindungen der Formel 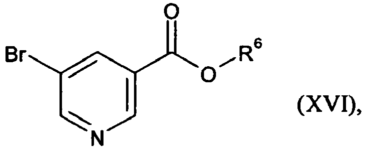 in welcher
R⁶ die oben angegebene Bedeutung aufweist,
mit Verbindungen der Formel 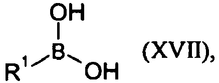 in welcher
R' die oben angegebene Bedeutung aufweist,
umgesetzt werden.

Die Umsetzung erfolgt im Allgemeinen in inerten Lösungsmitteln, in Gegenwart eines Katalysators, gegebenenfalls in Gegenwart eines Zusatzreagenzes, bevorzugt in einem Temperaturbereich von Raumtemperatur bis zum Rückfluss des Lösungsmittels bei Normaldruck.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder andere Lösungsmittel wie Nitrobenzol, Dimethylformamid, Dimethylacetamid, Dimethylsulfoxid oder N-Methylpyrrolidon, gegebenenfalls wird diesen Lösungsmitteln etwas Wasser zugesetzt. Bevorzugt ist Toluol mit Wasser oder eine Mischung aus 1,2-Dimethoxyethan, Dimethylformamid und Wasser.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat oder Bis-(diphenylphosphanferrocenyl)-palladium-(II)-chlorid.

Zusatzreagenzien sind beispielsweise Kaliumacetat, Cäsium-, Kalium- oder Natriumcarbonat, Bariumhydroxid, Kalium-tert-butylat, Cäsiumfluorid, Kaliumfluorid oder Kaliumphosphat, bevorzugt sind Kaliumfluorid oder Natriumcarbonat.

Die Verbindungen der Formeln (XVI) und (XVII) sind bekannt oder lassen sich nach bekannten Verfahren aus den entsprechenden Ausgangsverbindungen synthetisieren.

Die Verbindungen der Formel (IV) sind bekannt oder können hergestellt werden, indem Verbindungen der Formel 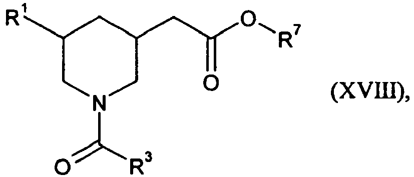 in welcher
R¹ und R³ die oben angegebene Bedeutung aufweisen, und
R⁷ für Methyl oder Ethyl steht,
mit einer Base umgesetzt werden.

Die Verseifung erfolgt nach den unter der Verseifung von Verbindungen der Formel (XII) angegebenen Reaktionsbedingungen.

Die Verbindungen der Formel (XVIII) sind bekannt oder können hergestellt werden, indem Verbindungen der Formel 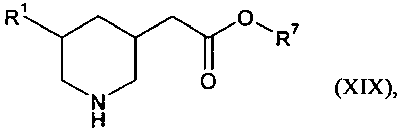 in welcher
R¹ und R⁷ die oben angegebene Bedeutung aufweisen,
mit Verbindungen der Formel (XIV) umgesetzt werden.

Die Umsetzung erfolgt nach den unter der Umsetzung von Verbindungen der Formel (XIII) mit Verbindungen der Formel (XIV) angegebenen Reaktionsbedingungen.

Die Verbindungen der Formel (XIX) sind bekannt oder können hergestellt werden, indem Verbindungen der Formel 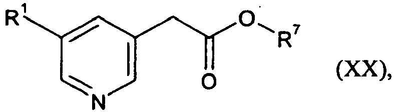 in welcher
R¹ und R⁷ die oben angegebene Bedeutung aufweisen,
hydriert werden.

Die Hydrierung erfolgt nach den unter der Hydrierung von Verbindungen der Formel (XV) angegebenen Reaktionsbedingungen.

Die Verbindungen der Formel (XX) sind bekannt oder können hergestellt werden, indem Verbindungen der Formel 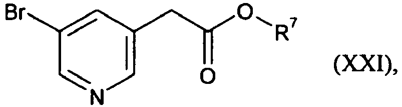 in welcher
R⁷ die oben angegebene Bedeutung aufweist,
mit Verbindungen der Formel (XVII) umgesetzt werden.

Die Umsetzung erfolgt nach den unter der Umsetzung von Verbindungen der Formel (XVI) mit Verbindungen der Formel (XVII) angegebenen Reaktionsbedingungen.

Die Verbindungen der Formel (XXI) sind bekannt oder lassen sich nach bekannten Verfahren aus den entsprechenden Ausgangsverbindungen synthetisieren.

Die Verbindungen der Formel (VI) sind bekannt oder können hergestellt werden, indem Verbindungen der Formel 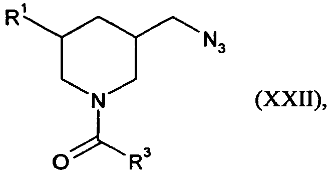 in welcher
R¹ und R³ die oben angegebene Bedeutung aufweisen,
mit einem Reduktionsmittel umgesetzt werden.

Die Umsetzung erfolgt im Allgemeinen in inerten Lösungsmitteln, bevorzugt in einem Temperaturbereich von Raumtemperatur bis zum Rückfluss der Lösungsmittel und in einem Druckbereich von Normaldruck bis 100 bar.

Reduktionsmittel sind beispielsweise Wasserstoff mit Palladium auf Aktivkohle, mit Rhodium auf Aktivkohle, mit Ruthenium auf Aktivkohle oder daraus gemischte Katalysatoren, oder Wasserstoff mit Palladium auf Aluminiumoxid oder mit Rhodium auf Aluminiumoxid, oder Triphenylphosphin, oder Cer(III)chlorid-Heptahydrat mit Natriumiodid, bevorzugt ist Wasserstoff mit Palladium auf Aktivkohle oder mit Rhodium auf Aktivkohle.

Inerte Lösungsmittel sind beispielsweise Alkohole wie Methanol, Ethanol, n-Propanol, isoPropanol, n-Butanol oder tert-Butanol, bevorzugt ist Methanol oder Ethanol.

Die Verbindungen der Formel (XXII) sind bekannt oder können hergestellt werden, indem Verbindungen der Formel (II) mit Natriumazid umgesetzt werden.

Die Umsetzung erfolgt im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan, Tetrachlormethan oder 1,2-Dichlorethan, oder andere Lösungsmittel wie Dimethylformamid, Dimethylacetamid oder Acetonitril, bevorzugt ist Dimethylformamid.

In den Verbindungen der oben genannten Verfahren sind freie Aminogruppen gegebenenfalls während der Umsetzung durch dem Fachmann bekannte Schutzgruppen geschützt, bevorzugt ist eine tert-Butoxycarbonyl-Schutzgruppe. Diese Schutzgruppen werden nach der Umsetzung durch dem Fachmann bekannte Reaktionen abgespalten, bevorzugt ist die Umsetzung mit Trifluoressigsäure oder konzentierter Salzsäure.

Die Herstellung der Verbindungen der Formel (I) kann durch folgende Syntheseschemata verdeutlicht werden.

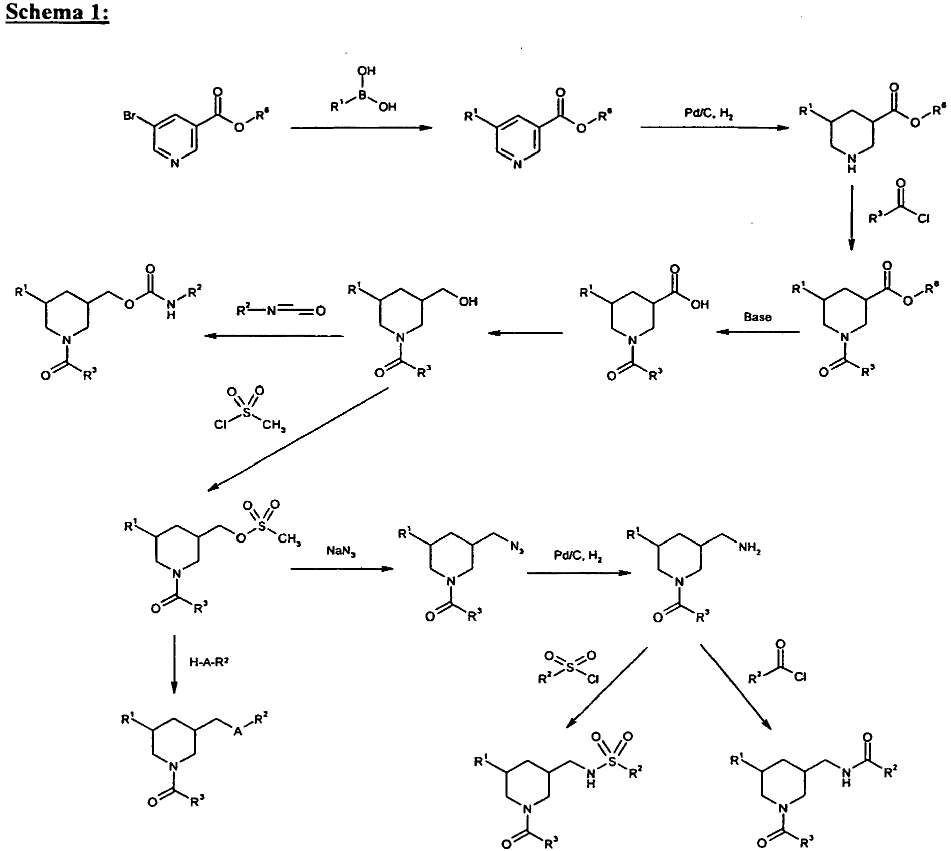

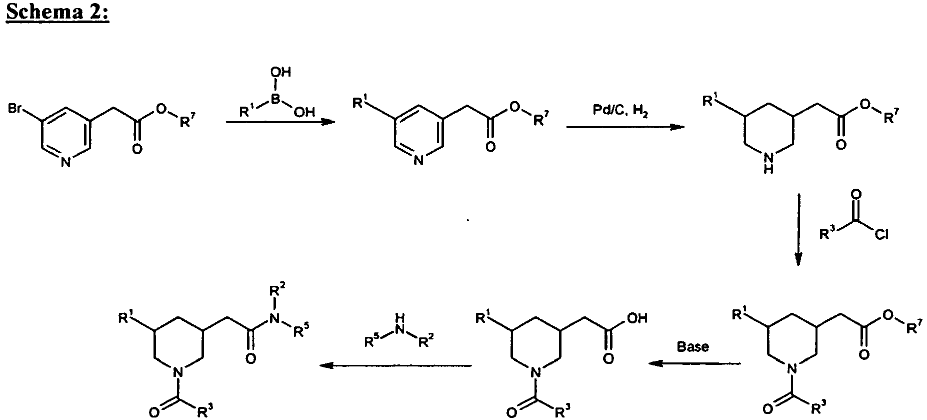

Die erfindungsgemäßen Verbindungen zeigen ein nicht vorhersehbares, wertvolles pharmakologisches und pharmakokinetisches Wirkspektrum. Es handelt sich dabei um selektive Antagonisten des PAR-1-Rezeptors, die insbesondere als Thrombozytenaggregationshemmer, als Hemmer der Endothelproliferation und als Hemmer des Tumorwachstums wirken.

Sie eigenen sich daher zur Verwendung als Arzneimittel zur Behandlung und/oder Prophylaxe von Krankheiten bei Menschen und Tieren.

Weiterin hierin beschrieben ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischämien, Schlaganfall (Stroke) und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit intravasalen Körpern, wie z. B. künstlichen Herzklappen, Kathetern, intraaortale Ballongegenpulsation und Schrittmachersonden.

Thromboembolische Komplikationen treten ferner auf bei mikroangiopathischen hämolytischen Anämien, extrakorporalen Blutkreisläufen, wie z. B. Hämodialyse, Hämofiltration, ventricular assist devices und Kunstherz, sowie Herzklappenprothesen.

Außerdem kommen die erfindungsgemäßen Verbindungen auch zur Beeinflussung der Wundheilung, für die Prophylaxe und/oder Behandlung von atherosklerotischen Gefäßerkrankungen und entzündlichen Erkrankungen wie rheumatische Erkrankungen des Bewegungsapparats, koronaren Herzkrankheiten, von Herzinsuffizienz, von Bluthochdruck, von entzündlichen Erkrankungen, wie z.B. Asthma, COPD, entzündlichen Lungenerkrankungen, Glomerulonephritis und entzündlichen Darmerkrankungen in Betracht, darüber hinaus ebenso für die Prophylaxe und/oder Behandlung der Alzheimer'schen Erkrankung, Autoimmunerkrankungen, Morbus Crohn und Kolitis Ulzerosa. Außerdem können die erfindungsgemäßen Verbindungen zur Inhibition des Tumorwachstums und der Metastasenbildung, bei Mikroangiopathien, altersbedingter Makuladegeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Weiterhin eignen sich die erfindungsgemäßen Verbindungen zur Behandlung von Krebs. Krebserkrankungen schließen unter anderem ein: Karzinome (hierunter Brustkrebs, hepatozelluläre karzinome, Lunkenkrebs, kolorektaler Krebs, Kolonkrebs und Melanome), Lympohome (z.B. Non-Hodgkin-Lymphome und Mykosis fungoides), Leukämien, Sarkome, Mesotheliome, Himkrebs (z.B. Gliome), Germinome (z.B. Hodenkrebs und Ovarialkrebs), Choriokarzinome, Nierenkrebs, Bauchspeicheldrüsenkrebs, Schilddrüsenkrebs, Kopf- und Halskrebs, Endometrieum-Krebs, Zervix-Krebs, Blasenkrebs, Magenkrebs und multiples Myelom.

Außerdem vermittelt auf Endothelzellen exprimiertes PAR-1 Signale, die in Gefäßwachstum münden ("Angiogenese"), ein Vorgang, der zur Ermöglichung von Tumorwachstum über ca. 1 mm³ hinaus unerläßlich ist. Induktion der Angiogenese ist auch bei anderen Erkrankungen relevant, hierunter Erkrankungen des rheumatischen Formenkreises (z.B. rheumatoide Arthritis), bei Lungenerkrankungen (z.B. Lungenfibrose, pulmonale Hypertonie, insbesondere pulmonalarterielle Hypertonie, Erkrankungen, die durch Lungengefäßverschlüsse charakterisiert sind), Arteriosklerose, Plaqueruptur, diabetische Retinopathie und feuchte Makuladegeneration.

Darüber hinaus sind die erfindungsgemäßen Verbindungen zur Behandlung von Sepsis geeignet. Sepsis (oder Septikämie) ist eine häufige Erkrankung mit hoher Letalität. Anfängliche Symptome der Sepsis sind typischerweise unspezifisch (z.B. Fieber, reduziertes Allgemeinbefinden), im weiteren Verlauf kann es jedoch zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es zur endothelialen Schädigung mit Erhöhung der Gefäßpermeabilität und Austritt von Flüssigkeit und Proteinen in den Extravasalraum kommen. Im weiteren Verlauf kann es zur Dysfunktion oder dem Versagen eines Organs (z.B. Nierenversagen, Leberversagen, Atemversagen, zentralnervöse Defizite und Herz-/Kreislaufversagen) bis hin zum Multiorganversagen kommen. Hiervon kann prinzipiell jedes Organ betroffen sein, am häufigsten tritt Organdysfunktion und -Versagen bei der Lunge, der Niere, dem Herz-Kreislaufsystem, dem Gerinnungssystem, dem zentralnervösen System, endokrinen Drüsen und der Leber auf. Eine Sepsis kann mit einem "Acute Respiratory Distress Syndrome" (nachfolgend als ARDS bezeichnet) einhergehen. Ein ARDS kann auch unabhängig von einer Sepsis auftreten. "Septischer Schock" bezeichnet das Auftreten einer behandlungspflichtigen Blutdruckerniedrigung, die eine weitere Organschädigung begünstigt und mit einer Verschlechterung der Prognose einhergeht.

Krankheitserreger können Bakterien (gram-negativ und gram-positiv), Pilze, Viren und/oder Eukaryonten sein. Eintrittspforte bzw. Primärinfektion können z.B. Pneumonie, Harnwegsinfekt, Peritonitis sein. Die Infektion kann, muß aber nicht zwingend, mit einer Bakteriämie einhergehen.

Sepsis wird definiert als das Vorliegen einer Infektion und eines "systemic inflammatory response syndrome" (nachfolgend mit "SIRS" bezeichnet). SIRS tritt im Rahmen von Infekten, aber auch von anderen Zuständen wie Verletzungen, Verbrennungen, Schock, Operationen, Ischämie, Pankreatitis, Reanimation oder Tumoren auf. Nach der Definition des ACCP/SCCM Consensus Conference Committee von 1992 (Crit. Care Med. 1992, 20, 864-874) werden die zur Diagnose "SIRS" erforderlichen Symptome zur Diagnose und Meßparameter beschrieben (u.a. veränderte Körpertemperatur, erhöhte Herzfrequenz, Atemschwierigkeiten und verändertes Blutbild). In der späteren (2001) SCCM/ESICM/ACCP/ATS/SIS International Sepsis Definitions Conference wurden die Kriterien im Wesentlichen beibehalten, in Details jedoch verfeinert (Levy et al., Crit. Care Med. 2003, 31, 1250-1256).

DIC und SIRS können im Rahmen einer Sepsis, aber auch infolge von Operationen, Tumorerkrankungen, Verbrennungen oder anderen Verletzungen auftreten. Bei der DIC kommt es an der Oberfläche von geschädigten Endothelzellen, Fremdkörperoberflächen oder verletztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin, Fibrinogen) und Plättchen, wodurch die Gerinnungsfähigkeit des Blutes herabgesetzt wird und schwere Blutungen auftreten können.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, einschließlich extrakorporaler Kreisläufe, zur Beschichtung künstlicher Oberflächen von *in vivo* oder *ex vivo* eingesetzten medizinischen Hilfsmitteln und Geräten oder bei biologischen Proben, die Blutplättchen enthalten.

Weiterhin hierin beschrieben ist die Verwendung der erfindungsgemäßen Verbindungen zur Beschichtung von medizinischen Instrumenten und Implantaten, z.B. Kathetern, Prothesen, Stents oder künstlichen Herzklappen. Die erfindungsgemäßen Verbindungen können dabei fest an die Oberfläche gebunden sein oder über einen bestimmten Zeitraum aus einer Trägerbeschichtung in die unmittelbare Umgebung zur lokalen Wirkung freigesetzt werden.

weiterin hierin beschrieben ist die Verwendung der erfindungsgemäßen Verbindungen zur Behandlung und/oder Prophylaxe von Erkrankungen, insbesondere der zuvor genannten Erkrankungen.

weiterin hierin beschrieben ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

weiterin hierin beschrieben ist ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
Kalziumkanalblocker, z.B. Amlodipin Besilat (z.B. Norvasc^{®}), Felodipin, Diltiazem, Verapamil, Nifedipin, Nicardipin, Nisoldipin und Bepridil;
Iomerizin;
Statine, z.B. Atorvastatin, Fluvastatin, Lovastatin, Pitavastatin, Pravastatin, Rosuvastatin, und Simvastatin;
Cholesterinabsorption-Inhibitoren, z.B. Ezetimibe und AZD4121;
Cholesteryl Ester Transfer Protein ("CETP") Inhibitoren, z.B. Torcetrapib;
Niedrigrnolekualre Heparine, z.B. Dalteparin Natrium, Ardeparin, Certoparin, Enoxaparin, Parnaparin, Tinzaparin, Reviparin und Nadroparin;
Weitere Antikoagulanzien, z.B. Warfarin, Marcumar, Fondaparinux;
Antiarrhythmika, z.B. Dofetilid, Ibutilid, Metoprolol, Metoprololtartrat, Propranolol, Atenolol, Ajmalin, Disopyramid, Prajmalin, Procainamid, Quinidin, Spartein, Aprindine, Lidocain, Mexiletin, Tocamid, Encamid, Flecamid, Lorcamid, Moricizin, Propafenon, Acebutolol, Pindolol, Amiodaron, Bretylium-Tosylat, Bunaftin, Sotalol, Adenosin, Atropin und Digoxin;
Alpha-adrenerge Agonisten, z.B. Doxazosin-Mesylat, Terazoson und Prazosin;
Beta-adrenerge Blocker, z.B. Carvedilol, Propranolol, Timolol, Nadolol, Atenolol, Metoprolol, Bisoprolol, Nebivolol, Betaxolol, Acebutolol und Bisoprolol;
Aldosteron-Antagonisten, z.B. Eplerenon und Spironolacton;
Angiotensin-converting-enzyme Inhbitoren ("ACE-Inhibitoren"), z.B. Moexipril, Quinapril-Hydrochlorid, Ramipril, Lisinopril, Benazepril-Hydrochlorid, Enalapril, Captopril, Spirapril, Perindopril, Fosinopril und Trandolapril,;
Angiotensin II Receptorblockers ("ARBs"), z.B. Olmesartan-Medoxomil, Candesartan, Valsartan, Telmisartan, Irbesartan, Losartan und Eprosartan,;
Endothelinantagonisten, z.B. Tezosentan, Bosentan und Sitaxsentan-Natrium;
Neutral Endopeptidaseinhibitoren, z.B. Candoxatril und Ecadotril;
Phosphodiesteraseinhibitoren, z.B. Milrinoon, Theophyllin, Vinpocetin, EHNA (erythro-9-(2-hydroxy-3-nonyl)adenine), Sildenafil, Vardenafil und Tadalafil;
Fibrinolytika, z.B. Reteplase, Alteplase und Tenecteplase;
GP IIb/IIIa-Antagonisten, z.B. Integrillin, Abciximab und Tirofiban;
Direkte Thrombininhibitoren, z.B. AZD0837, Argatroban, Bivalirudin und Dabigatran;
Indirekte Thrombininhibitoren, z.B. Odiparcil;
Direkte und indirekte Faktor Xa Inhibitoren, z.B. Fondaparinux-Natrium, Apixaban, Razaxaban, Rivaroxaban (BAY 59-7939), KFA-1982, DX-9065a, AVE3247, Otamixaban (XRP0673), AVE6324, SAR377142, Idraparinux, SSR126517, DB-772d, DT-831j, YM-150, 813893, LY517717 und DU-1766.;
Direkte und indirekte Faktor Xa/IIa Inhibitoren, z.B. Enoxaparin-Natrium, AVE5026, SSR128428, SSR128429 und BIBT-986 (Tanogitran);
Lipoprotein-assoziierte Phospholipase A2 ("LpPLA2") Modulatoren;
Diuretika, z.B. Chlorthalidon, Ethacrynsäure, Furosemid, Amilorid, Chlorothiazid, Hydrochlorothiazid, Methylchtothiazid und Benzthiazid;
Nitrate, z.B. Isosorbide-5-Mononitrat;
Thromboxan-Antagonisten, z.B. Seratrodast, Picotamid und Ramatroban;
Plättchenaggregations-Inhibitoren, z.B. Clopidogrel, Tiklopidin, Cilostazol, Aspirin, Abciximab, Limaprost, Eptifibatide und CT-50547;
Cyklooxygenase-Inhibitoren, z.B. Meloxicam, Rofecoxib und Celecoxib;
B-Typ Natriuretic Peptide, z.B. Nesiritid und Ularitide;
NVIFGF-Modutatoren, z.B. XRP0038;
HT1B/5-HT2A-Antagonisten, z.B. SL65.0472;
Guanylatcyclase-Aktivatoren, z.B. Ataciguat (HMR1766), HMR1069, Riociguat und Cinaciguat;
e-NOS Transkriptions-Enhancers, z.B. AVE9488 and AVE3085;
Anti-atherogene Substanzen, z.B. AGI-1067:
CPU-Inhibitoren, z.B. AZD9684;
Renininhibitoren, z.B. Aliskirin und VNP489;
Inhibitoren der Adenosindiphosphat-induzierten Plättchenaggregation, z.B. Clopidogrel, Tiklopidin, Prasugrel, AZD6140, Ticagrelor und Elinogrel;
NHE-1 Inhibitoren, z.B. AVE4454 und AVE4890.

Antibiotische Therapie: Verschiedene Antibiotika oder antifungale Medikamenten-Kombinationen kommen in Frage, entweder als kalkulierte Therapie (vor Vorliegen des mikrobiellen Befundes) oder als spezifische Therapie; Flüssigkeitstherapie, z.B. Kristalloide oder kolloidale Flüssigkeiten; Vasopressoren, z.B. Norepinephrine, Dopamine oder Vasopressin; Inotrope Therapie, z.B. Dobutamin; Kortikosteroide, z.B. Hydrokortison, oder Fludrokortison; rekombinantes humanes aktivierte Protein C, Xigris; Blutprodukte, z.B. Erythrozytenkonzentrate, Thrombozytenkonzentrate, Erythropietin oder Fresh Frozen Plasma; Maschinelle Beatmung bei sepsisinduziertem Acute Lung Injury (ALI) bzw. Acute Respiratory Distress Syndrome (ARDS), z.B. Permissive Hyperkapnie, niedrigere Tidalvolumina; Sedierung: z.B. Diazepam, Lorazepam, Midazolam oder Propofol. Opioide: z.B. Fentanyl, Hydromorphon, Morphin, Meperidin oder Remifentanil. NSAIDs: z.B. Ketorolac, Ibuprofen oder Acetaminophen. Neuromuskuläre Blockade: z.B. Pancuronium; Glukose-Kontrolle, z.B. Insulin, Glukose; Nierenersatzverfahren, z.B. kontinuierliche veno-venöse-Hämofiltration oder intermittierende Hämodialyse. Dopamin niedrig-dosiert zur renalen Protektion; Antikoagulantien, z.B. zur Thromboseprophylaxe oder bei Nierenersatzverfahren, z.B. unfraktionierte Heparine, low molecular weight Heparine, Heparinoide, Hirudin, Bivalirudin oder Argatroban; Bikarbonat-Therapie; Streßulkusprophylaxe, z.B. H2-Rezeptorinhibitoren, Antazida

Medikamente bei proliferativen Erkrankungen: Urazil, Chlormethin, Cyklophosphamid, Ifosfamid, Melphalan, Chlorambucil, Pipobroman, Triethylenemelamin, Triethylenethiophosphoramin, Busulfan, Carmustine, Lomustine, Streptozocin, Dacarbazine, Methotrexate, 5- Fluorouracil, Floxuridine, Cytarabine, 6-Mercaptopurine, 6-Thioguanine, Fludarabine phosphate, Pentostatine, Vinblastine, Vincristine, Vindesine, Bleomycin, Dactinomycin, Daunorubicin, Doxorubicin, Epirubicin, Idarubicin, Paclitaxel, Mithramycin, Deoxycoformycin, Mitomycin-C, L-Asparaginase, Interferons, Etoposide, Teniposide 17.alpha.- Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estranrustine, Medroxyprogesteroneacetate, Leuprolide, Flutamide, Toremifene, Goserelin, Cisplatin, Carboplatin, Hydroxyurea, Amsacrine, Procarbazine, Mitotane, Mitoxantrone, Levamisole, Navelbene, Anastrazole, Letrazole, Capecitabine, Reloxafine, Droloxafine, Hexamethylmelamine, Oxaliplatin (Eloxatin^{®}), Iressa (gefmitib, Zd1839), XELODA^{®} (capecitabine), Tarceva^{®} (erlotinib), Azacitidine (5-Azacytidine; 5-AzaC), Temozolomide (Temodar^{®}, Gemcitabine (e.g., GEMZAR^{®} (gemcitabine HCl)), Vasostatin oder eine Kombination zweier oder mehrerer der oben genannten.

Weiterer Gegenstand der vorliegenden Erfindung ist ein Verfahren zur Verhinderung der Blutkoagulation *in vitro,* insbesondere bei Blutkonserven oder biologischen Proben, die Blutplättchen enthalten, das dadurch gekennzeichnet ist, dass eine antikoagulatorisch wirksame Menge der erfindungsgemäßen Verbindung zugegeben wird.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutische geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 100 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Votumen). So bedeutet beispielsweise "10% w/v": 100 ml Lösung oder Suspension enthalten 10 g Substanz.

### A) Beispiele

### Abkürzungen:

- ca.: circa
- CDI: Carbonyldiimidazol
- d: Tag(e), Dublett (bei NMR)
- DC: Dünnschicht-Chromatographie
- DCI: direkte chemische Ionisation (bei MS)
- dd: Doppeltes Dublett (bei NMR)
- DMAP: 4-Dimethylaminopyridin
- DMF: *N*,*N*-Dimethylformamid
- DMSO: Dimethylsulfoxid
- DPPA: Diphenylphosphorazidat
- DSC: Disuccinimidylcarbonat
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)-*N*,*N*,*N*',*N*'-tetramethyluronium-Hexafluorphosphat
- HPLC: Hochdruck-, Hochleistungsflüssigchromatographie
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- PYBOP: Benzotriazol-1-yloxy-tris(pyrrolidino)phosphonium-Hexafluorophosphat
- q: Quartett (bei NMR)
- RP: reverse phase (bei HPLC)
- RT: Raumtemperatur
- Rₜ: Retentionszeit (bei HPLC)
- s: Singulett (bei NMR)
- t: Triplett (bei NMR)
- THF: Tetrahydrofuran

### HPLC-Methoden:

Methode 1A: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

Methode 2A: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 9 min 0% B → 9.2 min 2% B → 10 min 2% B; Fluss: 0.75 ml/min; Säulentemperatur: 30°C; UV-Detektion: 210 nm.

Methode 3A: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 15 min 90% B →15.2 min 2% B → 16 min 2% B; Fluss: 0.75 ml/min; Säulentemperatur: 30°C; UV-Detektion: 210 nm.

Methode 4A: Phase: Kromasil 100, C18, 5 µm, 250 mm x 4 mm; Eluent: Wasser/Acetonitril 50:50; Fluss: 1 ml/min; T: 40°C; UV: 210 nm.

### LC-MS-Methoden:

Methode 1B: Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 1.5 min 10% A → 2.2 min 10% A; Ofen: 50°C; Fluss: 0.33 ml/min; UV-Detektion: 210 nm.

Methode 2B: Gerätetyp MS: Waters ZQ; Gerätetyp HPLC: Agilent 1100 Series; UV DAD; Säule: Thermo Hypersil GOLD 3 µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100% A → 3.0 min 10% A → 4.0 min 10% A → 4.1 min 100%; Ofen: 55°C; Fluss: 2 ml/min; UV-Detektion: 210 nm.

Methode 3B: Gerätetyp MS: Micromass ZQ; Gerätetyp HPLC: HP 1100 Series; UV DAD; Säule: Phenomenex Gemini 3 µ 30 mm x 3.00 mm; Eluent A: 11 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min. 2 ml/min; Ofen: 50°C; UV-Detektion: 210 nm.

Methode 4B: Gerätetyp MS: Micromass ZQ; Gerätetyp HPLC: Waters Alliance 2795; Säule: Phenomenex Synergi 2.5µMAX-RP 100A Mercury 20 mm x 4 mm; Eluent A: 1 1 Wasser + 0.5 ml 50%ige Ameisensäure, Eluent B: 11 Acetonitril + 0.5 ml 50%ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

Methode 5B: Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%ige Ameisensäure, Eluent B: 1 1 Acetonitril + 0.5ml 50%ige Ameisensäure; Gradient: 0.0 min 100% A - 0.2 min 100% A → 2.9 min 30% A → 3.1 min 10% A → 5.5 min 10% A; Ofen: 50°C; Fluss: 0.8 ml/min; UV-Detektion: 210 nm.

Methode 6B: Instrument: Micromass Quattro LCZ mit HPLC Agilent Serie 1100; Säule: Phenomenex Synergi 2.5 µMAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208- 400 nm.

Methode 7B: Gerätetyp MS: Waters (Micromass) Quattro Micro; Gerätetyp HPLC: Agilent 1100 Serie; Säule : Thermo Hypersil GOLD 3 µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100% A → 3.0 min 10% A → 4.0 min 10% A → 4.01 min 100% A (Flow 2.5 ml) → 5.00 min 100% A; Ofen: 50°C; Fluss: 2 ml/min; UV-Detektion: 210 nm

Methode 8B: Instrument: Waters ACQUITY SQD UPLC System; Säule: Waters Acquity UPLC HSS T3 1.8 µ50 mm x 1 mm; Eluent A: 1 1 Wasser + 0.25 ml 99%ige Ameisensäure, Eluent B: 1 1 Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 210 - 400 nm.

### Präparative Diastereomerentrennung:

Methode 1C: Phase: Kromasil 100 C18, 5 µm 250 mm x 20 mm, Eluent: Wasser/Acetonitril 50:50; Fluss: 25 ml/min, Temperatur: 40°C; UV-Detektion: 210 nm.

Methode 2C: Phase: Sunfire C18, 5 µm OBD 19 mm x 150 mm, Eluent: Wasser/Acetonitril 62:38; Fluss: 25 ml/min, Temperatur: 40°C; UV-Detektion: 210 nm.

### Präparative Enantiomerentrennung:

Methode 1D: Phase: Daicel Chiralpak AD-H, 5 µm 250 mm x 20 mm; Eluent: iso-Hexan/Ethanol 50:50; Fluss: 1 5 ml/min; Temperatur: 40°C; UV-Detektion: 220 nm.

Methode 2D: Phase: Daicel Chiralpak AD-H, 5 µm 250 mm x 20 mm, Eluent: iso-Hexan/Ethanol 55:45; Fluss: 1 5 ml/min, Temperatur: 40°C; UV-Detektion: 220 nm.

Methode 3D: Phase: Daicel Chiralpak AD-H, 5 µm 250 mm x 20 mm, Eluent: iso-Hexan/Ethanol 80:20; Fluss: 15 5 ml/min, Temperatur: 40°C; UV-Detektion: 220 nm.

Methode 4D: Phase: Daicel Chiralcel OD-H, 5 µm 250 mm x 20 mm, Eluent: iso-Hexan/Ethanol 90:10; Fluss: 1 5 ml/min, Temperatur: 30°C; W-Detektion: 220 nm.

Methode 5D: Phase: Daicel Chiralpak AD-H, 5 µm 250 mm x 20 mm, Eluent: tert-Butylmethylether/Methanol 90:10; Fluss: 15 ml/min, Temperatur: 30°C; UV-Detektion: 220 nm.

### Analytische Enantiomerentrennung:

Methode 1E: Phase: Daicel Chiralpak AD-H, 5 µm 250 mm x 4.6 mm; Eluent: iso-Hexan/Ethanol 50:50; Fluss: 1 ml/min; Temperatur: 40°C; UV-Detektion: 220 nm.

Methode 2E: Phase: Daicel Chiralpak AS-H, 5 µm 250 mm x 4.6 mm; Eluent: iso-Hexan/Ethanol 50:50; Fluss: 1 ml/min; Temperatur: 40°C; UV-Detektion: 220 nm.

Methode 3E: Phase: Daicel Chiralpak OD-H, 5 µm 250 mm x 4.6 mm; Eluent: iso-Hexan/Ethanol 85:15; Fluss: 1 ml/min; Temperatur: 40°C; UV-Detektion: 220 nm.

Methode 4E: Phase: Daicel Chiralpak AD-H, 5 µm 250 mm x 4 mm; Eluent: tert-Butylmethylether/Methanol: 90:10; Fluss: 1 ml/min; Temperatur: 25°C; UV-Detektion: 220 nm.

Als Mikrowellenreaktor wurde ein "single mode" Gerät vom Typ Emrys^{™} Optimizer verwendet.

### Ausganesverbindungen

### Allgemeine Methode 1A: Suzuki-Kupplung

Eine Mischung des entsprechenden Brompyridins in Toluol (1.8 ml/mmol) wird unter Argon bei RT mit Tetrakis-(triphenylphosphin)-palladium (0.02 eq.), mit einer Lösung der entsprechenden Arylboronsäure (1.2 eq.) in Ethanol (0.5 ml/mmol) und mit einer Lösung aus Kaliumfluorid (2.0 eq.) in Wasser (0.2 ml/mmol) versetzt. Das Reaktionsgemisch wird mehrere Stunden bis zur weitgehend vollständigen Umsetzung unter Rückfluß gerührt. Nach Zugabe von Essigsäureethylester und Phasentrennung wird die organische Phase einmal mit Wasser und einmal mit gesättigter, wässriger Natriumchlorid-Lösung gewaschen, getrocknet (Magnesiumsulfat), filtriert und im Vakuum eingeengt. Das Rohprodukt wird mittels Flash-Chromatographie (Kieselgel-60, Eluent: Dichlormethan-Methanol-Gemische) aufgereinigt.

### Allgemeine Methode 2A: Hydrierung des Pyridins

Eine Lösung des Pyridins in Ethanol (9 ml/mmol) wird unter Argon mit Palladium auf Aktivkohle (angefeuchtet mit ca. 50% Wasser, 0.3 g/mmol) versetzt und bei 60°C über Nacht in einer 50 bar Wasserstoff-Atmosphäre hydriert. Anschließend wird der Katalysator über eine Filterschicht abfiltriert und mehrmals mit Ethanol gewaschen. Die vereinigten Filtrate werden im Vakuum eingeengt.

### Allgemeine Methode 3A: Umsetzung mit Carbamoylchloriden oder Carbonylchloriden

Eine Lösung des Piperidins in Dichlormethan (2.5 ml/mmol) wird unter Argon bei 0°C tropfenweise mit *N*,*N*-Diisopropylethylamin (1.2 eq.) und dem entsprechenden Carbamoylchlorid oder Carbonylchlorid (1.2 eq.) versetzt. Das Reaktionsgemisch wird bei RT gerührt. Nach Zugabe von Wasser und Phasentrennung wird die organische Phase dreimal mit Wasser und einmal mit gesättigter, wässriger Natriumchlorid-Lösung gewaschen, getrocknet (Natriumsulfat), filtriert und im Vakuum eingeengt.

### Allgemeine Methode 4A: Verseifung

Eine Lösung des entsprechenden Esters in einem Gemisch aus Tetrahydrofuran/Wasser (3:1, 12.5 ml/mmol) wird bei RT mit Lithiumhydroxid (2 eq.) versetzt. Das Reaktionsgemisch wird bei 60°C gerührt und anschließend mit wässriger, 1 N Salzsäure-Lösung auf pH 1 gestellt. Nach Zugabe von Wasser/Essigsäureethylester wird die wässrige Phase dreimal mit Essigsäureethylester extrahiert. Die vereinigten organischen Phasen werden getrocknet (Natriumsulfat), filtriert und im Vakuum eingeengt.

### Allgemeine Methode 5A: Hydrierung des Pyridins mittels Durchfluss-Hydrierapparatur

Eine Lösung des Pyridins in konzentrierter Essigsäure (ca. 35 ml/mmol) wird in einer Durchfluss-Hydrierapparatur ("H-Cube" der Firma ThalesNano, Budapest, Ungarn) hydriert (Bedingungen: 10% Pd/C-Katalysator, "controlled"-Modus, 60 bar, 0.5 ml/min, 85°C). Nach Entfernen des Lösungsmittels am Rotationsverdampfer wird das entsprechende Rohprodukt erhalten, welches gegebenenfalls mittels präparativer HPLC gereinigt wird.

### Allgemeine Methode 6A: Methylesterverseifung/Epimerisierung

Zu einer Lösung des entsprechenden Methylesters (1.0 eq.) in Methanol (35-40 ml/mmol) wird bei RT Kalium-*tert*.-butylat (10 eq.) gegeben. Die Mischung wird über Nacht bei 60°C gerührt. Bei unvollständiger Umsetzung wird Wasser (1.0 eq.) zugesetzt und bis zum vollständigen Umsatz bei 60°C gerührt. Zur Aufarbeitung wird im Vakuum das Methanol entfernt, der Rückstand mit Wasser versetzt und mit 1N Salzsäure auf pH=1 gestellt. Die Mischung wird mit Essigsäureethylester extrahiert, die organische Phase über Magnesiumsulfat getrocknet, filtriert und im Vakuum eingeengt.

### Beispiel 1A

### Methyl-(5-brompyridin-3-yl)acetat

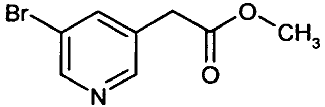

Eine Lösung von 3.0 g (13.8 mmol) 5-Brom-3-pyridylessigsäure in 150 ml THF wurde mit 3.3 g (30.8 mmol) *O*-Methyl-*N,N*'-diisopropylisoharnstoff versetzt. Das Reaktionsgemisch wurde 2 Stunden bei Rückflußtemperatur gerührt. Zur Aufarbeitung wurde im Vakuum Tetrahydrofuran entfernt, das Reaktionsgemisch mit Essigsäureethylester versetzt und mit Wasser und gesättigter, wässriger Natriumhydrogencarbonat-Lösung gewaschen. Die organische Phase wurde über Magnesiumsulfat getrocknet und im Vakuum eingeengt. Man erhielt so 4.2 g Rohprodukt in 74%-iger Reinheit (LC-MS), welches ohne weitere Reinigungsoperationen umgesetzt wurde.

LC-MS (Methode 1B): Rₜ = 0.83 min; MS (ESIpos): m/z = 231 [M+H]⁺.

### Beispiel 2A

### Methyl-{5-[4-(trifluormethyl)phenyl]pyridin-3-yl}acetat

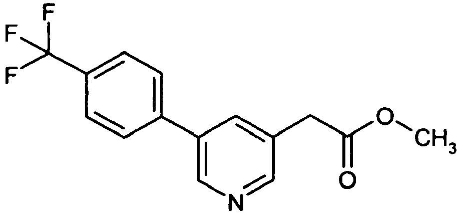

Eine Mischung von 5.5 g (17.9 mmol) Methyl-(5-brompyridin-3-yl)acetat in *N*,*N*'-Dimethylformamid/1,2-Dimethoxyethan (6.3 ml2.5 ml/mmol) wurde unter Argon bei Raumtemperatur mit 206 mg (0.18 mmol) Tetrakis(triphenylphosphin)palladium, 5.11 g (26.9 mmol) [4-(Trifluormethyl)phenyl]boronsäure, 3.80 g (35.86 mmol) Natriumcarbonat und 9 ml Wasser versetzt. Das Reaktionsgemisch wurde über Nacht bei 85°C gerührt. Zur Aufarbeitung wurde das Reaktionsgemisch eingeengt, mit Essigsäureethylester versetzt und mit Wasser gewaschen. Die organische Phase wurde über Magnesiumsulfat getrocknet und im Vakuum eingeengt. Der Rückstand wurde mittels präparativer HPLC aufgereinigt. Ausbeute: 850 mg (15% d. Th.)

LC-MS (Methode 4B): Rₜ = 1.79 min; MS (ESIpos): m/z = 296 [M+H]⁺.

### Beispiel 3A

### Methyl-{5-[4-(trifluormethyl)phenyl]piperidin-3-yl}acetate-Hydroacetat [racemisches cis-/trans-Isomerengemisch]

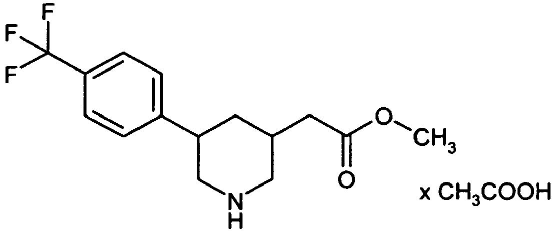

Nach der Allgemeinen Methode 2A wurden 850 mg (2.88 mmol) der Verbindung aus Beispiel 2A hydriert. Ausbeute: 5.46 g (100% d. Th.)

LC-MS (Methode 4B): Rₜ = 1.00 und 1.04 min (cis-/trans-Isomere); MS (ESIpos): m/z = 302 [M+H-AcOH]⁺.

### Beispiel 4A

### Methyl-{1-(morpholin-4-ylcarbonyl)-5-[4-(trifluormethyl)phenyl]piperidin-3-yl}acetat [racemisches cis-/trans-Isomerengemisch]

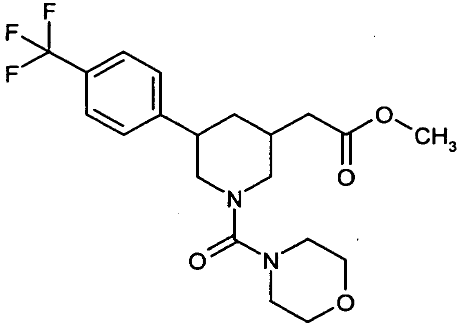

Nach der Allgemeinen Methode 3A wurden 1.16 g (3.21 mmol) der Verbindung aus Beispiel 3A mit 0.96 g (6.42 mmol) Morpholin-4-carbonylchlorid umgesetzt. Man erhielt so 850 mg Rohprodukt in 84%-iger Reinheit (LC-MS), welches ohne weitere Reinigungsoperationen umgesetzt wurde.

LC-MS (Methode 1B): Rₜ = 1.22 und 1.24 min (cis-/trans-Isomere); MS (ESIpos): m/z = 415 [M+H]⁺.

### Beispiel 5A

### {1-(Morpholin-4-ylcarbonyl)-5-[4-(trifluormethyl)phenyl]piperidin-3-yl}essigsäure [racemisches cis-/trans-Isomerengemisch]

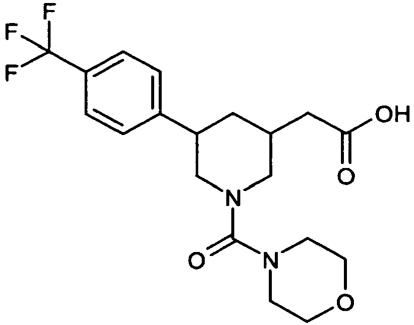

Nach der Allgemeinen Methode 4A wurden 850 mg (2.1 mmol) der Verbindung aus Beispiel 4A mit 98 mg (4.1 mmol) Lithiumhydroxid umgesetzt. Man erhielt so 1.0 g Rohprodukt in 88%-iger Reinheit (LC-MS), welches ohne weitere Reinigungsoperationen umgesetzt wurde.

LC-MS (Methode 3B): Rₜ = 2.10 min; MS (ESIpos): m/z = 401 [M+H]⁺.

### Beispiel 6A

### 1-(Morpholin-4-ylcarbonyl)-5-[4-(trifluormethyl)phenyl]piperidin-3-hydroxymethyl [racemisches cis-/trans-Isomerengemisch]

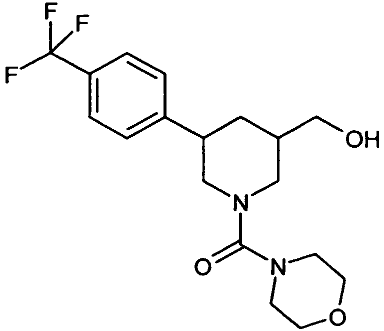

Zu einer auf 0°C gekühlten Suspension von 1.19 g (31.5 mol) Natriumborhydrid in 7 ml THF wurden 5.0 g (ca. 10.5 mmol) 1-(Morpholin-4-ylcarbonyl)-5-[4-(trifluormethyl)phenyl]piperidin-3-carbonsäure [racemisches cis-/trans-Isomerengemisch], gelöst in 8 ml THF, zugetropft. Anschließend wurden 6 ml (47.2 mmol) Bortrifluorid-Diethylether-Komplex hinzugegeben. Nach beendeter Zugabe wurde die Lösung bei Raumtemperatur über Nacht gerührt. Das Reaktionsgemisch wurde unter Eiskühlung mit einer 1N Salzsäure-Lösung versetzt, mit Wasser verdünnt, und die wässrige Phase wurde mit Dichlormethan extrahiert. Die organische Phase wurde über Magnesiumsulfat getrocknet, filtriert und im Vakuum eingeengt. Man erhielt so 3.7 g Rohprodukt, welches ohne weitere Reinigungsoperationen umgesetzt wurde.

LC-MS (Methode 4B): Rₜ = 1.64 min; MS (ESIpos): m/z = 373 [M+H]⁺.

### Beispiel 7A

### 1-(Morpholin-4-ylcarbonyl)-5-[4-(trifluormethyl)phenyl]piperidin-3-methansulfonat [racemisches cis-/trans-Isomerengemisch]

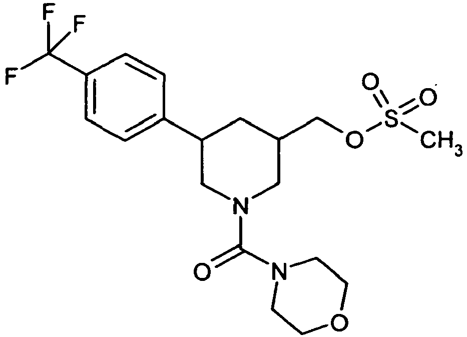

Eine auf 0°C gekühlte Lösung von 3.59 g (9.65 mmol) der Verbindung aus Beispiel 6A in 68 ml Dichlormethan wurde mit 2.21 g (19.30 mmol) Methansulfonsäurechlorid und 2.7 ml (19.30 mmol) Triethylamin versetzt und für 16 h bei Raumtemperatur gerührt. Das Reaktionsgemisch wurde mit Wasser versetzt, die organische Phase abgetrennt und mit Wasser gewaschen, über Magnesiumsulfat getrocknet und im Vakuum eingeengt. Der Rückstand wurde nochmal in 120 ml Dichlormethan gelöst, auf 0°C gekühlt und mit 13.5 ml (96.48 mmol) Triethylamin und 118 mg (0.96 mmol) Dimethylaminopyridin versetzt. Anschließend wurden 2.21 g (19.30 mmol) Methansulfonsäurechlorid zugegeben, und die Mischung wurde 1 h bei Raumtemperatur gerührt. Das Reaktionsgemisch wurde mit Wasser versetzt, die organische Phase abgetrennt und mit Wasser gewaschen, über Magnesiumsulfat getrocknet und im Vakuum eingeengt. Man erhielt so 2.2 g Rohprodukt in 74%-iger Reinheit (LC-MS), welches ohne weitere Reinigungsoperationen umgesetzt wurde.

LC-MS (Methode 4B): Rₜ = 1.88 min; MS (ESIpos): m/z = 451 [M+H]⁺.

### Beispiel 8A

### {3-(Azidomethyl)-5-[4-(trifluormethyl)phenyl]piperidin-1-yl}(morpholin-4-yl)methanon [racemisches cis-/trans-Isomerengemisch]

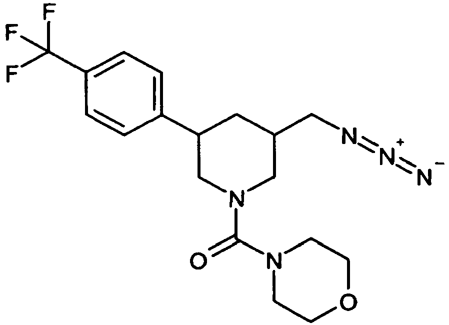

Eine Lösung von 3.6 g (ca. 6.47 mmol) der Verbindung aus Beispiel 7A in 220 ml *N*,*N-*Dimethylformamid wurde unter Argon mit 420 mg (6.47 mmol) Natriumazid versetzt und über Nacht bei 70°C gerührt. Ein Großteil des Lösungsmittels wurde im Vakuum abdestilliert und der Rückstand mit Essigsäureethylester verdünnt. Es wurde mehrmals mit gesättigter, wässriger Natriumhydrogencarbonat-Lösung gewaschen, über Magnesiumsulfat getrocknet, filtriert und im Vakuum zur Trockne eingeengt. Man erhielt so 2.55 g Rohprodukt in 78%-iger Reinheit (LC-MS), welches ohne weitere Reinigungsoperationen umgesetzt wurde.

LC-MS (Methode 3B): Rₜ = 2.44 min; MS (ESIpos): m/z = 398 [M+H]⁺.

### Beispiel 9A

### {3-(Aminomethyl)-5-[4-(trifluormethyl)phenyl]piperidin-1-yl}(morpholin-4-yl)methanon [racemisches cis-/trans-Isomerengemisch]

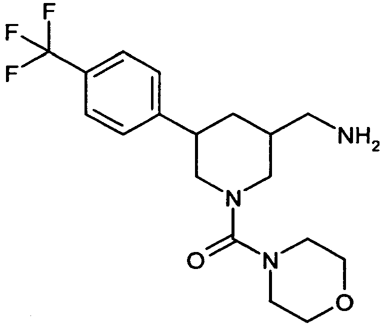

Eine Lösung von 2.0 g (ca. 5.03 mmol) der Verbindung aus Beispiel 8A in 70 ml Ethanol wurde nach Zugabe von 536 mg Palladium auf Aktivkohle (50%ig) 24 Stunden bei Raumtemperatur und Normaldruck hydriert. Es wurde über Kieselgur filtriert und der Rückstand mit Ethanol gewaschen. Das Filtrat wurde im Vakuum zur Trockne eingeengt. Man erhielt so 1.79 g Rohprodukt in 63%-iger Reinheit (LC-MS), welches ohne weitere Reinigungsoperationen umgesetzt wurde.

LC-MS (Methode 3B): Rₜ = 1.29 min; MS (ESIpos): m/z = 372 [M+H]⁺.

### Beispiel 10A

### 5-[4-(Trifluormethyl)phenyl]pyridin-3-carbonsäuremethylester

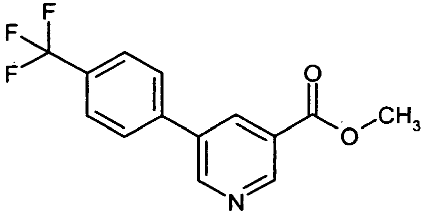

Nach der Allgemeinen Methode 1A wurden 28 g (132 mmol) 5-Bromnicotinsäuremethylester und 30 g (158 mmol, 1.2 eq.) 4-Trifluormethylphenylboronsäure umgesetzt. Ausbeute: 32 g (85% d. Th.)

LC-MS (Methode 4B): Rₜ = 2.27 min; MS (ESIpos): m/z = 282 [M+H]⁺.

### Beispiel 11A

### 5-[4-(Trifluormethyl)phenyl]piperidin-3-carbonsäuremethylester [racemisches cis-/trans-Isomerengemisch]

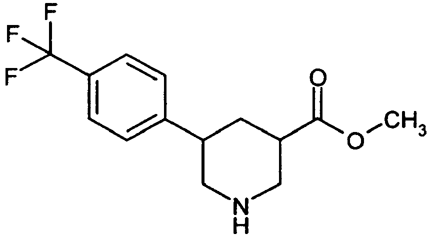

Nach der Allgemeinen Methode 2A wurden 32 g (112 mmol) 5-[4-(Trifluormethyl)phenyl]-pyridin-3-carbonsäuremethylester hydriert. Ausbeute: 26 g (82% d. Th.)

LC-MS (Methode 3B): Rₜ = 1.35 und 1.41 min (cis-/trans-Isomere); MS (ESIpos): m/z = 288 [M+H]⁺.

### Beispiel 12A

### 1-(Morpholin-4-ylcarbonyl)-5-[4-(trifluormethyl)phenyl]piperidin-3-carbonsäuremethylester [racemisches cis-/trans-Isomerengemisch]

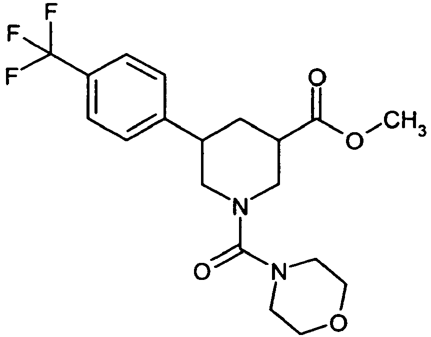

Nach der Allgemeinen Methode 3A wurden 9.25 g (32.2 mmol) 5-[4-(Trifluormethyl)phenyl]-piperidin-3-carbonsäuremethylester mit 9.63 g (64.7 mmol) Morpholin-4-carbonylchlorid umgesetzt. Man erhielt so 16.3 g Rohprodukt in 76%-iger Reinheit (LC-MS), welches ohne weitere Reinigungsoperationen umgesetzt wurde.

LC-MS (Methode 1B): Rₜ = 1.19 und 1.22 min (cis-/trans-Isomere); MS (ESIpos): m/z = 401 [M+H]⁺.

### Beispiel 13A

### 1-(Morpholin-4-ylcarbonyl)-5-[4-(trifluormethyl)phenyl]piperidin-3-carbonsäure [racemisches cis-Isomer]

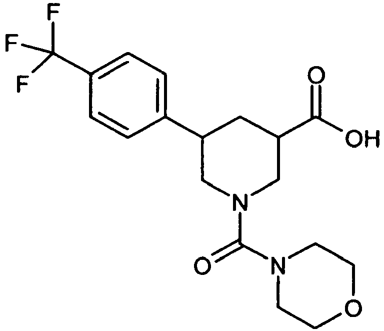

Nach der Allgemeinen Methode 4A wurden 22.19 g (39.90 mmol) der Verbindung aus Beispiel 12A und 44.78 g (399.0 mmol) Kalium-tert.-butylat umgesetzt. Ausbeute: 18.29 g (100% d. Th.)

LC-MS (Methode 7B): Rₜ = 1.95 min; MS (ESIpos): m/z = 387 [M+H]⁺.

### Beispiel 14A

### Methyl-1-[(1,1-dioxidothiomorpholin-4-yl)carbonyl]-5-[4-(trifluormethyl)phenyl]piperidin-3-carboxylat [racemisches cis-/trans-Isomerengemisch]

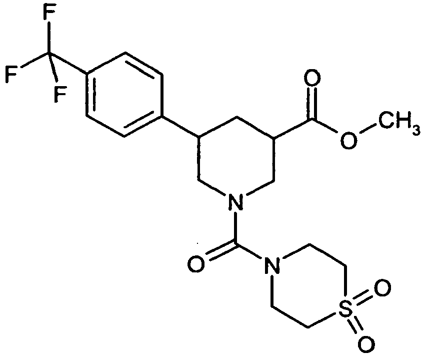

5.19 g (18.1 mmol) 5-[4-(Trifluormethyl)phenyl]piperidin-3-carbonsäuremethylester wurden in 240 ml *N*-Methyl-2-pyrrolidon gelöst und bei 150°C mit 5.42 g (18.1 mmol) 4-Nitrophenylthiomorpholin-4-carboxylat-1,1-dioxid sowie 2.75 g (18.1 mmol) 1,8-Diazabicyclo[5.4.0]undec-7-en versetzt. Die Reaktionsmischung wurde für 4.5 h gerührt. Zur Aufarbeitung wurde das *N*-Methyl-2-pyrrolidon im Vakuum entfernt, der Rückstand mit Essigsäureethylester versetzt und mit wässriger 1N Natriumhydroxid-Lösung gewaschen. Die organische Phase wurde über Magnesiumsulfat getrocknet, filtriert und im Vakuum eingeengt. Man erhielt so 7.61 g Rohprodukt in 71%-iger Reinheit (LC-MS), welches ohne weitere Reinigungsoperationen umgesetzt wurde.

LC-MS (Methode 8B): Rₜ = 0.94 und 0.96 min; MS (ESIpos): m/z = 449 [M+H]⁺.

### Beispiel 15A

### 1-[(1,1-Dioxidothiomorpholin-4-yl)carbonyl]-5-[4-(trifluormethyl)phenyl]piperidin-3-carbonsäure [racemisches cis-Isomer]

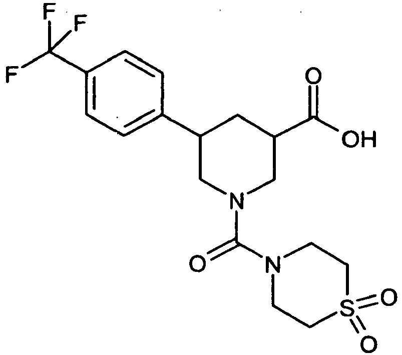

Nach der Allgemeinen Methode 6A wurden 640 mg (1.09 mmol) der Verbindung aus Beispiel 14A mit 1.23 g (10.1 mmol) Kalium-*tert*-butylat umgesetzt. Ausbeute: 110 mg (21% d. Th.)

LC-MS (Methode 8B): Rₜ = 1.01 min; MS (ESIpos): m/z = 435 [M+H]⁺.

### Beispiel 16A

### 1,1-Dioxidothiomorpholin-4-yl){3-(hydroxymethyl)-5-[4-(trifluormethyl)phenyl]piperidin-1-yl}-methanon [racemisches cis-Isomer]

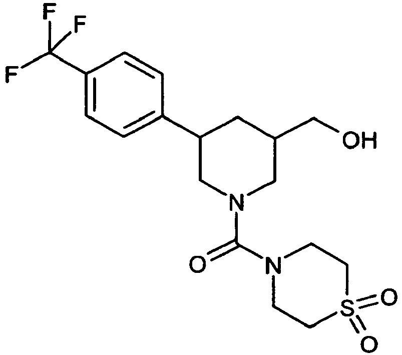

Zu einer auf 0°C gekühlten Suspension von 19 mg (0.51 mmol) Natriumborhydrid in 24 ml THF wurden 110 mg (0.253 mmol) der Verbindung aus Beispiel 15A gelöst in 12 ml THF zugetropft. Anschließend wurden 0.086 ml (0.68 mmol) Bortrifluorid-Diethylether-Komplex hinzugegeben. Die Reaktionsmischung wurde 0.5 h bei 0°C und danach 2 h bei RT gerührt. Zur Aufarbeitung wurde das Reaktionsgemisch unter Eiskühlung mit einer 1N Salzsäure-Lösung versetzt, mit Wasser verdünnt und die wässrige Phase wird mit Dichlormethan extrahiert. Die organische Phase wurde über Magnesiumsulfat getrocknet, filtriert und im Vakuum eingeengt. Man erhielt so 130 mg Rohprodukt in 76%-iger Reinheit (LC-MS), welches ohne weitere Reinigungsoperationen umgesetzt wurde.

LC-MS (Methode 8B): Rₜ = 0.83 min; MS (ESIpos): m/z = 421 [M+H]⁺.

### Ausführungsbeispiele

### Allgemeine Methode 1: Sulfonamidbildung

Eine Lösung aus dem entsprechenden Amin (1.0 eq.) in Dichlormethan (21 ml/mmol) wird bei RT mit N,N-Diisopropylethylamin (2.5 eq.) und dem entsprechenden Sulfonsäurechlorid (1.5 eq.) versetzt. Das Reaktionsgemisch wird über Nacht bei Raumtemperatur gerührt. Zur Aufarbeitung wird Dichlormethan im Vakuum entfernt und der Rückstand mittels präparativer HPLC gereinigt.

### Allgemeine Methode 2: Amidbildung 1

Eine Lösung aus dem entsprechenden Amin (1.0 eq.) in Dichlormethan (21 ml/mmol) wird bei Raumtemperatur mit N,N-Diisopropylethylamin (2.5 eq.) und dem entsprechenden Carbonsäurechlorid (1.2 eq.) versetzt. Das Reaktionsgemisch wird über Nacht bei Raumtemperatur gerührt. Zur Aufarbeitung wird Dichlormethan im Vakuum entfernt und der Rückstand mittels präparativer HPLC gereinigt.

### Allgemeine Methode 3: Amidbildung 2

Eine Lösung aus 1.0 Äquivalenten der entsprechenden Carbonsäure in Dimethylformamid wird unter Argon bei Raumtemperatur mit HATU (1.5 eq.) und *N*,*N*-Diisopropylethylamin (2.5 eq.) versetzt. Nach 30 Minuten werden 1.1 Äquivalenten des entsprechenden Amins zugegeben. Das Reaktionsgemisch wird 16 h bei Raumtemperatur gerührt. Das Reaktionsgemisch wird eingeengt, und der Rückstand wird mittels präparativer HPLC aufgereinigt.

### Allgemeine Methode 4: Amidbildung 3

Eine Lösung aus 1.0 Äquivalenten der entsprechenden Carbonsäure in Tetrahydrofuran wird unter Argon bei RT mit PYBOP (1.5 eq.) und N,N-Diisopropylethylamin (7.0 eq.) versetzt. Nach 40 Minuten werden 1.2 Äquivalente des entsprechenden Amins zugegeben. Das Reaktionsgemisch wird 16 h bei RT gerührt. Das Reaktionsgemisch wird eingeengt, der Rückstand in Essigsäureethylester aufgenommen, mehrmals mit Wasser sowie einer gesättigten, wässrigen Natriumchlorid-Lösung gewaschen, über Magnesiumsulfat getrocknet, filtriert und im Vakuum eingeengt. Der Rückstand wird mittels präparativer HPLC gereinigt.

### Allgemeine Methode 5: Carbamatbildung 1

Eine Lösung aus 1.0 Äquivalenten eines Alkohols in Dichlormethan (2 ml/0.20 mmol) wird mit einer 4 N Chlorwasserstoff-Dioxan-Lösung (0.5 eq.) und dem entsprechenden Isocyanat (1.2 eq.) versetzt. Das Reaktionsgemisch wird 16 h bei RT gerührt. Das Reaktionsgemisch wird eingeengt und anschließend mittels präparativer HPLC aufgereinigt.

### Allgemeine Methode 6: Carbamatbildung 2

Eine Lösung aus dem entsprechenden Amin (1.0 eq.) in Dioxan (15ml/mmol) wird bei RT mit 4-Dimethylaminopyridin (0.1 eq.) und dem entsprechenden Isocyanat (1.2 eq.) versetzt. Das Reaktionsgemisch wird über Nacht bei Raumtemperatur gerührt. Zur Aufarbeitung wird Dioxan im Vakuum entfernt und der Rückstand mittels präparativer HPLC gereinigt.

### Allgemeine Methode 7: Amine

Eine Lösung aus 1.0 Äquivalenten eines Mesylates in *N*,*N*-Dimethylformamid (27 ml/mmol) wird mit Molsieb vorgelegt und unter Argon mit dem entsprechende Amin versetzt. Das Reaktionsgemisch wird 16 h bei 120°C gerührt. Das Reaktionsgemisch wird eingeengt, in Essigsäureethylester aufgenommen, mehrmals mit einer gesättigten, wässrigen Natriumhydrogencarbonat-Lösung gewaschen, über Magnesiumsulfat getrocknet, filtriert und im Vakuum eingeengt. Der Rückstand wird mittels präparativer HPLC aufgereinigt.

### Allgemeine Methode 8: Amine

Die Lösung eines Mesylates (1.0 eq.) in *N*,*N*'-Dimethylformamid (10 ml/mmol) wird mit dem entsprechenden Amin (12.0 eq.) versetzt und in einer Single Mode-Mikrowelle (Emrys Optimizer) für 0.5 h bei 150°C gerührt. Das Reaktionsgemisch wird eingeengt, in Essigsäureethylester aufgenommen, mehrmals mit einer gesättigten wässrigen Natriumhydrogencarbonat-Lösung gewaschen, über Magnesiumsulfat getrocknet, filtriert und im Vakuum eingeengt. Der Rückstand wird mittels präparativer HPLC gereinigt.

### Beispiel 1

### 2,2-Dimethyl-N-({1-(morpholin-4-ylcarbonyl)-5-[4-(trifluormethyl)phenyl]piperidin-3-yl}methyl)propanamid [racemisches cis-Isomer]

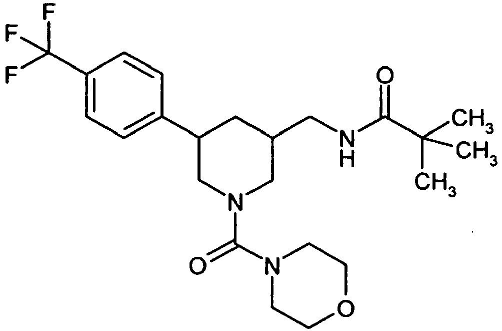

Nach der Allgemeinen Methode 2 wurden 123 mg (ca. 0.21 mmol) der Verbindung aus Beispiel 9A und 30 mg (0.25 mmol) Trimethylacetylchlorid umgesetzt. Ausbeute: 31 mg (32% d. Th.)

LC-MS (Methode 3B): Rₜ = 2.24 min; MS (ESIpos): m/z = 456 [M+H]⁺;

¹H-NMR (400 MHz, DMSO-d₆): δ = 7.68 (d, 2H), 7.59 (t, 1H), 7.51 (d, 2H), 3.63 (br d, 2H), 3.57-3.53 (m, 4H), 3.19-3.08 (m, 5H), 3.04-2.91 (m, 2H), 2.85-2.71 (m, 2H), 1.86 (br d, 1H), 1.77 (m, 1H), 1.34 (q, 1H), 1.09 (s, 9H).

### Beispiel 2

### 3-Chlor-N-({1-(morpholin-4-ylcarbonyl)-5-[4-(trifluormethyl)phenyl]piperidin-3-yl}methyl)-benzolcarboxamid [racemisches cis-Isomer]

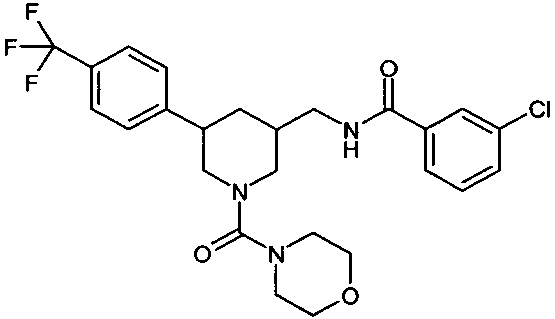

Nach der Allgemeinen Methode 2 wurden 100 mg (ca. 0.10 mmol) der Verbindung aus Beispiel 9A und 29 mg (0.17 mmol) 3-Chlorbenzoylchlorid umgesetzt. Ausbeute: 5 mg (8% d. Th.)

LC-MS (Methode 1B): Rₜ = 1.30 min; MS (ESIpos): m/z = 510 [M+H]⁺;

¹H-NMR (400 MHz, DMSO-d₆): δ = 8.7 (t, 1H); 7.89 (s, 1H), 7.81 (d, 1H), 7.68 (d, 2H), 7.61 (br d, 1H), 7.55-7.50 (m, 3H), 3.70 (br d, 1H), 3.63 (d, 1H), 3.51-3.47 (m, 4H), 3.25-3.18 (m, 2H), 3.13-3.05 (m, 5H), 2.91-2.79 (m, 2H), 1.96-1.85 (m, 2H), 1.44 (q, 1H).

### Beispiel 3

### 2-(Methylsulfonyl)-N-({1-(morpholin-4-ylcarbonyl)-5-[4-(trifluormethyl)phenyl]piperidin-3-yl}methyl)acetamid [racemisches cis-Isomer]

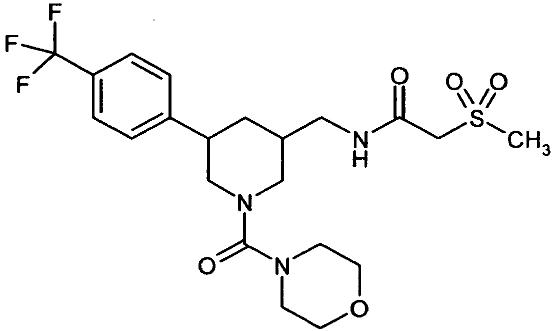

Nach der Allgemeinen Methode 3 wurden 88 mg (ca. 0.15 mmol) der Verbindung aus Beispiel 9A und 19 mg (0.14 mmol) (Methylsulfonyl)essigsäure umgesetzt. Diastereomerentrennung von 38 mg des Rückstands nach Methode 1C ergab 2 mg des Beispiels 3.

LC-MS (Methode 1B): Rₜ = 1.05 min; MS (ESIpos): m/z = 492 [M+H]⁺.

### Beispiel 4

### 3-Fluor-N-({1-(morpholin-4-ylcarbonyl)-5-[4-(trifluormethyl)phenyl]piperidin-3-yl}methyl)-benzolcarboxamid [racemisches cis-Isomer]

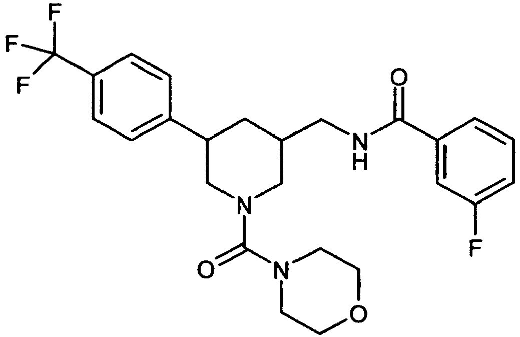

Nach der Allgemeinen Methode 2 wurden 131 mg (ca. 0.22 mmol) der Verbindung aus Beispiel 9A und 42 mg (0.27 mmol) 3-Fluorbenzoylchlorid umgesetzt. Ausbeute: 11 mg (10% d. Th.)

LC-MS (Methode 1B): Rₜ = 1.25 min; MS (ESIpos): m/z = 494 [M+H]⁺;

¹H-NMR (400 MHz, DMSO-d₆): δ = 8.72-8.70 (m, 1H), 7.89 (br s, 1H), 7.81 (d, 1H), 7.68 (d, 2H), 7.61 (br d, 1H), 7.54-7.49 (m, 3H), 3.67 (dd, 2H), 3.56-3.42 (m, 5H), 3.25-3.18 (m, 2H), 3.16-3.04 (m, 4H), 2.91-2.79 (m, 2H), 1.96-1.90 (m, 2H), 1.45 (q, 1H).

### Beispiel 5

### 3-Methoxy-N-({1-(morpholin-4-ylcarbonyl)-5-[4-(trifluormethyl)phenyl]piperidin-3-yl}methyl)-benzolcarboxamid [enantiomerenreines cis-Isomer]

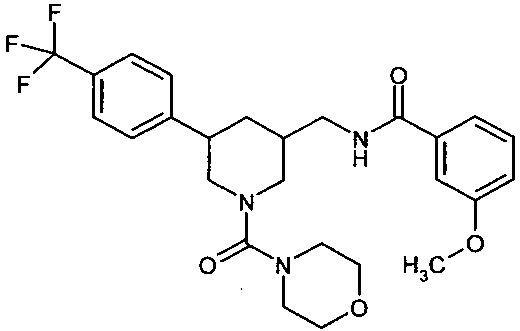

Nach der Allgemeinen Methode 2 wurden 129 mg (ca. 0.22 mmol) der Verbindung aus Beispiel 9A und 45 mg (0.26 mmol) 3-Methoxybenzolcarbonylchlorid umgesetzt. Enantiomerentrennung von 47 mg des Racemates nach Methode 1D ergab 9 mg des Beispiels 5.

HPLC (Methode 1E): Rₜ = 8.35 min, >97.0% ee; LC-MS (Methode 3B): Rₜ = 2.33 min; MS (ESIpos): m/z = 506 [M+H]⁺;

¹H-NMR (400 MHz, DMSO-d₆): δ = 8.57 (t, 1H), 7.68 (d, 2H), 7.53 (d, 2H), 7.44-7.36 (m, 3H), 7.08 (dd, 1H), 3.80 (s, 3H), 3.70 (br d, 1H), 3.64 (d, 1H), 3.50-3.46 (m, 4H), 3.23-3.18 (m, 2H), 3.14-3.06 (m, 4H), 2.92-2.79 (m, 2H), 2.00-1.90 (m, 2H), 1.93 (q, 1H).

### Beispiel 6

### N-({1-(Morpholin-4-ylcarbonyl)-5-[4-(trifluormethyl)phenyl]piperidin-3-yl}methyl)methan-sulfonamid [enantiomerenreines cis-Isomer]

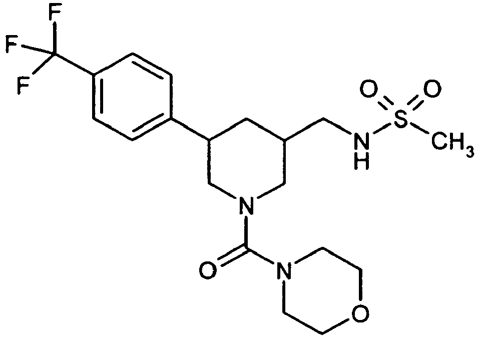

Nach der Allgemeinen Methode 1 wurden 104 mg (ca. 0.18 mmol) der Verbindung aus Beispiel 9A und 24 mg (0.21 mmol) Methansulfonsäurechlorid umgesetzt. Enantiomerentrennung des Rückstands nach Methode 2D ergab 8 mg des Beispiels 6 (Enantiomer 1) und 7 mg des Beispiels 7 (Enantiomer 2).

HPLC (Methode 1E): Rₜ = 10.28 min, >99.0% ee; LC-MS (Methode 2B): Rₜ = 2.01 min; MS (ESIpos): m/z = 450 [M+H]⁺;

¹H-NMR (400 MHz, DMSO-d₆): δ = 7.68 (d, 2H), 7.52 (d, 2H), 7.13 (t, 1H), 3.77 (br d, 3H), 3.63 (d, 1H), 3.58-3.51 (m, 4H), 3.16-3.10 (m, 4H), 2.90 (s, 3H), 2.93-2.76 (m, 3H), 1.93 (br d, 1H), 1.79 (br s, 1H), 1.37 (q, 1H).

### Beispiel 7

### N-({1-(Morpholin-4-ylcarbonyl)-5-[4-(trifluormethyl)phenyl]piperidin-3-yl}methyl)methan-sulfonamid [enantiomerenreines cis-Isomer]

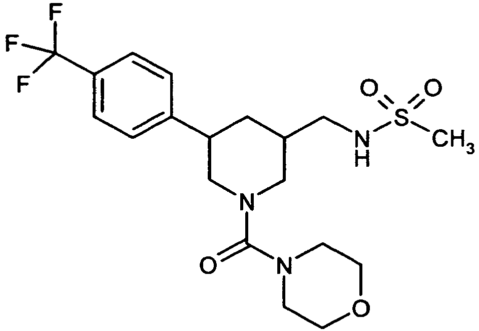

Nach der Allgemeinen Methode 1 wurden 104 mg (ca. 0.18 mmol) der Verbindung aus Beispiel 9A und 24 mg (0.21 mmol) Methansulfonsäurechlorid umgesetzt. Enantiomerentrennung des Rückstands nach Methode 2D ergab 8 mg des Beispiels 6 (Enantiomer 1) und 7 mg des Beispiels 7 (Enantiomer 2).

HPLC (Methode 1E): Rₜ = 12.14 min, >99.0% ee; LC-MS (Methode 2B): Rₜ = 2.01 min; MS (ESIpos): m/z = 450 [M+H]⁺.

### Beispiel 8

### 2-Fluor-N-({1-(morpholin-4-ylcarbonyl)-5-[4-(trifluomethyl)phenyl]piperidin-3-yl}methyl)-benzolsulfonamid [enantiomerenreines cis-Isomer]

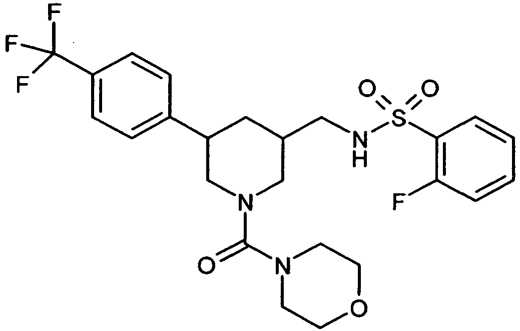

Nach der Allgemeinen Methode 1 wurden 126 mg (ca. 0.21 mmol) der Verbindung aus Beispiel 9A und 49 mg (0.21 mmol) 2-Fluorbenzolsulfonsäurechlorid umgesetzt. Enantiomerentrennung des Rückstands nach Methode 3D ergab 10 mg des Beispiels 8 (Enantiomer 1) und 10 mg des Beispiels 9 (Enantiomer 2).

HPLC (Methode 2E): Rₜ = 11.53 min, >98.0% ee; LC-MS (Methode 3B): Rₜ = 2.42 min; MS (ESIpos): m/z = 530 [M+H]⁺;

¹H-NMR (400 MHz, DMSO-d₆): δ = 8.07 (br s, 1H), 7.79 (dt, 1H), 7.74-7.67 (m, 3H), 7.47 (d, 2H), 7.44-7.37 (m, 2H), 3.72 (br d, 1H), 3.60 (d, 1H), 3.58-3.53 (m, 4H), 3.17-3.09 (m, 4H), 2.86-2.70 (m, 4H), 2.43 (t, 1H), 1.86 (br d, 1H), 1.74 (br s, 1H), 1.30 (q, 1H).

### Beispiel 9

### 2-Fluor-N-({1-(morpholin-4-ylcarbonyl)-5-[4-(trifluormethyl)phenyl]piperidin-3-yl}methyl)-benzolsulfonamid [enantiomerenreines cis-Isomer]

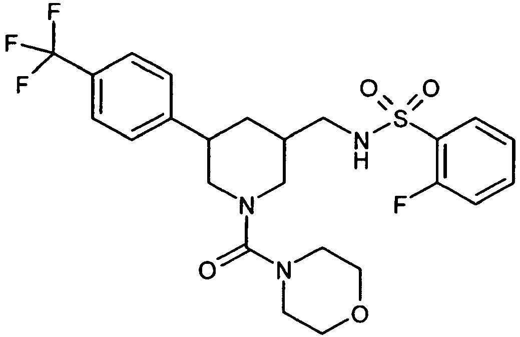

Nach der Allgemeinen Methode 1 wurden 126 mg (ca. 0.21 mmol) der Verbindung aus Beispiel 9A und 49 mg (0.21 mmol) 2-Fluorbenzolsulfonsäurechlorid umgesetzt. Enantiomerentrennung des Rückstands nach Methode 3D ergab 10 mg des Beispiels 8 (Enantiomer 1) und 10 mg des Beispiels 9 (Enantiomer 2).

HPLC (Methode 2E): Rₜ = 13.21 min, >96.0% ee; LC-MS (Methode 1B): Rₜ = 1.27 min; MS (ESIpos): m/z = 530 [M+H]⁺.

### Beispiel 10

### {1-(Morpholin-4-ylcarbonyl)-5-[4-(trifluormethoxy)phenyl]piperidin-3-yl}methyl-(3-fluorphenyl)-carbamat [racemisches cis-Isomer]

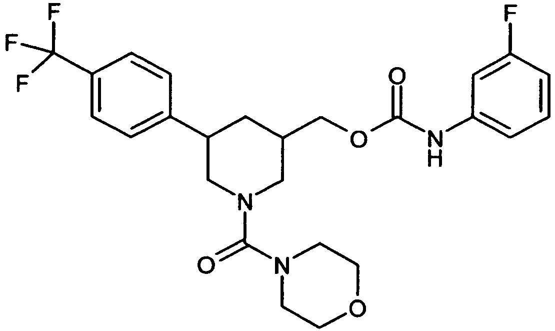

Nach der Allgemeinen Methode 6 wurden 100 mg (0.26 mmol) der Verbindung aus Beispiel 6A und 42 mg (0.31 mmol) 3-Fluorphenylisocyanat umgesetzt. Ausbeute: 24 mg (17% d. Th.)

LC-MS (Methode 4B): Rₜ = 2.35 min; MS (ESIpos): m/z = 526 [M+H]⁺;

¹H-NMR (400 MHz, DMSO-d₆): δ = 9.91 (s, 1H), 7.44-7.28 (m, 6H), 7.22 (d, 1H), 6.81 (dt, 1H), 4.06 (dd, 1H), 4.00 (dd, 1H), 3.78 (br d, 1H), 3.62 (d, 1H), 3.56-3.53 (m, 4H), 3.18-3.10 (m, 4H), 2.90-2.76 (m, 2H), 2.60 (t, 1H), 2.09-1.09 (m, 1H), 1.95 (br d, 1H), 1.45 (q, 1H).

### Beispiel 11

### {1-(Morpholin-4-ylcarbonyl)-5-[4-(trifluormethoxy)phenyl]piperidin-3-yl}methyl-(2-chlorphenyl)-carbamat [racemisches cis-Isomer]

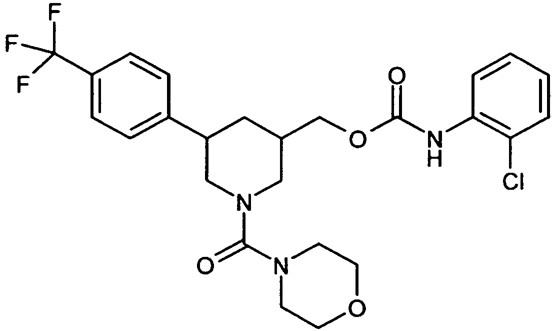

Nach der Allgemeinen Methode 6 wurden 50 mg (0.13 mmol) der Verbindung aus Beispiel 6A und 23 mg (0.15 mmol) 2-Chlorphenylisocyanat umgesetzt. Ausbeute: 18 mg (26% d. Th.)

LC-MS (Methode 3B): Rₜ = 2.82 min; MS (ESIpos): m/z = 542 [M+H]⁺.

### Beispiel 12

### {1-(Morpholin-4-ylcarbonyl)-5-[4-(trifluormethoxy)phenyl]piperidin-3-yl}methyl-(4-methylphenyl)carbamat

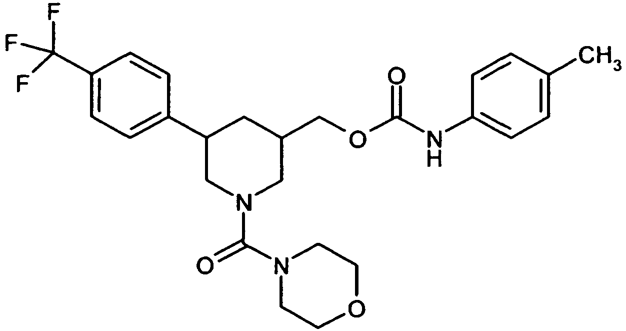

Nach der Allgemeinen Methode 6 wurden 50 mg (0.13 mmol) der Verbindung aus Beispiel 6A und 20 mg (0.15 mmol) 1-Isocyanato-4-methylbenzol umgesetzt. Ausbeute: 21 mg (32% d. Th.)

LC-MS (Methode 4B): Rₜ = 2.35 min; MS (ESIpos): m/z = 522 [M+H]⁺.

### Beispiel 13

### N-Benzyl-2-{1-(morpholin-4-ylcarbonyl)-5-[4-(trifluormethyl)phenyl]piperidin-3-yl}acetamid [enantiomerenreines cis-Isomer]

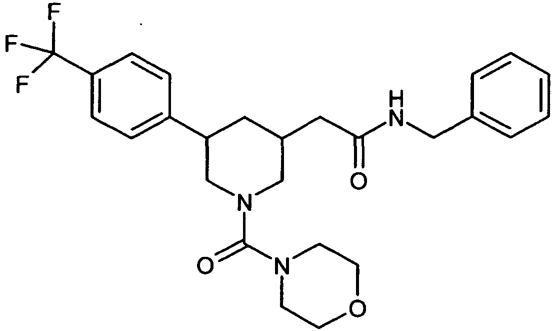

Nach der Allgemeinen Methode 3 wurden 100 mg (ca. 0.23 mmol) der Verbindung aus Beispiel 5A und 28 mg (0.27 mmol) Benzylamin umgesetzt. Enantiomerentrennung des Rückstands nach Methode 4D ergab 17 mg des Beispiels 13.

HPLC (Methode 3E): Rₜ = 10.04 min, >99.0% ee; LC-MS (Methode 1B): Rₜ = 1.23 min; MS (ESIpos): m/z = 490 [M+H]⁺;

¹H-NMR (400 MHz, DMSO-d₆): δ = 8.41 (t, 1H), 7.68 (d, 2H), 7.50 (d, 2H), 7.31-7.20 (m, 5H), 4.31-4.21 (m, 2H), 3.68 (d, 1H), 3.65 (d, 1H), 3.55-3.50 (m, 4H), 3.29 (s, 2H), 3.13-3.09 (m, 4H), 2.88 (tt, 1H), 2.76 (t, 1H), 2.14-2.12 (m, 2H), 2.07-2.00 (m, 1H), 1.90 (br d, 1H), 1.38(q, 1H).

### Beispiel 14

### N,N-Diethyl-2-{1-(morpholin-4-ylcarbonyl)-5-[4-(trifluormethyl)phenyl]piperidin-3-yl}acetamid [racemisches cis-Isomer]

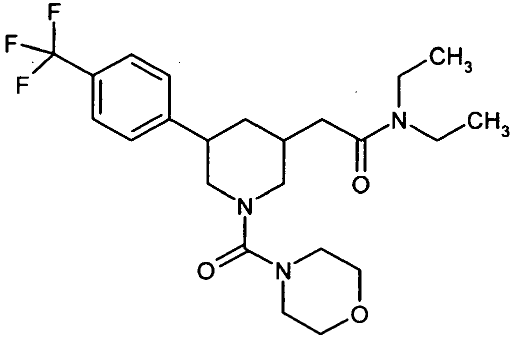

Nach der Allgemeinen Methode 3 wurden 100 mg (ca. 0.23 mmol) der Verbindung aus Beispiel 5A und 20 mg (0.27 mmol) Diethylamin umgesetzt. Diastereomerentrennung des Rückstands nach Methode 2C ergab 51 mg des Beispiels 14.

LC-MS (Methode 3B): Rₜ = 2.50 min; MS (ESIpos): m/z = 456 [M+H]⁺;

¹H-NMR (400 MHz, DMSO-d₆): δ = 7.68 (d, 2H), 7.51 (d, 2H), 3.72-3.65 (m, 2H), 3.60-3.53 (m, 4H), 3.30-3.23 (m, 5H), 3.20-3.10 (m, 4H), 2.95-2.83 (m, 1H), 2.72 (t, 1H), 2.25 (d, 2H), 2.13-2.00 (m, 1H), 1.93 (br d, 1H), 1.43 (q, 1H), 1.09 (t, 3H), 1.00 (t, 3H).

### Beispiel 15

### 2-{1-(Morpholin-4-ylcarbonyl)-5-[4-(trifluormethyl)phenyl]piperidin-3-yl}-N-phenylacetamid [racemisches cis-Isomer]

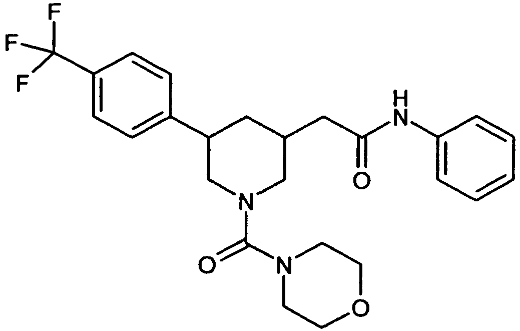

Nach der Allgemeinen Methode 3 wurden 100 mg (ca. 0.23 mmol) der Verbindung aus Beispiel 5A und 25 mg (0.27 mmol) Anilin umgesetzt. Diastereomerentrennung des Rückstands nach Methode 2C ergab 4 mg des Beispiels 15.

LC-MS (Methode 1B): Rₜ = 1.26 min; MS (ESIpos): m/z = 476 [M+H]⁺;

¹H-NMR (400 MHz, DMSO-d₆): δ = 9.94 (s, 1H), 7.68 (d, 2H), 7.58 (d, 2H), 7.52 (d, 4H), 7.28 (dd, 2H), 7.02 (dd, 1H), 3.75 (br d, 1H), 3.66 (br d, 1H), 3.56-3.50 (m, 4H), 3.16-3.10 (m, 4H), 2.97-2.87 (m, 1H), 2.80 (t, 1H), 2.35-2.29 (m, 2H), 2.17-2.05 (m, 1H), 1.96 (br d, 1H), 1.46 (q, 1H).

### Beispiel 16

### {1-(Morpholin-4-ylcarbonyl)-5-[4-(trifluormethyl)phenyl]piperidin-3-yl}methyl-isobutylcarbamat [racemisches cis-Isomer]

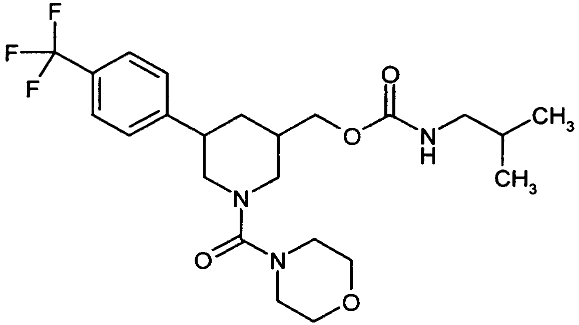

Nach der Allgemeinen Methode 5 wurden 100 mg (0.196 mmol) der Verbindung aus Beispiel 6A und 23 mg (0.235 mmol) Isobutylisocyanat umgesetzt. Ausbeute: 7 mg (12% d. Th.)

HPLC (Methode 8B): Rₜ= 1.16 min; MS (ESIpos): m/z = 472 [M+H]⁺;

¹H NMR (400 MHz, DMSO-d₆): δ = 7.69 (d, 2H), 7.52 (d, 2H), 7.19 (t, 1H), 3.91-3.85 (m, 2H), 3.72 (d, 1H), 3.64 (d, 1H), 3.60-3.53 (m, 4H), 3.18-3.10 (m, 4H), 2.94-2.85 (m, 1H), 2.83-2.77 (m, 3H), 1.98-1.84 (m, 2H), 1.70-1.58 (m, 1H), 1.42 (q, 1H), 0.82 (d, 6H).

### Beispiel 17

### {1-(Morpholin-4-ylcarbonyl)-5-[4-(trifluormethyl)phenyl]piperidin-3-yl}methyl-ethylcarbamat [racemisches cis-Isomer]

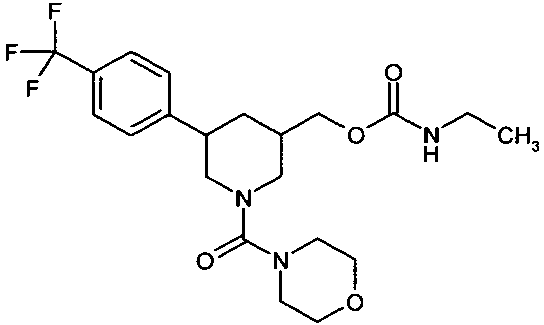

Nach der Allgemeinen Methode 5 wurden 200 mg (0.392 mmol) der Verbindung aus Beispiel 6A und 33 mg (0.470 mmol) Ethylisocyanat umgesetzt. Ausbeute: 73 mg (40% d. Th.)

HPLC (Methode 8B): Rₜ, = 1.06 min; MS (ESIpos): m/z = 444 [M+H]⁺;

¹H-NMR (400 MHz, DMSO-d₆): δ = 7.69 (d, 2H), 7.52 (d, 2H), 7.13 (t, 1H), 3.91 (dd, 1H), 3.86-3.59 (m, 3H), 3.56 (t, 4H), 3.14 (d, 4H), 3.05-2.95 (m, 2H), 2.95-2.73 (m, 2H), 1.91 (br s, 2H) 1.42 (d, 1H) 1.01 (t, 3H).

### Beispiel 18

### {1-(Morpholin-4-ylcarbonyl)-5-[4-(trifluormethyl)phenyl]piperidin-3-yl}methyl-(2-methoxyethyl)-carbamat [racemisches cis-Isomer]

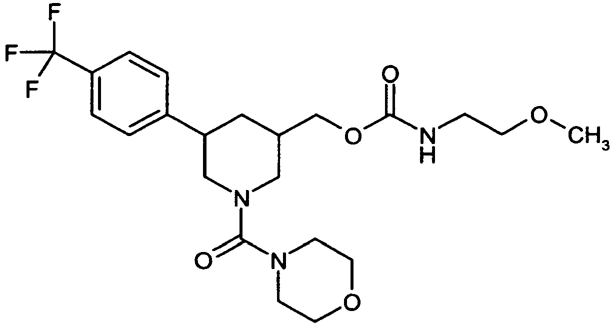

Nach der Allgemeinen Methode 5 wurden 200 mg (0.392 mmol) der Verbindung aus Beispiel 6A und 48 mg (0.470 mmol) 1-Isocyanato-2-methoxyethan umgesetzt. Ausbeute: 65 mg (35% d. Th.)

HPLC (Methode 8B): Rₜ = 1.02 min; MS (ESIpos): m/z = 474 [M+H]⁺;

¹H-NMR (400 MHz, DMSO-d₆): δ = 7.69 (d, 2H), 7.52 (d, 2H), 7.20 (t, 1H, 3.95-3.79 (m, 2 H), 3.72 (d, 1H), 3.64 (d, 1H), 3.60-3.49 (m, 4H), 3.22 (s, 3H), 3.19-3.06 (m, 6H), 2.94-2.86 (m, 1H), 2.78 (t, 1H), 1.91 (br s, 2H), 1.42 (q, 1H).

### Beispiel 19

### {1-(Morpholin-4-ylcarbonyl)-5-[4-(trifluormethyl)phenyl]piperidin-3-yl}methyl-isopropyl-carbamat [racemisches cis-Isomer]

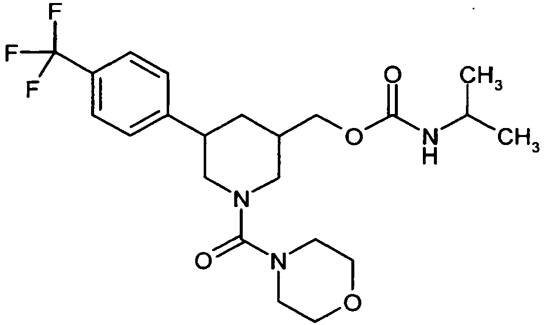

Nach der Allgemeinen Methode 5 wurden 200 mg (0.392 mmol) der Verbindung aus Beispiel 6A und 40 mg (0.470 mmol) 2-Isocyanatopropan umgesetzt. Ausbeute: 86 mg (48% d. Th.)

HPLC (Methode 8B): Rₜ = 1.11 min; MS (ESIpos): m/z = 458 [M+H]⁺;

¹H-NMR (400 MHz, DMSO-d₆): δ = 7.69 (d, 2H), 7.51 (s, 2H), 7.05 (d, 1H), 3.94-3.78 (m, 2H), 3.76-3.50 (m, 7H), 3.20-3.18 (m, 4H), 2.95-2.80 (m, 1H), 2.78 (t, 1H), 2.00-1.85 (d, 2H), 1.43 (q, 1H), 1.06 (d, 6H).

### Beispiel 20

### {1-(Morpholin-4-ylcarbonyl)-5-[4-(trifluormethyl)phenyl]piperidin-3-yl}methyl-(3-methoxy-2,2-dimethylpropyl)carbamat [racemisches cis-Isomer]

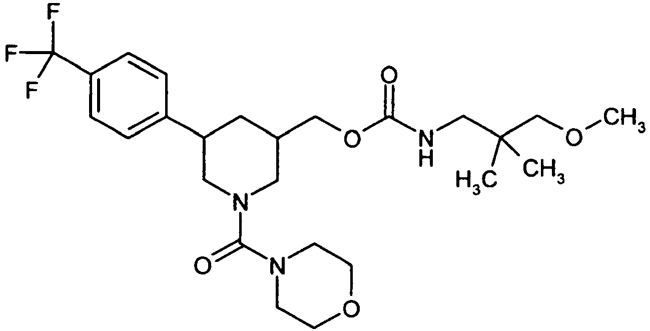

Nach der Allgemeinen Methode 5 wurden 200 mg (0.392 mmol) der Verbindung aus Beispiel 6A und 67 mg (0.470 mmol) 1-Isocyanato-3-methoxy-2,2-dimethylpropan umgesetzt. Ausbeute: 40 mg (20% d. Th.)

HPLC (Methode 1B): Rₜ = 1.32 min; MS (ESIpos): m/z = 516 [M+H]⁺;

¹H-NMR (400 MHz, DMSO-d₆): δ = 7.69 (d, 2H), 7.52 (d, 2H), 7.06 (t, 1H), 3.90 (dm, 2H), 3.74 (d, 1H), 3.64 (d, 1H), 3.59-3.52 (m, 4H), 3.21 (s, 3H), 3.18-3.01 (m., 4H), 3.01 (s, 3H), 2.95-2.85 (m, 3H), 2.79 (t, 1H), 2.01-1.85 (m, 2H), 1.42 (q, 1H), 0.79 (s, 6H).

### Beispiel 21

### {1-(Morpholin-4-ylcarbonyl)-5-[4-(trifluormethyl)phenyl]piperidin-3-yl}methyl-propylcarbamat [racemisches cis-Isomer]

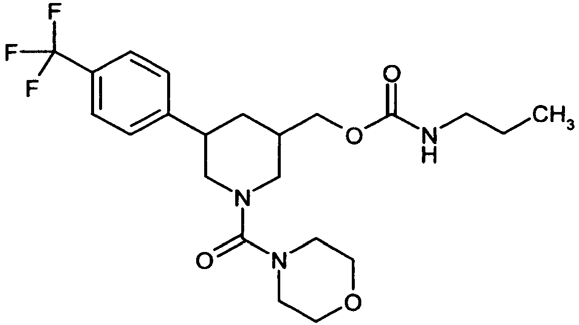

Nach der Allgemeinen Methode 5 wurden 200 mg (0.392 mmol) der Verbindung aus Beispiel 6A und 40 mg (0.470 mmol) Isobutylisocyanat umgesetzt. Ausbeute: 71 mg (40% d. Th.)

HPLC (Methode 8B): Rₜ = 1.11 min; MS (ESIpos): m/z = 458 [M+H]⁺;

¹H-NMR (400 MHz, DMSO-d₆): δ = 7.69 (d, 2H), 7.52 (d, 2H), 7.15 (t, 1H), 3.89-3.83 (m, 2H), 3.72 (d, 1H), 3.64 (d, 1H), 3.60-3.52 (m, 4H), 3.18-3.15 (m, 4H), 2.96-2.85 (m, 3H), 2.74 (t, 1H), 2.01-1.85 (m, 2H), 1.47-1.36 (m, 3H), 0.83 (t, 3H).

### Beispiel 22

### {3-(Methoxymethyl)-5-[4-(trifluormethyl)phenyl]piperidin-1-yl}(morpholin-4-yl)methanon [racemisches cis-Isomer]

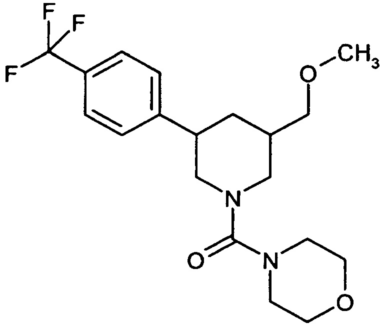

Unter Argon wurden 71 mg (2.22 mmol) Methanol in 5.0 ml *N,N*'-Dimethylformamid vorgelegt, mit Molsieb 3Å und 27 mg (0.666 mmol, 60%ig in Paraffinöl) Natriumhydrid versetzt und 30 min bei RT gerührt. Anschließend wurden 100 mg (0.222 mmol) des Mesylats aus Beispiel 7A in 1.0 ml *N,N*'-Dimethylformamid zugesetzt und für 1h bei RT gerührt. Die Reaktion wurde durch Zugabe von Wasser beendet, das Molsieb abfiltriert und das Filtrat mittels präparativer HPLC gereinigt. Ausbeute: 59 mg (68% d. Th.)

LC-MS (Methode 8B): Rₜ = 1.08 min; MS (ESIpos): m/z = 387 [M+H]⁺;

¹H-NMR (400 MHz, DMSO-d₆): δ = 7.68 (d, 2H), 7.52 (d, 2H), 3.73 (d, 1H), 3.62 (br s, 1H), 3.55 (t, 4H), 3.31-3.19 (m, 7H), 3.14 (d, 4H), 2.94-2.83 (m, 1H), 2.83-2.74 (m, 1H), 1.88 (d, 2H), 1.41 (q, 1H).

### Beispiel 23

### {3-(Ethoxymethyl)-5-[4-(trifluormethyl)phenyl]piperidin-1-yl}(morpholin-4-yl)methanon [racemisches cis-Isomer]

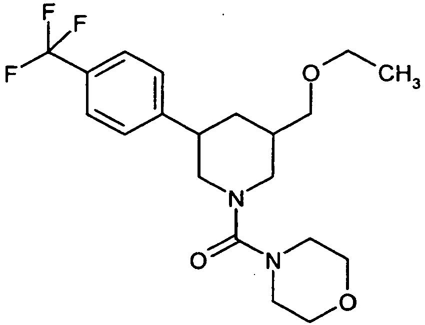

Unter Argon wurden 51 mg ( 1.11 mmol) Ethanol in 2.0 ml *N,N*'-Dimethylformamid vorgelegt, mit Molsieb 3Å und 13 mg (0.333 mmol, 60%ig in Paraffinöl) Natriumhydrid versetzt und 30 min bei RT gerührt. Anschließend wurden 50 mg (0.111 mmol) des Mesylats aus Beispiel 7A in 1.0 ml *N,N*'-Dimethylformamid zugesetzt und für 2 h bei RT gerührt. Die Reaktion wurde durch Zugabe von Wasser beendet, das Molsieb abfiltriert und das Filtrat mittels präparativer HPLC gereinigt. Ausbeute: 40.0 mg (87% d. Th.)

LC-MS (Methode 8B): Rₜ = 1.15 min; MS (ESIpos): m/z = 401 [M+H]⁺;

¹H-NMR (400 MHz, DMSO-d₆): δ = 7.68 (d, 2H), 7.52 (d, 2H), 3.75 (d, 1H), 3.64 (d, 1H), 3.56 (br s, 4H), 3.41 (quin, 2H), 3.21-3.32 (m, 3H), 3.14 (br s, 4H), 2.94-2.83 (m, 1H), 2.84-2.73 (m, 1H), 1.89 (d, 2H), 1.40 (q, 1H), 1.11 (t, 3H).

### Beispiel 24

### {3-(Isopropoxymethyl)-5-[4-(trifluormethyl)phenyl]piperidin-1-yl}(morpholin-4-yl)methanon [racemisches cis-Isomer]

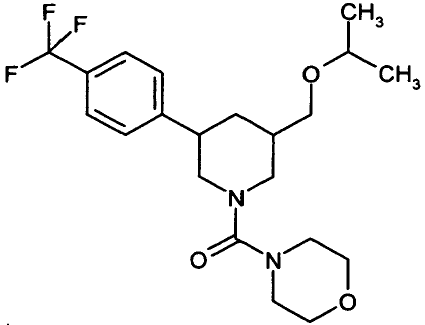

Unter Argon wurden 133 mg (2.22 mmol) Isopropanol in 5.0 ml *N,N*'-Dimethylformamid vorgelegt, mit Molsieb 3 Å und 27 mg (0.67 mmol, 60%ig in Paraffinöl) Natriumhydrid versetzt und 30 min bei RT gerührt. Anschließend wurden 100 mg (0.22 mmol) des Mesylats aus Beispiel 7A in 1.0 ml *N,N*'-Dimethylformamid zugesetzt und für 1 h bei RT gerührt. Die Reaktion wurde durch Zugabe von Wasser beendet, das Molsieb abfiltriert und das Filtrat mittels präparativer HPLC gereinigt. Ausbeute: 32.8 mg (36% d. Th.).

LC-MS (Methode 8B): Rₜ = 1.21 min; MS (ESIpos): m/z = 415 [M+H]⁺;

¹H-NMR (400 MHz, DMSO-d₆): δ = 7.68 (d, 2H), 7.51 (d, 2H), 3.75 (br s, 1H), 3.69-3.61 (m, 1H), 3.60-3.46 (m, 5H), 3.31-3.26 (m, 2H), 3.26-3.19 (m, 1H), 3.13 (d, 4H), 2.88 (br s, 1H), 2.83-2.73 (m, 1H), 1.87 (br s, 2H), 1.45-1.32 (m, 1H), 1.08 (d, 6H).

### Beispiel 25

### {3-[(Cyclobutyloxy)methyl]-5-[4-(trifluormethyl)phenyl]piperidin-1-yl}(morpholin-4-yl)methanon [racemisches cis-Isomer]

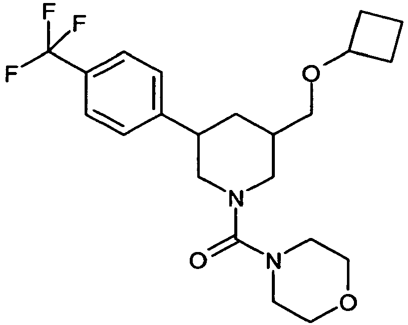

Unter Argon wurden 160 mg (2.22 mmol) Cyclobutanol in 5.0 ml *N,N*'-Dimethylformamid vorgelegt, mit Molsieb 3Å und 27 mg (0.666 mmol, 60%ig in Paraffinöl) Natriumhydrid versetzt und 30 min bei RT gerührt. Anschließend wurden 100 mg (0.222 mmol) des Mesylats aus Beispiel 7A in 1.0 ml *N,N*'-Dimethylfonmamid zugesetzt und für 1 h bei RT gerührt. Die Reaktion wurde durch Zugabe von Wasser beendet, das Molsieb abfiltriert und das Filtrat mittels präparativer HPLC gereinigt. Ausbeute: 63.8 mg (67% d. Th.)

LC-MS (Methode 8B): Rₜ = 1.25 min; MS (ESIpos): m/z = 427 [M+H]⁺;

¹H-NMR (400 MHz, DMSO-d₆): δ = 7.68 (d, 2H), 7.51 (d, 2H), 3.87 (quin, 1H), 3.75 (d, 1H), 3.64 (d, 1H), 3.55 (d, 4H), 3.28 (d, 1H), 3.24-3.05 (m, 6H), 2.94-2.74 (m, 2H), 2.20-2.08 (m, 2H), 1.96-1.71 (m, 4H), 1.69-1.55 (m, 1H), 1.53-1.29 (m, 2H).

### Beispiel 26

### {3-[(Cyclopentyloxy)methyl]-5-[4-(trifluormethyl)phenyl]pipeiridin-1-yl}(morpholin-4-yl)-methanon [racemisches cis-Isomer]

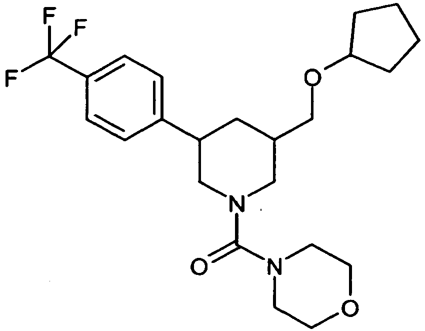

Unter Argon wurden 191 mg (2.22 mmol) Cyclopentanol in 5.0 ml *N,N*'-Dimethylformamid vorgelegt, mit Molsieb 3Å und 27 mg (0.666 mmol, 60%ig in Paraffinöl) Natriumhydrid versetzt und 30 min bei RT gerührt. Anschließend wurden 100 mg (0.222 mmol) des Mesylats aus Beispiel 7A in 1.0 ml *N,N*'-Dimethylformamid zugesetzt und für 1 h bei RT gerührt. Die Reaktion wurde durch Zugabe von Wasser beendet, das Molsieb abfiltriert und das Filtrat mittels präparativer HPLC gereinigt. Ausbeute: 33 mg (32% d. Th.)

LC-MS (Methode 8B): Rₜ = 1.13 min; MS (ESIpos): m/z = 441 [M+H]⁺;

¹H-NMR (400 MHz, DMSO-d₆): δ = 7.68 (d, 2H), 7.51 (d, 2H), 3.84 (tt, 1H), 3.79-3.70 (m, 1H), 3.69-3.61 (m, 1H), 3.56 (t, 4H), 3.27 (dd, 2H), 3.22-3.09 (m, 5H), 2.86 (d, 1H), 2.82-2.70 (m, 1H), 1.87 (br s, 2H), 1.71-1.44 (m, 8H), 1.44-1.31 (m, 1H).

### Beispiel 27

### {3-[(2-Methoxyethoxy)methyl]-5-[4-(trifluormethyl)phenyl]piperidin-1-yl}(morpholin-4-yl)-methanon [racemisches cis-Isomer]

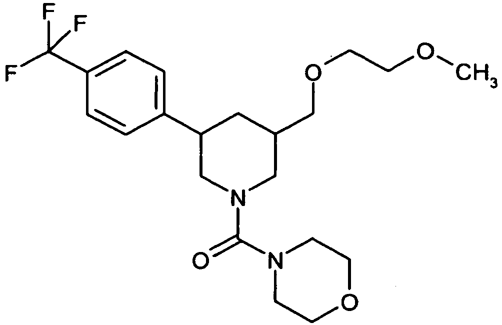

Unter Argon wurden 169 mg (2.22 mmol) Isopropanol in 5.0 ml *N,N*'-Dimethylformamid vorgelegt, mit Molsieb 3Å und 27 mg (0.67 mmol, 60%ig in Paraffinöl) Natriumhydrid versetzt und 30 min bei RT gerührt. Anschließend wurden 100 mg (0.22 mmol) des Mesylats aus Beispiel 7A in 1.0 ml *N,N*'-Dimethylformamid zugesetzt und für 1 h bei RT gerührt. Die Reaktion wurde durch Zugabe von Wasser beendet, das Molsieb abfiltriert und das Filtrat mittels präparativer HPLC gereinigt. Ausbeute: 59 mg (61% d. Th.).

LC-MS (Methode 1B): Rₜ = 1.22 min; MS (ESIpos): m/z = 431 [M+H]⁺;

¹H-NMR (400MHz, DMSO-d₆): δ = 7.68 (d, 2H), 7.52 (d, 2H), 3.76 (d, 1H), 3.64 (d, 1H), 3.60-3.40 (m, 8H), 3.25 (s, 3H), 3.14 (d, 4H), 2.95-2.83 (m, 1H), 2.83-2.73 (m, 1H), 1.88 (d, 2H), 1.39 (q, 1H); drei Protonen verdeckt.

### Beispiel 28

### Morpholin-4-yl{3-[(tetrahydro-2H-pyran-4-yloxy)methyl]-5-[4-(trifluormethyl)phenyl]piperidin-1-yl}methanon [racemisches cis-Isomer]

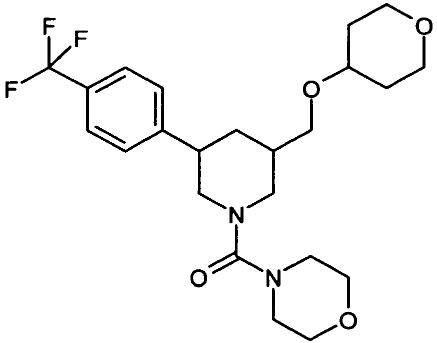

Unter Argon wurden 227 mg (2.22 mmol) Tetrahydro-2H-pyran-4-ol in 5.0 ml *N,N'-*Dimethylformamid vorgelegt, mit Molsieb 3Å und 27 mg (0.666 mmol, 60%ig in Paraffinöl) Natriumhydrid versetzt und 30 min bei RT gerührt. Anschließend wurden 100 mg (0.222 mmol) des Mesylats aus Beispiel 7A in 1.0 ml *N,N*'-Dimethylformamid zugesetzt und für 1 h bei RT gerührt. Die Reaktion wurde durch Zugabe von Wasser beendet, das Molsieb abfiltriert und das Filtrat mittels präparativer HPLC gereinigt. Ausbeute: 42 mg (42 % d. Th.)

LC-MS (Methode 7B): Rₜ = 2.28 min; MS (ESIpos): m/z = 457 [M+H]⁺;

¹H-NMR (400 MHz, DMSO-d₆): δ = 7.68 (d, 2H), 7.52 (d, 2H), 3.84-3.73 (m, 3H), 3.65 (d, 1H), 3.56 (t, 4H), 3.45 (tt, 1H), 3.40-3.23 (m, 5H), 3.14 (d, 4H), 2.95-2.84 (m, 1H), 2.83-2.74 (m, 1H), 1.96-1.77 (m, 4H), 1.48-1.31 (m, 7H).

### Beispiel 29

### {3-[(Benzyloxy)methyl]-5-[4-(trifluormethyl)phenyl]piperidin-1-yl}(morpholin-4-yl)methanon [racemisches cis-Isomer]

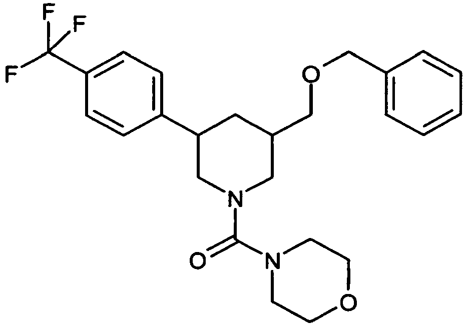

Unter Argon wurden 240 mg (2.22 mmol) Benzylalkohol in 5.0 ml *N,N*'-Dimethylformamid vorgelegt, mit Molsieb 3Å und 27 mg (0.666 mmol, 60%ig in Paraffinöl) Natriumhydrid versetzt und 30 min bei RT gerührt. Anschließend wurden 100 mg (0.222 mmol) des Mesylats aus Beispiel 7A in 1.0 ml *N,N*'-Dimethylformamid zugesetzt und über Nacht bei RT gerührt. Die Reaktion wurde durch Zugabe von Wasser beendet, das Molsieb abfiltriert und das Filtrat mittels präparativer HPLC gereinigt. Ausbeute: 71.4 mg (70% d. Th.)

LC-MS (Methode 7B): Rₜ = 2.65 min; MS (ESIpos): m/z = 463 [M+H]⁺;

¹H-NMR (400 MHz, DMSO-d₆): δ = 7.68 (d, 2H), 7.52 (d, 2H), 7.41-7.24 (m, 5H), 4.56-4.41 (m, 2H), 3.79 (d, 1H), 3.65 (d, 1H), 3.55 (t, 4H), 3.43-3.34 (m, 2H), 3.30 (s, 1H), 3.14 (d, 4H), 2.95-2.84 (m, 1H), 2.83-2.74 (m, 1H), 2.63-2.55 (m, 1H), 2.04-1.84 (m, 2H), 1.43 (q, 3H).

### Beispiel 30

### {3-[(3-Fluorphenoxy)methyl]-5-[4-(trifluormethyl)phenyl]piperidin-l-yl}(morpholin-4-yl)methanon [racemisches cis-Isomer]

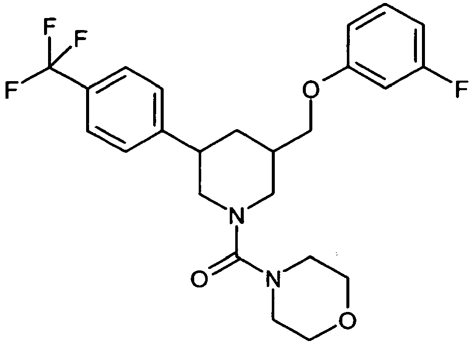

Unter Argon wurden 249 mg (2.22 mmol) 3-Fluorphenol in 5.0 ml *N,N*'-Dimethylformamid vorgelegt, mit Molsieb 3Å und 27 mg (0.67 mmol, 60%ig in Paraffinöl) Natriumhydrid versetzt und 30 min bei RT gerührt. Anschließend wurden 100 mg (0.22 mmol) des Mesylats aus Beispiel 7A in 1.0 ml *N,N*'-Dimethylformamid zugesetzt und über Nacht bei RT gerührt. Die Reaktion wurde durch Zugabe von Wasser beendet, das Molsieb abfiltriert und das Filtrat mittels präparativer HPLC gereinigt. Ausbeute: 87.1 mg (84% d. Th.)

LC-MS (Methode 7B): Rₜ = 2.69 min; MS (ESIpos): m/z = 467 [M+H]⁺;

¹H-NMR (400 MHz, DMSO-d₆): δ = 7.70 (d, 2H), 7.54 (d, 2H), 7.32 (q, 1H), 6.89-6.71 (m, 3H), 4.02-3.94 (m, 1H), 3.94-3.80 (m, 2H), 3.67 (d, 1H), 3.56 (t, 4H), 3.21-3.08 (m, 4H), 3.01-2.90 (m, 1H), 2.88-2.78 (m, 1H), 2.73-2.62 (m, 1H), 2.13 (br s, 1H), 2.03 (d, 1H), 1.61-1.47 (m, 3H).

### Beispiel 31

### {3-[(Isopropylsulfanyl)methyl]-5-[4-(trifluormethyl)phenyl]piperidin-1-yl}(morpholin-4-yl)methanon [racemisches cis-Isomer]

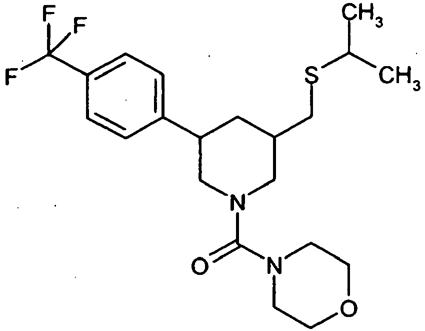

Unter Argon wurden 423 mg (5.55 mmol) Propan-2-thiol in 10.0 ml *N,N*'-Dimethylformamid vorgelegt, mit Molsieb 3Å und 67 mg (1.67 mmol, 60%ig in Paraffinöl) Natriumhydrid versetzt und 30 min bei RT gerührt. Anschließend wurden 250 mg (0.555 mmol) des Mesylats aus Beispiel 7A in 5.0 ml *N,N*'-Dimethylformamid zugesetzt und für 3 h bei RT gerührt. Die Reaktion wurde durch Zugabe von Wasser beendet, das Molsieb abfiltriert und das Filtrat mittels präparativer HPLC gereinigt. Ausbeute: 198 mg (80% d. Th.)

LC-MS (Methode 8B): Rₜ = 1.28 min; MS (ESIpos): m/z = 431 [M+H]⁺;

¹H-NMR (400 MHz, DMSO-d₆): δ = 7.68 (d, 2H), 7.51 (d, 2H), 3.85 (d, 1H), 3.64 (d, 1H), 3.57 (t, 4H), 3.15 (d, 4H), 2.97-2.84 (m, 2H), 2.83-2.73 (m, 1H), 2.48-2.35 (m, 2H), 2.03 (d, 1H), 1.77 (br s, 1H), 1.41 (q, 1H), 1.21 (d, 6H); ein Proton verdeckt.

### Beispiel 32

### {3-[(Ethylsulfanyl)methyl]-5-[4-(trifluormethyl)phenyl]piperidin-1-yl}(morpholin-4-yl)methanon [racemisches cis-Isomer]

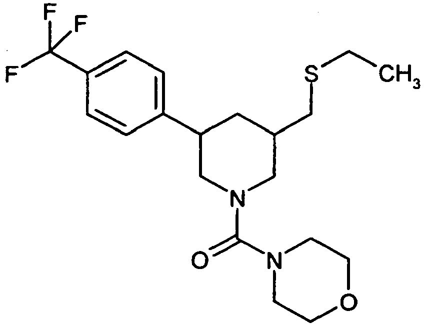

Unter Argon wurden 690 mg (11.1 mmol) Ethanthiol in 25.0 ml *N,N*'-Dimethylformamid vorgelegt, mit Molsieb 3Å und 133 mg (3.33 mmol, 60%ig in Paraffinöl) Natriumhydrid versetzt und 30 min bei RT gerührt. Anschließend wurden 500 mg (1.11 mmol) des Mesylats aus Beispiel 7A in 5.0 ml *N,N*'-Dimethylformamid zugesetzt und für 3 h bei RT gerührt. Die Reaktion wurde durch Zugabe von Wasser beendet, das Molsieb abfiltriert und das Filtrat mittels präparative HPLC gereinigt. Ausbeute: 223 ring (48% d. Th.)

LC-MS (Methode1B): Rₜ = 1.38 min; MS (ESIpos): m/z = 417 [M+H]⁺;

¹H-NMR (400 MHz, DMSO-d₆): δ = 7.69 (d, 2H), 7.51 (d, 2H), 3.85 (d, 1H), 3.64 (d, 1H), 3.57 (t, 4H), 3.15 (d, 4H), 2.95-2.84 (m, 1H), 2.83-2.73 (m, 1H), 2.49-2.39 (m, 3H), 2.02 (d, 1H), 1.79 (br s, 1 H), 1.41 (q, 1H), 1.18 (t, 3 H); zwei Protonen verdeckt.

### Beispiel 33

### {3-[(Isopropylsulfinyl)methyl]-5-[4-(trifluormethyl)phenyl]piperidin-1-yl}(morpholin-4-yl)methanon [racemisches cis-Diasteromerengemisch]

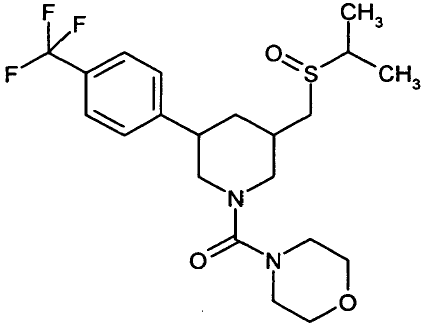

140 mg (0.325 mmol) des Thioethers aus Beispiel 31 in 14.0 ml Dichlormethan wurden mit 168 mg (0.488 mmol, 50%ig) *meta*-Chlorperoxybenzoesäure versetzt und 1 h bei RT gerührt. Die Reaktionslösung wurde im Vakuum eingeengt und der Rückstand mittels präparativer HPLC gereinigt. Ausbeute: 53 mg (36% d. Th.)

LC-MS (Methode 8B): Rₜ = 0.97 min; MS (ESIpos): m/z = 447 [M+H]⁺;

¹H-NMR (400 MHz, DMSO-d₆): δ = 7.70 (d, 2H), 7.53 (d, 2H), 3.92-3.75 (m, 1H), 3.65 (d, 1H), 3.57 (t, 4H), 3.21-3.11 (m, 4H), 3.03-2.89 (m, 1H), 2.88-2.77 (m, 2H), 2.70-2.59 (m, 2H), 2.57 (d, 2H), 2.57-2.55 (m, 1H), 2.19-2.00 (m, 2H), 1.56 (quin, 1H), 1.23-1.12 (m, 6H).

### Beispiel 34

### {3-[(Isopropylsulfonyl)methyl]-5-[4-(trifluormethyl)phenyl]piperidin-1-yl}(morpholin-4-yl)methanon [racemisches cis-Isomer]

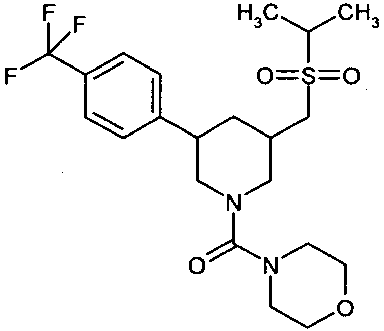

140 mg (0.325 mmol) des Thioethers aus Beispiel 31 in 14.0 ml Dichlormethan wurden mit 168 mg (0.488 mmol, 50%ig) *meta*-Chlorperoxybenzoesäure versetzt und 1 h bei RT gerührt. Die Reaktionslösung wurde im Vakuum eingeengt und der Rückstand mittels präparativer HPLC gereinigt. Ausbeute: 79 mg (52% d. Th.).

LC-MS (Methode 8B): Rₜ = 1.03 min; MS (ESIpos): m/z = 463 [M+H]⁺;

¹H-NMR (400 MHz, DMSO-d₆): δ = 7.70 (d, 2H), 7.52 (d, 2H), 3.96 (d, 1H), 3.65 (d, 1H), 3.56 (d, 4H), 3.30-3.21 (m, 2H), 3.19-3.05 (m, 6H), 3.02-2.90 (m, 1H), 2.85-2.73 (m, 1H), 2.65 (t, 1H), 2.33-2.21 (m, 1H), 2.10 (d, 1H), 1.56 (q, 1H), 1.25 (d, 6H); ein Proton verdeckt.

### Beispiel 35

### {3-[(Ethylsulfinyl)methyl]-5-[4-(trifluormethyl)phenyl}piperidin-1-yl}(morpholin-4-yl)methanon [racemisches cis-Diasteromerengemisch]

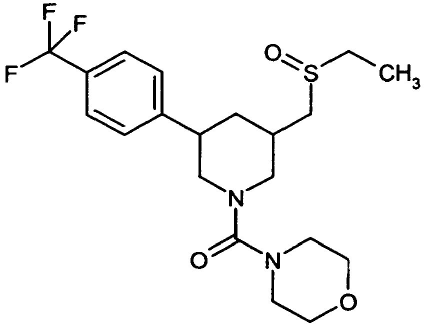

160 mg (0.384 mmol) des Thioethers aus Beispiel 32 in 16.0 ml Dichlormethan wurden mit 199 mg (0.576 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 60 mg (34% d. Th.)

LC-MS (Methode 1B): Rₜ = 1.04 min; MS (ESIpos): m/z = 433 [M+H]⁺;

¹H-NMR (400 MHz, DMSO-d₆): δ = 7.69 (d, 2H), 7.53 (d, 2H), 3.91-3.76 (m, 1H), 3.64 (d, 1H), 3.57 (t, 4H), 3.16 (d, 4H), 3.03-2.88 (m, 1H), 2.88-2.74 (m, 2H), 2.75-2.56 (m, 4H), 2.21-1.98 (m, 2H), 1.55 (quin, 1H), 1.19 (dt, 3H).

### Beispiel 36

### {3-[(Ethylsulfonyl)methyl]-5-[4-(trifluormethyl)phenyl]piperidin-1-yl}(morpholin-4-yl)methanon [racemisches cis-Isomer]

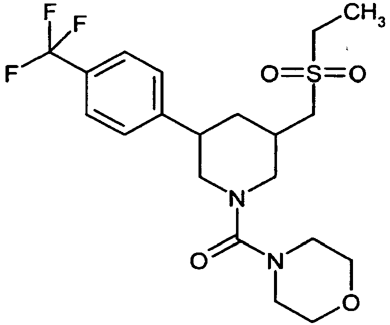

160 mg (0.384 mmol) des Thioethers aus Beispiel 32 in 16.0 ml Dichlormethan wurden mit 199 mg (0.576 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 83 mg (48% d. Th.)

LC-MS (Methode 1B): Rₜ = 1.11 min; MS (ESIpos): m/z = 449 [M+H]⁺;

¹H-NMR (400 MHz, DMSO-d₆): δ = 7.70 (d, 2H), 7.52 (d, 2H), 3.95 (d, 1H), 3.64 (d, 1H), 3.56 (d, 4H), 3.21-3.06 (m, 8H), 3.02-2.88 (m, 1H), 2.86-2.75 (m, 1H), 2.69-2.58 (m, 1H), 2.26 (br s, 1H), 2.13-2.03 (m, 1H), 1.55 (q, 1H), 1.23 (t, 6H).

### Beispiel 37

### Morpholin-4-yl{3-[(phenylsulfanyl)methyl]-5-[4-(trifluormethyl)phenyl]piperidin-1-yl}methanon [racemisches cis-Isomer]

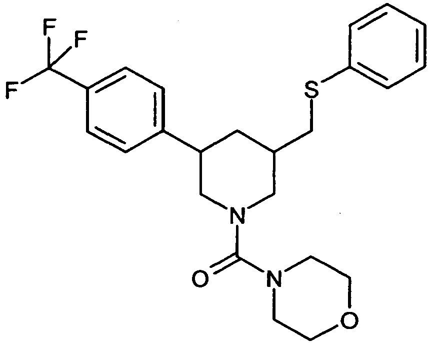

Unter Argon wurden 618 mg (5.55 mmol) Thiophenol in 10.0 ml *N,N*'-Dimethylformamid vorgelegt, mit Molsieb 3Å und 67 mg (1.67 mmol, 60%ig in Paraffinöl) Natriumhydrid versetzt und 30 min bei RT gerührt. Anschließend wurden 250 mg (0.555 mmol) des Mesylats aus Beispiel 7A in 5.0 ml *N,N*'-Dimethylformamid zugesetzt und für 3 h bei RT gerührt. Die Reaktion wurde durch Zugabe von Wasser beendet, das Molsieb abfiltriert und das Filtrat mittels präparativer HPLC gereinigt. Ausbeute: 211 mg (82% d. Th.)

LC-MS (Methode 1B): Rₜ = 1.46 min; MS (ESIpos): m/z = 465 [M+H]⁺;

¹H-NMR (400 MHz, DMSO-d₆): δ = 7.68 (d, 2H), 7.51 (d, 2H), 7.41-7.27 (m, 4H), 7.25-7.15 (m, 1H), 3.88 (d, 1H), 3.63 (d, 1H), 3.53 (t, 4H), 3.16-2.97 (m, 5H), 2.95-2.72 (m, 3H), 2.57 (s, 1H), 2.15-1.99 (m, 1H), 1.82 (br s, 1H), 1.49 (q, 1H).

### Beispiel 38

### {3-[(Benzylsulfanyl)methyl]-5-[4-(trifluormethyl)phenyl]piperidin-1-yl}(morpholin-4-yl)methanon [racemisches cis-Isomer]

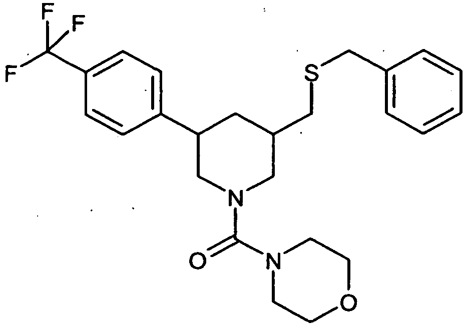

Unter Argon wurden 689 mg (5.55 mmol) Benzylthiol in 10.0 ml *N,N*'-Dimethylformamid vorgelegt, mit Molsieb 3Å und 67 mg (1.67 mmol, 60%ig in Paraffinöl) Natriumhydrid versetzt und 30 min bei RT gerührt. Anschließend wurden 250 mg (0.555 mmol) des Mesylats aus Beispiel 7A in 5.0 ml *N,N*'-Dimethylformamid zugesetzt und für 3 h bei RT gerührt. Die Reaktion wurde durch Zugabe von Wasser beendet, das Molsieb abfiltriert und das Filtrat mittels präparativer HPLC gereinigt. Ausbeute: 137 mg (52% d. Th.)

LC-MS (Methode 8B): Rₜ = 1.33 min; MS (ESIpos): m/z = 479 [M+H]⁺;

¹H-NMR (400 MHz, DMSO-d₆): δ = 7.68 (d, 2H), 7.50 (d, 2H), 7.39-7.17 (m, 5H), 3.83-3.71 (m, 3H), 3.68-3.50 (m, 5H), 3.14 (d, 4H), 2.83 (d, 1H), 2.78-2.69 (m, 1H), 2.48-2.26 (m, 3H), 1.96 (d, 1H), 1.76 (br s, 1H), 1.36 (q, 1H).

### Beispiel 39

### {3-[(Cyclohexylsulfanyl)methyl]-5-[4-(trifluormethyl)phenyl]piperidin-1-yl}(morpholin-4-yl)-methanon [racemisches cis-Isomer]

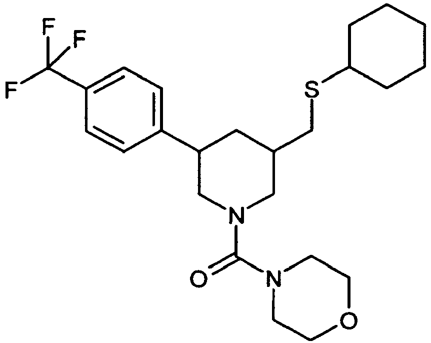

Unter Argon wurden 645 mg (5.55 mmol) Cyclohexylthiol in 8.0 ml *N,N*'-Dimethylformamid vorgelegt, mit Molsieb 3Å und 67 mg (1.67 mmol, 60%ig in Paraffinöl)) Natriumhydrid versetzt und 30 min bei RT gerührt. Anschließend wurden 250 mg (0.555 mmol) des Mesylats aus Beispiel 7A in 2.0 ml *N,N*'-Dimethylformamid zugesetzt und für 3 h bei RT gerührt. Die Reaktion wurde durch Zugabe von Wasser beendet, das Molsieb abfiltriert und das Filtrat mittels präparativer HPLC gereinigt. Ausbeute: 166 mg (64% d. Th.)

LC-MS (Methode 7B): Rₜ = 2.92 min; MS (ESIpos): m/z = 471 [M+H]⁺;

¹H-NMR (400 MHz, DMSO-d₆): δ = 7.68 (d, 2H), 7.51 (d, 2H), 3.86 (d, 1H), 3.64 (d, 1H), 3.57 (t, 4H), 3.14 (br s, 4H), 2.94-2.83 (m, 1H), 2.83-2.73 (m, 1H), 2.67 (d, 1H), 2.48-2.31 (m, 2H), 2.02 (d, 1H), 1.91 (br s, 2H), 1.81-1.62 (m, 3H), 1.55 (d, 1H), 1.40 (q, 1H), 1.30-1.17 (m, 4H).

### Beispiel 40

### (3-{[(3-Fluorphenyl)sulfanyl]methyl}-5-[4-(trifluormethyl)phenyl]piperidin-1-yl)(morpholin-4-yl)methanon [racemisches cis-Isomer]

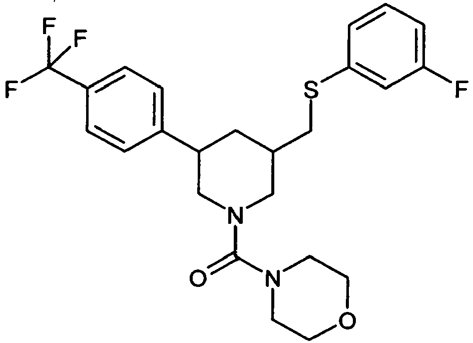

Unter Argon wurden 748 mg (5.55 mmol) 3-Fluorthiophenol in 8.0 ml *N,N*'-Dimethylformamid vorgelegt, mit Molsieb 3Å und 67 mg (1.67 mmol, 60%ig in Paraffinöl) Natriumhydrid versetzt und 30 min bei RT gerührt. Anschließend wurden 250 mg (0.555 mmol) des Mesylats aus Beispiel 7A in 2.0 ml *N,N*'-Dimethylformamid zugesetzt und für 3 h bei RT gerührt. Die Reaktion wurde durch Zugabe von Wasser beendet, das Molsieb abfiltriert und das Filtrat mittels präparativer HPLC gereinigt. Ausbeute: 220 mg (82% d. Th.)

LC-MS (Methode 7B): Rₜ = 2.73 min; MS (ESIpos): m/z = 483 [M+H]⁺;

¹H-NMR (400 MHz, DMSO-d₆): δ = 7.69 (d, 2H), 7.51 (d, 2H), 7.42-7.30 (m, 1H), 7.23 (d, 1H), 7.18 (d, 1H), 7.01 (dt, 1H), 3.86 (d, 1H), 3.62 (d, 1H), 3.53 (t, 4H), 3.14-3.03 (m, 5H), 3.01-2.93 (m, 1H), 2.92-2.83 (m, 1H), 2.83-2.74 (m, 1H), 2.62-2.55 (m, 1H), 2.14-2.03 (m, 1H), 1.83 (br s, 1H), 1.49 (q, 1H).

### Beispiel 41

### Morpholin-4-yl{3-[(phenylsulfinyl)methyl]-5-[4-(trifluormethyl)phenyl]piperidin-1-yl}methanon [racemisches cis-Diasteromerengemisch]

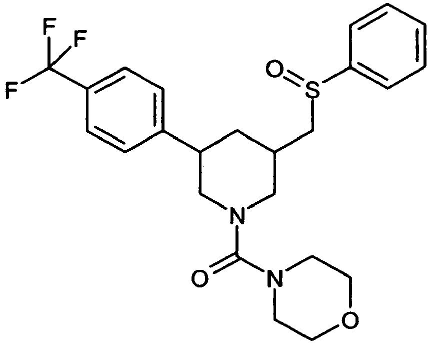

160 mg (0.344 mmol) des Thioethers aus Beispiel 37 in 15.0 ml Dichlormethan wurden mit 178 mg (0.576 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 60 mg (36% d. Th.)

LC-MS (Methode 8B): Rₜ = 1.05 min; MS (ESIpos): m/z = 481 [M+H]⁺.

### Beispiel 42

### Morpholin-4-yl{3-[(phenylsulfonyl)methyl]-5-[4-(trifluormethyl)phenyl]piperidin-1-yl}methanon [racemisches cis-Isomer]

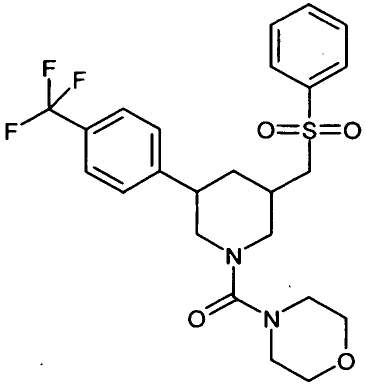

160 mg (0.344 mmol) des Thioethers aus Beispiel 37 in 15.0 ml Dichlormethan wurden mit 178 mg (0.576 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 83 mg (48% d. Th.)

LC-MS (Methode 8B): Rₜ = 1.09 min; MS (ESIpos): m/z = 497 [M+H]⁺;

¹H-NMR (500 MHz, DMSO-d₆): δ = 7.94 (d, 2H), 7.76 (d, 1H), 7.72-7.65 (m, 4H), 7.47 (d, 2H), 3.93 (d, 1H), 3.66-3.52 (m, 5H), 3.47-3.28 (m, 2H), 3.13 (d, 4H), 2.94-2.82 (m, 1H), 2.81-2.72 (m, 1H), 2.64 (t, 1H), 2.00 (d, 2H), 1.52 (q, 1H).

### Beispiel 43

### {3-[(Cyclohexylsulfinyl)methyl]-5-[4-(trifluormethyl)phenyl]piperidin-1-yl}(morpholin-4-yl)-methanon [racemisches cis-Diasteromerengemisch]

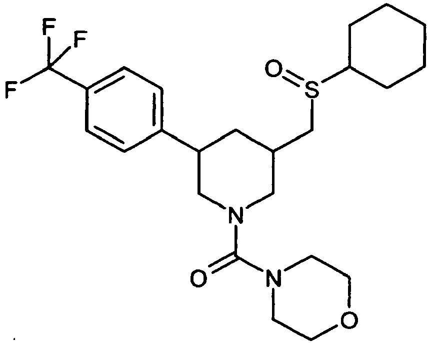

120 mg (0.255 mmol) des Thioethers aus Beispiel 39 in 11.0 ml Dichlormethan wurden mit 132 mg (0.382 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 32 mg (26% d. Th.)

LC-MS (Methode 8B): Rₜ = 1.09 und 1.10 min; MS (ESIpos): m/z = 487 [M+H]⁺.

### Beispiel 44

### {3-[(Cyclohexylsulfonyl)methyl]-5-[4-(trifluormethyl)phenyl]piperidin-1-yl}(morpholin-4-yl)-methanon [racemisches cis-Isomer]

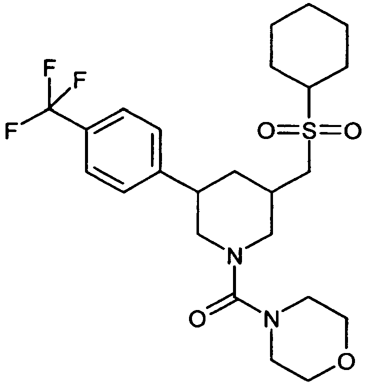

120 mg (0.255 mmol) des Thioethers aus Beispiel 39 in 11.0 ml Dichlormethan wurden mit 132 mg (0.382 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 59 mg (46% d. Th.)

LC-MS (Methode 8B): Rₜ = 1.16 min; MS (ESIpos): m/z = 503 [M+H]⁺;

¹H-NMR (500 MHz, DMSO-d₆): δ = 7.69 (d, 2H), 7.51 (d, 2H), 3.96 (d, 1H), 3.65 (d, 1H), 3.57 (t, 4H), 3.15 (d, 4H), 3.10-3.01 (m, 3H), 3.00-2.91 (m, 1H), 2.83-2.73 (m, 1H), 2.64 (t, 1H), 2.27 (br s, 1H), 2.16-2.01 (m, 3H), 1.82 (d, 2H), 1.64 (d, 1H), 1.55 (q, 1H), 1.44-1.22 (m, 4H), 1.21-1.09 (m, 1H).

### Beispiel 45

### (3-{[(3-Fluorphenyl)sulfinyl]methyl}-5-[4-(trifluormethyl)phenyl]piperidin-1-yl)(morpholin-4-yl)-methanon [racemisches cis-Diasteromerengemisch]

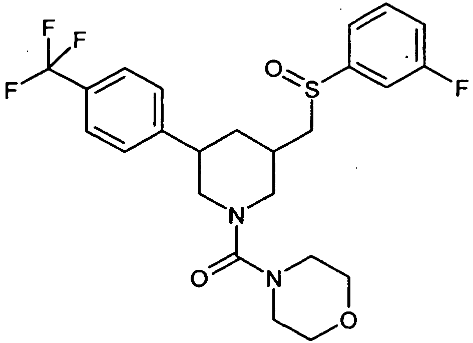

160 mg (0.332 mmol) des Thioethers aus Beispiel 40 in 14.0 ml Dichlormethan wurden mit 172 mg (0.576 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 65 mg (40% d. Th.)

LC-MS (Methode 1B): Rₜ = 1.22 min; MS (ESIpos): m/z = 499 [M+H]⁺.

### Beispiel 46

### (3-{[(3-Fluorphenyl)sulfonyl]methyl}-5-[4-(trifluormethyl)phenyl]piperidin-1-yl)(morpholin-4-yl)methanon [racemisches cis-Isomer]

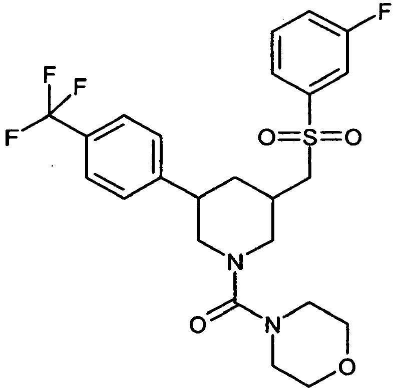

160 mg (0.332 mmol) des Thioethers aus Beispiel 40 in 14.0 ml Dichlormethan wurden mit 172 mg (0.576 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 55 mg (32% d. Th.)

LC-MS (Methode 1B): Rₜ = 1.27 min; MS (ESIpos): m/z = 515 [M+H]⁺;

¹H-NMR (500 MHz, DMSO-d₆): δ = 7.83-7.72 (m, 3H), 7.71-7.60 (m, 3H), 7.48 (d, 2H), 3.92 (d, 1H), 3.65-3.53 (m, 5H), 3.51-3.36 (m, 2H), 3.13 (d, 4H), 2.96-2.84 (m, 1H), 2.82-2.73 (m, 1H), 2.64 (t, 1H), 2.14-1.98 (m, 3H), 1.53 (q, 4H).

### Beispiel 47

### {3-[(Cyclopentylsulfanyl)methyl]-5-[4-(trifluormethyl)phenyl]piperidin-1-yl}(morpholin-4-yl)methanon [racemisches cis-Isomer]

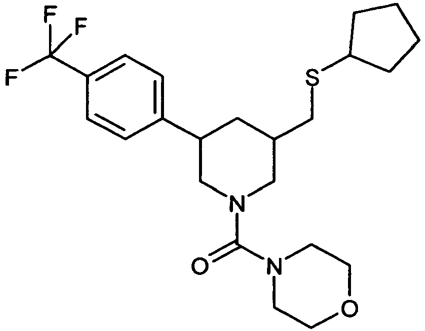

Unter Argon wurden 749 mg (7.33 mmol) Cyclopentylthiol in 11.0 ml *N,N*'-Dimethylformamid vorgelegt, mit Molsieb 3Å und 88 mg (2.20 mmol, 60%ig in Paraffinöl) Natriumhydrid versetzt und 30 min bei RT gerührt. Anschließend wurden 330 mg (0.733 mmol) des Mesylats aus Beispiel 7A in 2.0 ml *N,N*'-Dimethylformamid zugesetzt und für 3 h bei RT gerührt. Die Reaktion wurde durch Zugabe von Wasser beendet, das Molsieb abfiltriert und das Filtrat mittels präparativer HPLC gereinigt. Ausbeute: 83 mg (25% d. Th.)

LC-MS (Methode 7B): Rₜ = 2.82 min; MS (ESIpos): m/z = 457 [M+H]⁺;

¹H-NMR (400 MHz, DMSO-d₆): δ = 7.68 (d, 2H), 7.51 (d, 2H), 3.86 (d, 1H), 3.64 (d, 1H), 3.57 (t, 4H), 3.20-3.05 (m, 6H), 2.94-2.84 (m, 1H), 2.83-2.74 (m, 1H), 2.48-2.36 (m, 3H), 2.07-1.88 (m, 3H), 1.79 (br s, 1H), 1.72-1.61 (m, 2H), 1.60-1.48 (m, 2H), 1.47-1.34 (m, 3H).

### Beispiel 48

### {3-[(Cyclopentylsulfonyl)methyl]-5-[4-(trifluormethyl)phenyl]piperidin-1-yl}(morpholin-4-yl)methanon [racemisches cis-Isomer]

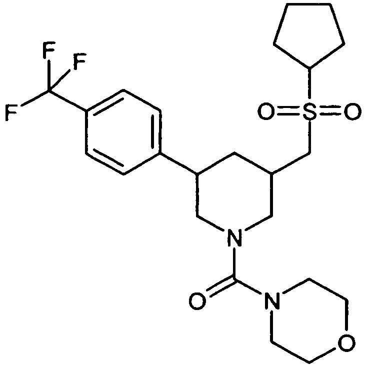

40 mg (0.088 mmol) des Thioethers aus Beispiel 47 in 4.0 ml Dichlormethan wurden mit 61 mg (0.175 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 37 mg (87% d. Th.)

LC-MS (Methode 8B): Rₜ = 1.08 min; MS (ESIpos): m/z = 489 [M+H]⁺;

¹H-NMR (400 MHz, DMSO-d₆): δ = 7.70 (d, 2H), 7.51 (d, 2H), 3.96 (d, 1H), 3.69-3.51 (m, 6H), 3.14 (br s, 4H), 3.11-3.04 (m, 1H), 2.83-2.74 (m, 1H), 2.63 (d, 1H), 2.07 (s, 6H), 1.90 (br s, 3H), 1.71-1.53 (m, 4H).

### Beispiel 49

### {3-[(Benzylsulfinyl)methyl]-5-[4-(trifluormethyl)phenyl]piperidin-1-yl}(morpholin-4-yl)methanon [racemisches cis-Diasteromerengemisch]

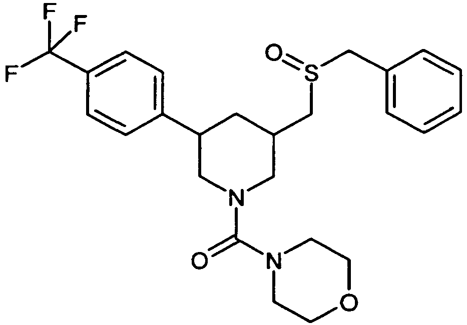

100 mg (0.209 mmol) des Thioethers aus Beispiel 38 in 9.0 ml Dichlormethan wurden mit 108 mg (0.315 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 41 mg (38% d. Th.)

LC-MS (Methode 7B): Rₜ = 2.23 min; MS (ESIpos): m/z = 495 [M+H]⁺;

¹H-NMR (400 MHz, DMSO-d₆): δ = 7.68 (d, 2H), 7.52 (d, 2H), 7.43-7.28 (m, 5H), 4.17 (dd, 1H), 4.00 (d, 1H), 3.80 (t, 1H), 3.63 (d, 1H), 3.56 (br s, 4H), 3.21-3.08 (m, 4H), 3.01-2.76 (m, 2H), 2.75-2.57 (m, 3H), 2.23-1.87 (m, 2H), 1.64-1.44 (m, 1H).

### Beispiel 50

### {3-[(Benzylsulfonyl)methyl]-5-[4-(trifluormethyl)phenyl]piperidin-1-yl}(morpholin-4-yl)-methanon [racemisches cis-Isomer]

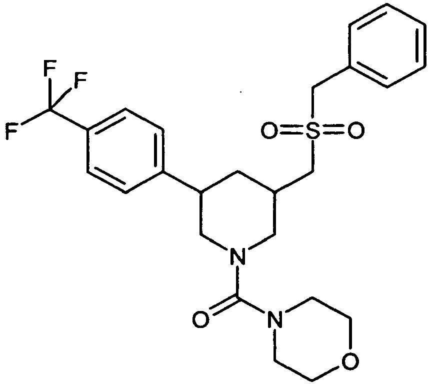

100 mg (0.209 mmol) des Thioethers aus Beispiel 38 in 9.0 ml Dichlormethan wurden mit 108 mg (0.315 mmol, 50%ig) *meta*-Chlorperoxybenzoesäure versetzt und 45 min bei RT gerührt. Die Reaktionslösung wurde im Vakuum eingeengt und der Rückstand mittels präparativer HPLC gereinigt. Ausbeute: 37 mg (34% d. Th.)

LC-MS (Methode 7B): Rₜ = 2.36 min; MS (ESIpos): m/z = 511 [M+H]⁺;

¹H-NMR (400 MHz, DMSO-d₆): δ = 7.69 (d, 2H), 7.50 (d, 2H), 7.44-7.35 (m, 5H), 4.58-4.46 (m, 2H), 3.90 (d, 1H), 3.62 (d, 1H), 3.56 (d, 4H), 3.13 (br. s., 4H), 3.07 (d, 2H), 2.96-2.85 (m, 1H), 2.80-2.71 (m, 1H), 2.66-2.56 (m, 1H), 2.22 (br s, 1H), 2.06-1.94 (m, 1H), 1.53 (q, 1H).

### Beispiel 51

### (3-{[(2-Methoxyethyl)(methyl)amino]methyl}-5-[4-(trifluormethyl)phenyl]piperidin-1-yl)-(morpholin-4-yl)methanon [racemisches cis-Isomer]

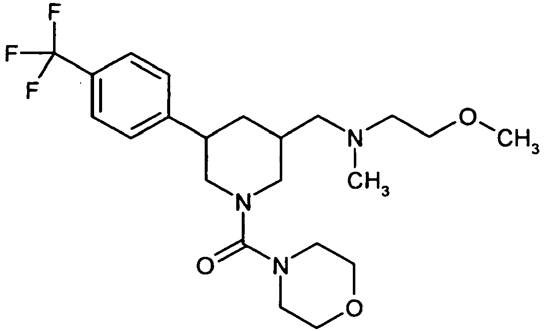

Nach der Allgemeinen Methode 7 wurden 300 mg (ca. 0.333 mmol) der Verbindung aus Beispiel 7A und 356 mg (3.996 mmol) Methoxyethanamin umgesetzt. Ausbeute: 63 mg (40% d. Th.)

HPLC (Methode 8B): Rₜ = 0.80 min; MS (ESIpos): m/z = 444 [M+H]⁺.

### Beispiel 52

### (3-{[Cyclopropyl(methyl)amino]methyl}-5-[4-(trifluormethyl)phenyl]piperidin-1-yl)(morpholin-4-yl)methanon [racemisches cis-Isomer]

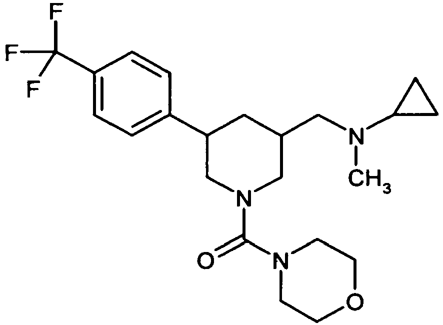

Nach der Allgemeinen Methode 8 wurden 300 mg (ca. 0.333 mmol) der Verbindung aus Beispiel 7A und 341 mg (4.794 mmol) *N*-Methylcyclopropanamin umgesetzt. Ausbeute: 29 mg (17% d. Th.)

HPLC (Methode 8B): Rₜ = 0.80 min; MS (ESIpos): m/z = 426 [M+H]⁺.

### Beispiel 53

### {3-[(3-Hydroxypyrrolidin-1-yl)methyl]-5-[4-(trifluormethyl)phenyl]piperidin-1-yl}(morpholin-4-yl)methanon [racemisches cis-Isomer]

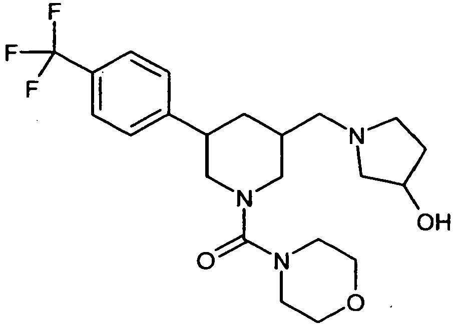

Nach der Allgemeinen Methode 8 wurden 300 mg (ca. 0.333 mmol) der Verbindung aus Beispiel 7A und 292 mg (4.794 mmol) 3-Pyrrolidinol umgesetzt. Ausbeute: 79 mg (45% d. Th.)

HPLC (Methode 8B): Rₜ = 0.75 min; MS (ESIpos): m/z = 442 [M+H]⁺.

### Beispiel 54

### {1-[(1,1-Dioxidothiomorpholin-4-yl)carbonyl]-5-[4-(trifluormethyl)phenyl]piperidin-3-yl}methyl-propylcarbamat [racemisches cis-Isomer]

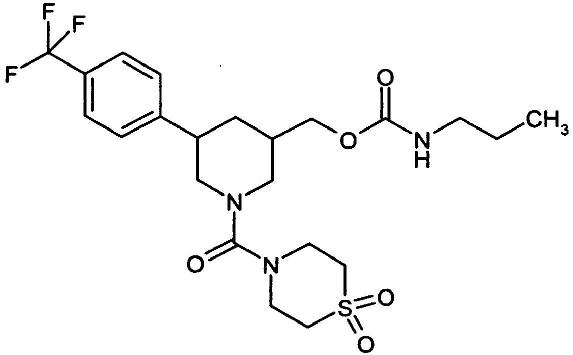

Nach der Allgemeinen Methode 5 wurden 115 mg (0.120 mmol) der Verbindung aus Beispiel 16A und 12 mg (0.144 mmol) Isobutylisocyanat umgesetzt. Ausbeute: 19 mg (29% d. Th.)

LC-MS (Methode 8B): Rₜ = 1.0 min; MS (ESIpos): m/z = 506 [M+H]⁺;

¹H-NMR (500 MHz, DMSO-d₆): δ = 7.69 (d, 2H), 7.52 (d, 1H), 7.15-7.13 (m, 1H), 3.95-3.82 (m, 2H), 3.78-3.66 (m, 2H), 3.60-3.54 (m, 3H), 3.19-3.12 (m, 3H), 2.95-2.78 (m, 4H), 1.92 (m, 2H), 1.46-1.35 (m, 3H), 0.82 (t, 3H).

### Beispiel 55

### N-({1-(Morpholin-4-ylcarbonyl)-5-[4-(trifluormethyl)phenyl]piperidin-3-yl}methyl)cyclopropan-carboxamid [enantiomerenreines cis-Isomer]

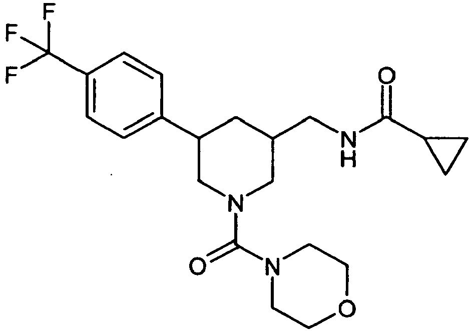

Nach der Allgemeinen Methode 2 wurden 129 mg (ca. 0.219 mmol) {3-(Aminomethyl)-5-[4-(trifluormethyl)phenyl]piperidin-1-yl}(morpholin-4-yl)methanon umgesetzt. Enantiomerentrennung des Racemates nach Methode 5D ergab 13 mg der Titelverbindung aus Beispiel 55 und 13 mg der Titelverbindung aus Beispiel 56.

LC-MS (Methode 8B): Rₜ = 0.97 min; MS (ESIpos): m/z = 440 [M+H]⁺;

HPLC (Methode 4E): Rₜ = 6.67 min, >99.0% ee;

¹H-NMR (400 MHz, DMSO-d₆): δ = 8.16-8.13 (m, 1H), 7.68 (d, 2H), 7.52 (d, 2H), 3.68-3.62 (m, 2H), 3.60-3.55 (m, 4H), 3.17-3.07 (m, 4H), 3.04-2.99 (m, 2H), 2.89-2.71 (m, 2H); 1.89 (d, 1H), 1.80-1.65 (m, 1H), 1.57-1.50 (m, 1H), 1.37 (q, 1H), 0.70-0.60 (m, 4H).

### Beispiel 56

### N-({1-(Morpholin-4-ylcarbonyl)-5-[4-(trifluormethyl)phenyl]piperidin-3-yl}methyl)cyclopropan-carboxamid [enantiomerenreines cis-Isomer]

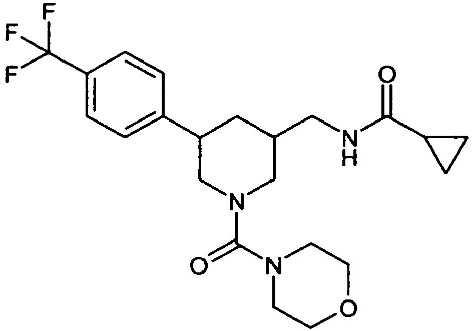

Nach der Allgemeinen Methode 2 wurden 129 mg (ca. 0.219 mmol) {3-(Aminomethyl)-5-[4-(trifluormethyl)phenyl]piperidin-1-yl}(morpholin-4-yl)methanon umgesetzt. Enantiomerentrennung des Racemates nach Methode 5D ergab 13 mg der Titelverbindung aus Beispiel 55 und 13 mg der Titelverbindung aus Beispiel 56.

LC-MS (Methode 8B): Rₜ = 0.97 min; MS (ESIpos): m/z = 440 [M+H]⁺;

HPLC (Methode 4E): Rₜ = 7.14 min, >85.0% ee;

¹H-NMR (400 MHz, DMSO-d₆): δ = 8.16-8.13 (m, 1H), 7.68 (d, 2H), 7.52 (d, 2H), 3.68-3.62 (m, 2H), 3.60-3.55 (m, 4H), 3.17-3.07 (m, 4H), 3.04-2.99 (m, 2H), 2.89-2.71 (m, 2H); 1.89 (d, 1H), 1.80-1.65 (m, 1H), 1.57-1.50 (m, 1H), 1.37 (q, 1H), 0.70-0.60 (m, 4H).

### B) Bewertung der physiolosischen Wirksamkeit

### Abkürzungen:

- BSA: Rinderserum-Albumin
- DMEM: Dulbecco's Modified Eagle Medium
- EGTA: Ethylenglykol-glykol-bis-(2-aminoäthyl)-*N,N*,*N',N*'-tetra-essigsäure
- FCS: Fetal Calf Serum
- HEPES: 4-(2-Hydroxyethyl)-1-piperazinethansulfonsäure
- [3H]haTRAP: tritiated high affinity thrombin receptor activating peptide
- PRP: Plättchenreiches Plasma

Die Eignung der erfindungsgemäßen Verbindungen zur Behandlung von thromboembolischen Erkrankungen kann in folgenden Assaysystemen gezeigt werden:

### 1.) In vitro Assays

### 1.a) Zellulärer, funktioneller in vitro-Test

Die Identifizierung von Antagonisten des humanen Protease Aktivierten Rezeptors 1 (PAR-1) sowie die Quantifizierung der Wirksamkeit der hier beschriebenen Substanzen erfolgt mit Hilfe einer rekombinanten Zelllinie. Die Zelle leitet sich ursprünglich von einer embryonalen Nierenzelle des Menschen (HEK293; ATCC: American Type Culture Collection, Manassas, VA 20108, USA) ab. Die Testzelllinie exprimiert konstitutiv eine modifizierte Form des calciumsensitiven Photoproteins Aequorin, das nach Rekonstitution mit dem Co-Faktor Coelenterazin bei Erhöhungen der freien Calcium-Konzentration im inneren mitochondrialen Kompartiment Licht emittiert (Rizzuto R, Simpson AW, Brini M, Pozzan T.; Nature 1992, 358, 325-327). Zusätzlich exprimiert die Zelle stabil den endogenen humanen PAR-1-Rezeptor sowie den endogenen purinergen Rezeptor P2Y2. Die resultierende PAR-1-Testzelle reagiert auf Stimulation des endogenen PAR-1 oder P2Y2-Rezeptors mit einer intrazellulären Freisetzung von Calcium-Ionen, die durch die resultierende Aequorin-Lumineszens mit einem geeigneten Luminometer quantifiziert werden kann (Milligan G, Marshall F, Rees S, Trends in Pharmacological Sciences 1996, 17, 235-237).

Für die Prüfung der Substanz-Spezifität wird deren Wirkung nach Aktivierung des endogenen PAR-1-Rezeptors mit der Wirkung nach Aktivierung des endogenen purinergen P2Y2-Rezeptors verglichen, der den gleichen intrazellulären Signalweg nutzt.

Testablauf: Die Zellen werden zwei Tage (48 Std.) vor dem Test in Kulturmedium (DMEM F12, ergänzt mit 10% FCS, 2 mM Glutamine, 20 mM HEPES, 1.4 mM Pyruvat, 0.1 mg/ml Gentamycin, 0.15% Na-Bicarbonat; BioWhittaker Cat.# BE04-687Q; B-4800 Verviers, Belgien) in 384-Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (in mM: 140 Natriumchlorid, 5 Kaliumchlorid, 1 Magnesiumchlorid, 2 Calciumchlorid, 20 Glucose, 20 HEPES), das zusätzlich den Co-Faktor Coelenterazin (25 µM) und Glutathion (4 mM) enthält, ausgetauscht und die Mikrotiterplatte anschließend für weitere 3-4 Stunden inkubiert. Dann werden die Testsubstanzen auf die Mikrotiterplatte pipettiert und 5 Minuten nach Übertragung der Testsubstanzen in die Wells der Mikrotiterplatte wird die Platte in das Luminometer transferiert, eine PAR-1-Agonist-Konzentration, die EC₅₀ entspricht, zugeschossen und sofort das resultierende Lichtsignal im Luminometer gemessen. Zur Unterscheidung einer Antagonist-Substanzwirkung von einer toxischen Wirkung wird unmittelbar anschließend der endogene purinerge Rezeptor mit Agonist aktiviert (ATP, 10 µM Endkonzentration) und das resultierende Lichtsignal gemessen. Die Ergebnisse sind in Tabelle A gezeigt:

**Tabelle A:**

| **Beispiel-Nr.** | **IC₅₀ [nM]** |
|---|---|
| **1** | 177 |
| **6** | 8 |
| **15** | 56 |
| **40** | 3 |
| **48** | 24 |

### 1.b) PAR-1 Rezeptor Bindungsassay

Thrombozytenmembranen werden mit 12 nM [3H]haTRAP und Testsubstanz in verschiedenen Konzentrationen in einem Puffer (50 mM Tris pH 7.5, 10 mM Magnesiumchlorid, 1 mM EGTA, 0.1% BSA) bei Raumtemperatur für 80 min inkubiert. Danach wird der Ansatz auf eine Filterplatte übertragen und zweimal mit Puffer gewaschen. Nach Zugabe von Scintillationsflüssigkeit wird die Radioaktivität auf dem Filter in einem beta-Counter vermessen.

### 1.c) Thrombozytenaggregation in Plasma

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natrium Citrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert.

Für die Aggregationsmessungen werden Aliquots des plättchenreichen Plasmas mit aufsteigenden Konzentrationen an Prüfsubstanz 10 min bei 37°C inkubiert. Anschließend wird die Aggregation durch Zugabe eines Thrombin-Rezeptor Agonisten (TRAP6, SFLLRN) in einem Aggregometer ausgelöst und mittels der turbidimetrischen Methode nach Born (Born, G.V.R., Cross M.J., The Aggregation of Blood Platelets; J Physiol. 1963, 168, 178-195) bei 37°C bestimmt. Die SFLLRN-Konzentration, die zur maximalen Aggregation führt, wird gegebenenfalls jeweils für jeden Spender individuell ermittelt.

Zur Berechnung der inhibitorischen Wirkung wird die maximale Zunahme der Lichttransmission (Amplitude der Aggregationskurve in %) innerhalb 5 Minuten nach Zugabe des Agonisten in Gegenwart und Abwesenheit von Prüfsubstanz ermittelt und die Inhibition berechnet. Aus den Inhibitionskurven wird die Konzentration berechnet, die die Aggregation zu 50% hemmt.

### 1.d) Thrombozytenaggregation in Puffer

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1000g zentrifugiert. Das Thrombozyten-Pellet wird mit WaschPuffer resuspendiert und 10 Minuten bei 1000g zentrifugiert. Die Thrombozyten werden in Inkubations-Puffer resuspendiert und auf 200000 Z/µl eingestellt. Vor Versuchsbeginn wird Calciumchlorid und Magnesiumchlorid, Endkonzentration je 2mM (Stammlösung 2M, 1:1000 Verdünnung) zugegeben. Besonderheit: bei ADP-induzierter Aggregation wird nur Calciumchlorid zugegeben. Folgende Agonisten können eingesetzt werden: TRAP6-Trifluoracetat- Salz, Kollagen, Humanes a-Thrombin und U-46619. Die Konzentration des Agonisten wird zu jedem Spender ausgetestet.

Testdurchführung: Es werden 96er-Loch Mikrotiterplatten verwendet. Die Prüfsubstanz wird in DMSO verdünnt und 2 µl je Loch vorgelegt. 178 µl Thrombozyten-Suspension werden zugegeben und 10 Minuten bei Raumtemperatur vorinkubiert. 20 µl Agonist werden zugegeben und die Messung im Spectramax, OD 405 nm, sofort gestartet. Die Kinetik wird in 11 Messungen von je 1 Minute bestimmt. Zwischen den Messungen wird 55 Sekunden geschüttelt.

### 1.e) Thrombozytenaggregation in fibrinogendepletiertem Plasma

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen.

Herstellung von fibrinogendepletiertem Plasma: Zur Gewinnung von plättchenarmem Plasma wird das Citrat-Vollblut bei 140g für 20 min abzentrifugiert. Das plättchenarme Plasma wird im Verhältnis 1:25 mit Reptilase (Roche Diagnostic, Deutschland) versetzt und vorsichtig invertiert. Es folgen 10 min Inkubation bei 37°C im Wasserbad mit direkt folgender Inkubation auf Eis für 10 min. Das Plasma-Reptilase Gemisch wird bei 1300g für 15 min zentrifugiert und der Überstand (fibrinogendepletiertes Plasma) wird gewonnen.

Thrombozyten-Isolierung; Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1300g zentrifugiert. Das Thrombozyten-Pellet wird mit WaschPuffer resuspendiert und 10 Minuten bei 1300g zentrifugiert. Die Thrombozyten werden in Inkubations-Puffer resuspendiert und auf 400000 Z/µl eingestellt und Calciumchloridlösung mit einer Endkonzentration von 5 mM (1/200Verdünnung) zugegeben.

Für die Aggregationsmessungen werden Aliquots (98 µl fibrinogendepletiertes Plasma und 80 µl Thrombozytensuspension) mit aufsteigenden Konzentrationen an Prüfsubstanz 10 min bei RT inkubiert. Anschließend wird die Aggregation durch Zugabe von humanem alpha Thrombin in einem Aggregometer ausgelöst und mittels der turbidimetrischen Methode nach Born (Born, G.V.R., Cross M.J., The Aggregation of Blood Platelets; J. Physiol. 1963, 168, 178-195) bei 37°C bestimmt. Die alpha Thrombin Konzentration, die gerade eben zur maximalen Aggregation führt, wird jeweils für jeden Spender individuell ermittelt.

Zur Berechnung der inhibitorischen Wirkung wird die Zunahme der maximalen Lichttransmission (Amplitude der Aggregationskurve in %) innerhalb 5 Minuten nach Zugabe des Agonisten in Gegenwart und Abwesenheit von Prüfsubstanz ermittelt und die Inhibition berechnet. Aus den Inhibitionskurven wird die Konzentration berechnet, die die Aggregation zu 50% hemmt.

### 1.f) Stimulation gewaschener Thrombozyten und Analyse in der Durchflusszytometrie

Isolierung_gewaschener Thrombozyten: Humanes Vollblut wird mittels Venenpunktion von freiwilligen Spendern gewonnen und in Monovetten (Sarstedt, Nümbrecht, Deutschland) überführt, die als Antikoagulans Natriumcitrat enthalten (1 Teil Natriumcitrat 3.8% + 9 Teile Vollblut). Die Monovetten werden bei 900 Umdrehungen pro Minute und 4°C über einen Zeitraum von 20 Minuten zentrifugiert (Heraeus Instruments, Deutschland; Megafuge 1.0RS). Das plättchenreiche Plasma wird vorsichtig abgenommen und in ein 50 ml-Falconröhrchen überführt. Nun wird das Plasma mit ACD-Puffer (44 mM Natriumcitrat, 20.9 mM Zitronensäure, 74.1 mM Glucose) versetzt. Das Volumen des ACD-Puffers entspricht einem Viertel des Plasmavolumens. Durch zehnminütige Zentrifugation bei 2500 Umdrehungen und 4°C werden die Thrombozyten sedimentiert. Danach wird der Überstand vorsichtig abdekantiert und verworfen. Die präzipitierten Thrombozyten werden zunächst vorsichtig mit einem Milliliter Waschpuffer (113 mM Natriumchlorid, 4 mM Dinatriumhydrogenphosphat, 24 mM Natriumdihydrogenphosphat, 4 mM Kaliumchlorid, 0.2 mM Ethylenglycol-bis-(2-aminoethyl)-NNN'N'-tetraessigsäure, 0.1% Glucose) resuspendiert und dann mit Waschpuffer auf ein Volumen aufgefüllt, das dem der Plasmamenge entspricht. Der Waschvorgang wird ein zweites Mal durchgeführt. Nachdem die Thrombozyten durch eine erneute zehnminütige Zentrifugation bei 2500 Umdrehungen und 4°C präzipitiert worden sind, werden sie vorsichtig in einem Milliliter Inkubationspuffer (134 mM Natriumchlorid, 12 mM Natriumhydrogencarbonat, 2.9 mM Kaliumchlorid, 0.34 mM Natriumdihydrogencarbonat, 5 mM HEPES, 5 mM Glucose, 2 mM Calciumchlorid und 2 mM Magnesiumchlorid) resuspendiert und mit Inkubationspuffer auf eine Konzentration von 300.000 Thrombozyten pro µl eingestellt.

Färbung und Stimulierung der humanen Thrombozyten mit humanem α-Thrombin in Gegenwart oder Abwesenheit eines PAR-1-Antagonisten: Die Thrombozytensuspension wird mit der zu prüfenden Substanz bzw. des entsprechenden Lösungsmittels für 10 Minuten bei 37°C vorinkubiert (Eppendorf, Deutschland; Thermomixer Comfort). Durch Zugabe des Agonisten (0.5 µM bzw. 1 µM α-Thrombin; Kordia, Niederlande, 3281 NIH Units/mg; oder 30µg/ml Thrombin receptor activating peptide (TRAP6); Bachem, Schweiz) bei 37° und unter Schütteln von 500 Umdrehungen pro Minute wird die Thrombozytenaktivierung ausgelöst. Zu den Zeitpunkten 0, 1, 2.5, 5, 10 und 15 Minuten wird jeweils ein Aliquot von 50µl entnommen und in einen Milliliter einfach-konzentrierte CellFix™-Lösung (Becton Dickinson Immunocytometry Systems, USA) überführt. Zur Fixierung der Zellen werden sie 30 Minuten bei 4°C in der Dunkelheit inkubiert. Durch eine zehnminütige Zentrifugation bei 600 g und 4°C werden die Thrombozyten präzipitiert. Der Überstand wird verworfen und die Thrombozyten werden in 400 µl CellWash™ (Becton Dickinson Immunocytometry Systems, USA) resuspendiert. Ein Aliquot von 100 µl wird in ein neues FACS-Röhrchen überführt. 1µl des thrombozyten-identifizierenden Antikörpers und 1 µl des aktivierungszustands-detektierenden Antikörpers werden mit CellWash™ auf ein Volumen von 100 µl aufgefüllt. Diese Antikörperlösung wird dann zur Thrombozytensuspension gegeben und 20 Minuten bei 4°C in der Dunkelheit inkubiert. Im Anschluss an die Färbung wird das Ansatzvolumen durch Zugabe von weiteren 400 µl CellWash™ erhöht.

Zur Identifizierung der Thrombozyten wird ein fluorescein-isothiocyanat-konjugierter Antikörper eingesetzt, der gegen das humane Glykoprotein IIb (CD41) gerichtet ist (Immunotech Coulter, Frankreich; Cat. No. 0649). Mit Hilfe des phycoerythrin-konjugierten Antikörpers, der gegen das humane Glykoprotein P-Selektin (Immunotech Coulter, Frankreich; Cat. No. 1759) gerichtet ist, lässt sich der Aktivierungszustand der Thrombozyten bestimmen. P-Selektin (CD62P) ist in den α-Granula ruhender Thrombozyten lokalisiert. Es wird jedoch nach *in-vitro-* bzw. *in-vivo-*Stimulierung zur äußeren Plasmamembran translokalisiert.

Durchflusszytometrie und Auswertung der Daten: Die Proben werden im Gerät FACSCalibur™ Flow Cytometry System der Firma Becton Dickinson Immunocytometry Systems, USA, vermessen und mit Hilfe der Software CellQuest, Version 3.3 (Becton Dickinson Immunocytometry Systems, USA) ausgewertet und graphisch dargestellt. Das Maß der Thrombozytenaktivierung wird durch den Prozentsatz der CD62P-positiven Thrombozyten (CD41-positive Ereignisse) bestimmt. Es werden von jeder Probe 10.000 CD41-positive Ereignisse gezählt.

Die inhibitorische Wirkung der zu prüfenden Substanzen wird anhand der Reduktion der Thrombozytenaktivierung berechnet, die sich auf die Aktivierung durch den Agonisten bezieht.

### 1.g) Thrombozytenaggregationsmessung mit der Parallelplattenlplußkammer

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1000g zentrifugiert. Das Thrombozyten-Pellet wird mit WaschPuffer resuspendiert und 10 Minuten bei 1000g zentrifugiert. Für die Perfusionsstudie wird eine Mischung von 40% Erythrozyten und 60% gewaschene Thrombozyten (200.000/µl) hergestellt und in HEPES-Tyrode Puffer suspendiert. Die Messung der Thrombozytenaggregation unter Flußbedingungen wird mittels der Parallelplattenflußkammer "durchgeführt (B. Nieswandt et al., EMBO J. 2001, 20, 2120-2130; C. Weeterings, Arterioscler Thromb. Vasc. Biol. 2006, 26, 670-675; JJ Sixma, Thromb. Res. 1998, 92, 43-46). Glasobjektträger werden mit 100 pl humaner α-Thrombinlösung (gelöst in Tris-Puffer) über Nacht bei 4°C benetzt (α-Thrombin in verschiedenen Konzentrationen, z.B. 10 bis 50 µg/ml) und abschließend mittels 2% BSA abgeblockt.

Rekonstituiertes Blut wird über die Thrombin-benetzten Glasobjektträger über 5 Minuten mit konstanter Flußrate geleitet (z.B. Scherrate 300/Sekunde) und mittels Mikroskop-Videosystem beobachtet und aufgezeichnet. Die inhibitorische Wirkung der zu prüfenden Substanzen wird morphometrisch anhand der Reduktion der Plättchenaggregatsbildung ermittelt. Alternativ kann die Inhibierung der Plättchenaktivierung durch Durchflußzytometrie, z.B. über p-Selektin-Expression (CD62p), bestimmt werden (siehe Methode 1.f).

### 2.) Ex vivo Assay

### 2.a) Thrombozytenaggregation (Primaten, Meerschweinchen)

Meerschweinchen oder Primaten werden in wachem oder narkotisiertem Zustand oral, intravenös oder intraperitoneal mit Prüfsubstanzen in geeigneter Formulierung behandelt. Als Kontrolle werden andere Meerschweinchen oder Primaten in identischer Weise mit dem entsprechenden Vehikel behandelt. Nach je nach Applikationsart unterschiedlich langer Zeit wird aus den tief narkotisierten Tieren Blut durch Punktion des Herzens oder der Aorta gewonnen. Das Blut wird in Monovetten (Sarstedt, Nümbrecht, Deutschland) die als Antikoagulans Natriumcitrat 3.8% (1 Teil Citratlösung + 9 Teile Blut) enthalten, aufgenommen. Zur Gewinnung von plättchenreichem Plasma wird das Citrat-Vollblut bei 140g für 20 min zentrifugiert.

Die Aggregation wird durch Zugabe eines Thrombin-Rezeptor Agonisten (TRAP6, SFLLRN, 50 µg/ml; Konzentration wird in jedem Experiment je nach Tierart ermittelt) in einem Aggregometer ausgelöst und mittels der turbidimetrischen Methode nach Born (Born, G.V.R., Cross M.J., The Aggregation of Blood Platelets; J. Physiol. 1963, 168, 178-195) bei 37°C bestimmt.

Zur Aggregationsmessung wird die maximale Zunahme der Lichttransmission (Amplitude der Aggregationskurve in %) innerhalb 5 Minuten nach Zugabe des Agonisten ermittelt. Die inhibitorische Wirkung der verabreichten Prüfsubstanzen in den behandelten Tieren wird durch die Reduktion der Aggregation, bezogen auf den Mittelwert der Kontrolltiere, berechnet.

### 2.b) Thrombozytenaggregation und -aktivierungsmessung in der Parallelplattenflusskammer (Primaten)

Primaten werden in wachem oder narkotisiertem Zustand oral, intravenös oder intraperitoneal mit Prüfsubstanzen in geeigneter Formulierung behandelt. Als Kontrolle werden andere Tiere in identischer Weise mit dem entsprechenden Vehikel behandelt. Nach je nach Applikationsart unterschiedlich langer Zeit wird aus den Tieren Blut durch Venenpunktion gewonnen. Das Blut wird in Monovetten (Sarstedt, Nümbrecht, Deutschland) die als Antikoagulans Natriumcitrat 3.8% (1 Teil Citratlösung + 9 Teile Blut) enthalten, aufgenommen. Alternativ kann nicht-antikoaguliertes Blut mit Neutralmonovetten (Sarstedt) entnommen werden. In beiden Fällen wird das Blut zur Verhinderung der Fibringerinnselbildung mit Pefabloc FG (Pentapharm, Endkonzentration 3 mM) versetzt.

Citratvollblut wird vor der Messung durch Zugabe von CaCl₂-Lösung (Endkonzentration Ca⁺⁺ 5 mM) rekalzifiert. Nicht-antikoaguliertes Blut wird unmittelbar zur Messung in die Parallelplattenflusskammer eingeleitet. Die Messung der Thrombozytenaktivierung wird in der Kollagen-beschichteten Parallelplattenflußkammer morphometrisch oder durchflusszytometrisch wie in Methode 1.h) beschrieben durchgeführt.

### 3.) In vivo Assays

### 3.a) Thrombosemodelle

Die erfindungsgemäßen Verbindungen können in Thrombosemodellen in geeigneten Tierspezies, in denen die Thrombin-induzierte Plättchenaggregation über den PAR-1-Rezeptor vermittelt wird, untersucht werden. Als Tierspezies eignen sich Meerschweinchen und insbesondere Primaten (vergleiche: Lindahl, A.K., Scarborough, R.M., Naughton, M.A., Harker, L.A., Hanson, S.R., Thromb Haemost 1993, 69, 1196; Cook JJ, Sitko GR, Bednar B, Condra C, Mellott MJ, Feng D-M, Nutt RF, Shager JA, Gould RJ, Connolly TM, Circulation 1995, 91, 2961-2971; Kogushi M, Kobayashi H, Matsuoka T, Suzuki S, Kawahara T, Kajiwara A, Hishinuma I, Circulation 2003, 108 Suppl. 17, IV-280; Derian CK, Damiano BP, Addo MF, Darrow AL, D'Andrea MR, Nedelman M, Zhang H-C, Maryanoff BE, Andrade-Gordon P, J. Pharmacol. Exp. Ther. 2003, 304, 855-861). Alternativ können Meerschweinchen verwendet werden, die mit Inhibitoren von PAR-3 und/oder PAR-4 vorbehandelt werden (Leger AJ et al.; Circulation 2006, 113, 1244-1254), oder transgene PAR-3- und/oder PAR-4-Knockdown-Meerschweinchen.

### 3.b) Gerinnungsstörung und Organdysfunktion bei Disseminierter Intravasaler Gerinnung (DIC)

Die erfindungsgemäßen Verbindungen können in Modellen für DIC und/oder Sepsis in geeigneten Tierspezies untersucht. werden. Als Tierspezies eignen sich Meerschweinchen und insbesondere Primaten, bei Untersuchung der endothelvermittelten Effekte auch Mäuse und Ratten (vergleiche: Kogushi M, Kobayashi H, Matsuoka T, Suzuki S, Kawahara T, Kajiwara A, Hishinuma I, Circulation 2003, 108 Suppl. 17, IV-280; Derian CK, Damiano BP, Addo MF, Darrow AL, D'Andrea MR, Nedelman M, Zhang H-C, Maryanoff BE, Andrade-Gordon P, J. Pharmacol. Exp. Ther. 2003, 304, 855-861; Kaneider NC et al., Nat Immunol, 2007, 8, 1303-12; Camerer E et al., Blood, 2006, 107, 3912-21; Riewald M et al., J Biol Chem, 2005, 280, 19808-14.). Alternativ können Meerschweinchen verwendet werden, die mit Inhibitoren von PAR-3 und/oder PAR-4 vorbehandelt werden (Leger AJ et al., Circulation 2006, 113, 1244-1254), oder transgene PAR-3-und/oder PAR-4-Knockdown-Meerschweinchen.

### 3.b.1) Thrombin-Antithrombin-Komplexe

Thrombin-Antithrombin-Komplexe (nachfolgend als "TAT" bezeichnet) sind ein Maß für das durch Gerinnungsaktivierung endogen gebildete Thrombin. TAT werden mittels eines ELISA-Assays bestimmt (Enzygnost TAT micro, Dade-Behring). Aus Citratblut wird durch Zentrifugation Plasma gewonnen. Zu 50 µl Plasma wird 50 µl TAT-Probenpuffer gegeben, kurz geschüttelt und 15 min bei Raumtemperatur inkubiert. Die Proben werden abgesaugt, und das Well 3-malig mit Waschpuffer gewaschen (300 µl/Well). Die Platte wird zwischen den Waschgängen abgeklopft. Es wird Konjugatlösung (100 µl) hinzugegeben und 15 min bei Raumtemperatur inkubiert. Die Proben werden abgesaugt, und das Well 3-malig mit Waschpuffer gewaschen (300 µl/Well). Anschließend wird chromogenes Susbtrat hinzugegeben (100 µl/Well), 30 min im Dunkeln bei Raumtemperatur inkubiert, Stopplösung hinzugegeben (100 µl/Well), und die Farbbildung bei 492 nm gemessen (Saphire Plate reader).

### 3.b.2) Parameter für Organdysfunktion

Es werden verschiedene Parameter bestimmt, aufgrund derer Rückschlüsse auf die Funktionseinschränkung verschiedener innerer Organe durch die LPS-Gabe gezogen werden können, und der therapeutische Effekt von Prüfsubstanzen abgeschätzt werden kann. Citratblut oder ggf. Lithium-Heparin-Blut wird zentrifugiert, und die Parameter aus dem Plasma bestimmt. Folgende Parameter werden typischerweise erhoben: Kreatinin, Harnstoff, Aspartat-Aminotransferase (AST), Alanin-Aminotransferase (ALT), Gesamt-Bilirubin, Laktatdehydrogenase (LDH), Gesamt-Protein, Gesamt-Albumin und Fibrinogen. Die Werte geben Aufschluss auf die Funktion der Niere, der Leber, des Kreislaufes und der Gefäße.

### 3.b.3) Parameter für Entzündung

Das Ausmaß der durch Endotoxin ausgelösten Entzündungsreaktion lässt sich aus dem Anstieg von Entzündungsmediatoren im Plasma nachweisen, z.B. Interleukine (1, 6, 8 und 10), Tumornekrosefaktor alpha oder Monocyte Chemoattractant Protein-1. Hierzu können ELISAs oder das Luminex-System verwendet werden.

### 3.c) Antitumor Wirksamkeit

Die erfindungsgemäßen Verbindungen können in Modellen für Krebs getestet werden, z.B. im humanen Brustkrebs-Modell in immundefizienten Mäusen (vergleiche: S. Even-Ram et. al., Nature Medicine, 1988, 4, 909-914).

### 3.d) Antiangiogenetische Wirksamkeit

Die erfindungsgemäßen Verbindungen können in *in vitro* und *in vivo* Modellen für Angiogenese getestet werden (vergleiche: Caunt et al., Journal of Thrombosis and Haemostasis, 2003, 10, 2097-2102; Haralabopoulos et al., Am JPhysiol, 1997, C239-C245; Tsopanoglou et al., JBC, 1999, 274, 23969-23976; Zania et al., JPET, 2006, 318, 246-254).

### 3.e) Blutdruck- und Herzfrequenz-modulierende Wirkung

Die erfindungsgemäßen Verbindungen können in *in vivo* Modellen hinsichtlich Ihrer Wirkung auf den arteriellen Blutdruck und die Herzfrequenz untersucht werden. Hierzu werden Ratten (z.B. Wistar) mit implantierbaren Radiotelemetrieeinheiten instrumentiert, und es wird ein elektronisches Datenaquisitions- und Speicherungs-System (Data Sciences, MN, USA), bestehend aus einem chronisch implantierbaren Transducer/Transmitter-Einheit in Verbindung mit einem Flüssigkeits-gefüllten Katheter, verwendet. Der Transmitter wird in die Peritonealhöhle implantiert und der Sensor-Katheter in der deszendierenden Aorta positioniert. Die erfindungsgemäßen Verbindungen können appliziert werden (z.B. oral oder intravenös). Vor Behandlung wird der mittlere arterielle Blutdruck und die Herzfrequenz der unbehandelten und behandelten Tiere gemessen und sichergestellt, dass diese im Bereich von ca. 131-142 mmHg und 279-321 Schläge/Minute liegen. PAR-1-aktivierendes Peptid (SFLLRN; z.B. Dosen zwischen 0.1 und 5 mg/kg) wird intravenös verabreicht. Der Blutdruck und die Herzfrequenz werden in verschiedenen Zeitintervallen und Zeiträumen mit und ohne PAR-1-aktivierendem Peptid sowie mit und ohne einer der erfindungsgemäßen Verbindungen gemessen (vergleiche: Cicala C et al., The FASEB Journal, 2001, 15, 1433-5; Stasch JP et al., British Journal of Pharmacology 2002, 135, 344-355).

### 4.) Bestimmung der Löslichkeit

### Herstellung der Ausgangslösung (Urlösung):

Mindestens 1.5 mg der Testsubstanz werden in ein Wide Mouth 10 mm Screw V-Vial (Fa. Glastechnik Gräfenroda GmbH, Art.-Nr. 8004-WM-H/V15µ) mit passender Schraubkappe und Septum genau eingewogen, mit DMSO zu einer Konzentration von 50 mg/ml versetzt und 30 Minuten mittels eines Vortexers geschüttelt.

### Herstellung der Kalibrierlösungen:

Die notwendigen Pipettierschritte erfolgen in 1.2 ml 96er Deep Well Plate (DWP) mittels eines Liquid-Handling-Roboters. Als Lösemittel wird ein Gemisch aus Acetonitril/Wasser 8:2 verwendet.

*Herstellung der Ausgangslösung für Kalibrierlösungen (Stammlösung):* 10 µl der Urlösung werden mit 833 µl des Lösemittelgemisch versetzt (Konzentration = 600 µg/ml) und homogenisiert. Es werden von jeder Testsubstanz 1:100 Verdünnungen in separaten DWP's hergestellt und wiederum homogenisiert.

*Kalibrierlösung 5 (600 nglml):* 30 µl der Stammlösung werden mit 270 µl Lösemittelgemisch versetzt und homogenisiert.

*Kalibrierlösung 4 (60 nglml):* 30 µl der Kalibrierlösung 5 werden mit 270 µl Lösemittelgemisch versetzt und homogenisiert.

*Kalibrierlösung 3 (12 nglml):* 100 µl der Kalibrierlösung 4 werden mit 400 µl Lösemittelgemisch versetzt und homogenisiert.

*Kalibrierlösung 2 (1.2 nglml):* 30 µl der Kalibrierlösung 3 werden mit 270 µl Lösemittelgemisch versetzt und homogenisiert.

*Kalibrierlösung 1 (0.6 nglml):* 150 µl der Kalibrierlösung 2 werden mit 150 µl Lösemittelgemisch versetzt und homogenisiert.

### Herstellung der Probenlösungen:

Die notwendigen Pipettierschritte erfolgen in 1.2 ml 96er DWP mittels eines Liquid-Handling-Roboters. 10.1 µl der Stammlösung werden mit 1000 µl PBS-Puffer pH 6.5 versetzt. (PBS-Puffer pH 6.5: 61.86 g Natriumchlorid, 39.54 g Natriumdihydrogenphosphat und 83.35 g 1 N Natronlauge werden in einen 1 Liter-Messkolben eingewogen, mit Wasser aufgefüllt und ca. 1 Stunde gerührt. Von dieser Lösung werden 500 ml in einen 5 Liter-Messkolben gegeben und mit Wasser aufgefüllt. Es wird mit 1 N Natronlauge auf pH 6.5 einstellen.)

### Durchführung:

Die notwendigen Pipettierschritte erfolgen in 1.2 ml 96er DWP mittels eines Liquid-Handling-Roboters. Die so hergestellten Probenlösungen werden 24 Stunden bei 1400 rpm mittels eines temperierbaren Schüttlers bei 20°C geschüttelt. Von diesen Lösungen werden jeweils 180 µl abgenommen und in Beckman Polyallomer Centrifuge Tubes überführt. Diese Lösungen werden 1 Stunde mit ca. 223.000 x g zentrifugiert. Von jeder Probenlösung werden 100 µl des Überstandes abgenommen und 1:10 und 1:1000 mit PBS-Puffer 6.5 verdünnt.

### Analytik:

Die Proben werden mittels HPLC/MS-MS analysiert. Quantifiziert wird über eine Fünf-Punkt-Kalibrationskurve der Testverbindung. Die Löslichkeit wird in mg/l ausgedrückt. Analysensequenz: 1) Blank (Lösemittelgemisch); 2) Kalibrierlösung 0.6 ng/ml; 3) Kalibrierlösung 1.2 ng/ml; 4) Kalibrierlösung 12 ng/ml; 5) Kalibrierlösung 60 ng/ml; 6) Kalibrierlösung 600 ng/ml; 7) Blank (Lösemittelgemisch); 8) Probenlösung 1:1000; 9) Probenlösung 1:10.

### HPLC/MS-MSMethode:

HPLC: Agilent 1100, quat. Pumpe (G1311A), Autosampler CTC HTS PAL, Degaser (G1322A) und Säulenthermostat (G1316A); Säule: Oasis HLB 20 mm x 2.1 mm, 25 µ; Temperatur: 40°C; Eluent A: Wasser + 0.5 ml Ameisensäure/l; Eluent B: Acetonitril + 0.5 ml Ameisensäure/l; Flussrate: 2.5 ml/min; Stoptime 1.5 min; Gradient: 0 min 95% A, 5% B; Rampe: 0-0.5 min 5% A, 95% B; 0.5-0.84 min 5% A, 95% B; Rampe: 0.84-0.85 min 95% A, 5% B; 0.85-1.5 min 95% A, 5% B.

MS/MS: WATERS Quattro Micro Tandem MS/MS; Z-Spray API-Interface; HPLC-MS-Eingangssplitter 1:20; Messung im ESI-Mode.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 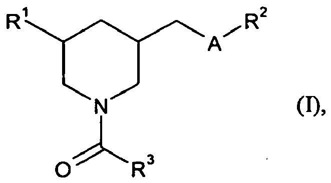 in welcher
A für ein Sauerstoffatom, ein Schwefelatom, -NR⁴-, -S(=O)- oder -S(=O)₂- steht,
wobei
R⁴ für Wasserstoff oder C₁-C₃-Alkyl steht,
oder
R² und R⁴ bilden zusammen mit dem Stickstoffatom an das sie gebunden sind einen 5- oder 6-gliedrigen Heterocyclus,
worin der Heterocyclus substituiert sein kann mit 1 bis 3 Substituenten, unabhängig voneinander ausgewählt aus der Gruppe bestehend aus Halogen, Cyano, Hydroxy, Amino, C₁-C₄-Alkyl, C₁-C₄-Alkoxy und C₁-C₄-Alkylamino,
oder
A für eine Gruppe der Formel 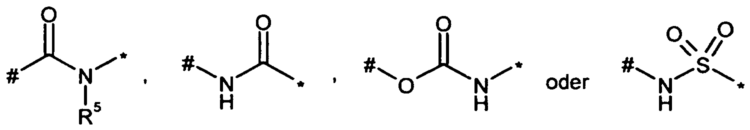 steht,
wobei
# die Anknüpfstelle an den Piperidinring ist,
* die Anknüpfstelle an R² ist,
und
R⁵ für Wasserstoff oder C₁-C₃-Alkyl steht,
R¹ für Phenyl steht,
wobei Phenyl substituiert sein kann mit 1 bis 3 Substituenten, unabhängig voneinander ausgewählt aus der Gruppe bestehend aus Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Methylsulfonyl, C₁-C₄-Alkyl, C₁-C₄-Alkoxy und C₁-C₄-Alkoxycarbonyl,
R² für C₁-C₆-Alkyl, C₃-C₆-Cycloalkyl, 4- bis 6-gliedriges Heterocyclyl, Phenyl oder 5- oder 6-gliedriges Heteroaryl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₁-C₆-Alkylamino und Phenyl,
worin Phenyl substituiert sein kann mit 1 bis 3 Substituenten, unabhängig voneinander ausgewählt aus der Gruppe bestehend aus Halogen und Trifluormethyl,
und
wobei C₁-C₆-Alkyl substituiert sein kann mit einem Substituenten ausgewählt aus der Gruppe bestehend aus C₁-C₄-Alkoxy, C₁-C₄-Alkylsulfonyl, C₃-C₆-Cycloalkyl und Phenyl,
worin Cycloalkyl und Phenyl substituiert sein können mit 1 bis 3 Substituenten, unabhängig voneinander ausgewählt aus der Gruppe bestehend aus Halogen, Cyano, Monofluormethyl, Difluormethyl, Trifluormethyl, Monofluormethoxy, Difluormethoxy, Trifluormethoxy, C₁-C₄-Alkyl und C₁-C₄-Alkoxy,
R³ für C₁-C₆-Alkyl, C₁-C₆-Alkoxy, C₁-C₆-Alkylamino, C₃-C₇-Cycloalkyl, 4- bis 7-gliedriges Heterocyclyl, Phenyl, 5- oder 6-gliedriges Heteroaryl, C₃-C₇-Cycloalkyloxy, C₃-C₇-Cycloalkylamino, 4- bis 7-gliedriges Heterocyclylamino, Phenylamino oder 5- oder 6-gliedriges Heteroarylamino steht,
wobei Alkyl, C₂-C₆-Alkoxy und Alkylamino substituiert sein können mit einem Substituenten ausgewählt aus der Gruppe bestehend aus Halogen, Hydroxy, Amino, Cyano, C₁-C₄-Alkoxy, C₁-C₄-Alkoxycarbonyl, C₃-C₇-Cycloalkyl, 4- bis 6-gliedriges Heterocyclyl, Phenyl und 5- oder 6-gliedriges Heteroaryl,
und
wobei Cycloalkyl, Heterocyclyl, Phenyl, Heteroaryl, Cycloalkyloxy, Cycloalkylamino, Heterocyclylamino, Phenylamino und Heteroarylamino substituiert sein können mit 1 bis 3 Substituenten, unabhängig voneinander ausgewählt aus der Gruppe bestehend aus Halogen, Cyano, Oxo, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxycarbonyl, Aminocarbonyl, C₁-C₄-Alkyl, C₁-C₄-Alkoxy, C₁-C₆-Alkylamino, C₁-C₄-Alkoxycarbonyl, C₁-C₄-Alkylaminocarbonyl und Cyclopropyl,
worin Alkyl substituiert sein kann mit einem Substituenten Hydroxy, oder eines ihrer Salze, ihrer Solvate oder der Solvate ihrer Salze.

2. Verbindung nach Anspruch 1, **dadurch gekennzeichnet, dass**
A für ein Sauerstoffatom, ein Schwefelatom, -NR⁴-, -S(=O)- oder -S(=O)₂- steht,
wobei
R⁴ für Wasserstoff, Methyl oder Ethyl steht,
oder
R² und R⁴ bilden zusammen mit dem Stickstoffatom an das sie gebunden sind ein 3-Hydroxypyrrolidin-1-yl,
oder
A für eine Gruppe der Formel 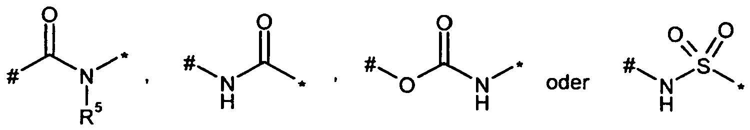 steht,
wobei
# die Anknüpfstelle an den Piperidinring ist,
* die Anknüpfstelle an R² ist,
und
R⁵ für Wasserstoff, Methyl oder Ethyl steht,
R¹ für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl und Methoxy,
R² für Methyl, Ethyl, Propyl, Isopropyl, 2-Methylprop-1-yl, tert-Butyl, Cyclopropyl, Cyclobutyl, Cyclopentyl, Cyclohexyl, Tetrahydro-2H-pyran-4-yl oder Phenyl steht,
wobei Phenyl substituiert sein kann mit 1 bis 2 Substituenten, unabhängig voneinander ausgewählt aus der Gruppe bestehend aus Halogen, Cyano, Methyl, Ethyl, Methoxy und Ethoxy,
und
wobei Methyl und Ethyl substituiert sein können mit einem Substituenten ausgewählt aus der Gruppe bestehend aus Methoxy, Methylsulfonyl und Phenyl,
R³ für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
oder eines ihrer Salze, ihrer Solvate oder der Solvate ihrer Salze.

3. Verbindung nach Anspruch 1 oder 2, **dadurch gekennzeichnet, dass**
A für eine Gruppe der Formel 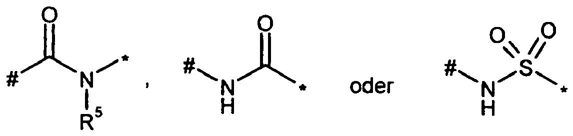 steht,
wobei
# die Anknüpfstelle an den Piperidinring ist,
* die Anknüpfstelle an R² ist,
und
R⁵ für Wasserstoff, Methyl oder Ethyl steht,
R¹ für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl und Methoxy,
R² für Methyl, Ethyl, Propyl, Isopropyl, 2-Methylprop-1-yl, tert-Butyl, Cyclopropyl, Cyclobutyl, Cyclopentyl, Cyclohexyl, Tetrahydro-2H-pyran-4-yl oder Phenyl steht,
wobei Phenyl substituiert sein kann mit 1 bis 2 Substituenten, unabhängig voneinander ausgewählt aus der Gruppe bestehend aus Halogen, Cyano, Methyl, Ethyl, Methoxy und Ethoxy,
und
wobei Methyl und Ethyl substituiert sein können mit einem Substituenten ausgewählt aus der Gruppe bestehend aus Methoxy, Methylsulfonyl und Phenyl,
R³ für Morpholin-4-yl, 1,1-Dioxidothiomorpholin-4-yl, 3-Hydroxyazetidiny-1-yl, 3-Hydroxypyrrolidin-1-yl, 4-Cyanopiperidin-1-yl oder 4-Hydroxypiperidin-1-yl steht,
oder eines ihrer Salze, ihrer Solvate oder der Solvate ihrer Salze.

4. Verbindung nach einem der Ansprüche 1 bis 3, **dadurch gekennzeichnet, dass** die Substituenten -R¹ und -CH₂-A-R² in cis-Position zueinander stehen.

5. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder
[A] eine Verbindung der Formel 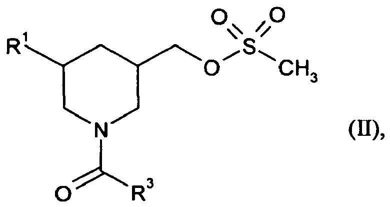 in welcher
R¹ und R³ die in Anspruch 1 angegebene Bedeutung aufweisen,
mit einer Verbindung der Formel
H—A^{—}R² (III),
in welcher
R² die in Anspruch 1 angegebene Bedeutung aufweist,
A für ein Sauerstoffatom, ein Schwefelatom oder -NR⁴- steht, und
R⁴ die in Anspruch 1 angegebene Bedeutung aufweist,
zu einer Verbindung der Formel 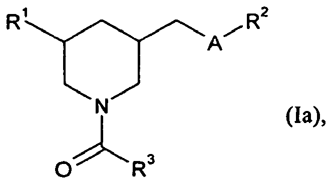 in welcher
A für ein Sauerstoffatom, ein Schwefelatom oder -NR⁴- steht, und
R¹, R², R³ und R⁴ die in Anspruch 1 angegebene Bedeutung aufweisen,
umgesetzt wird
oder
[B] eine Verbindung der Formel (I) in welcher A für ein Schwefelatom steht und R¹, R² und R³ die in Anspruch 1 angegebene Bedeutung aufweisen, mit einem Oxidationsmittel zu einer Verbindung der Formel 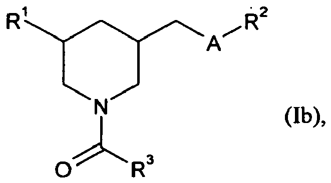 in welcher
A für -S(=O)- oder -S(=O)₂- steht, und
R¹, R² und R³ die in Anspruch 1 angegebene Bedeutung aufweisen,
umgesetzt wird
oder
[C] eine Verbindung der Formel 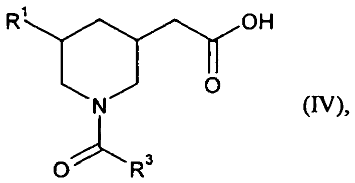 in welcher
R¹ und R³ die in Anspruch 1 angegebene Bedeutung aufweisen,
mit einer Verbindung der Formel 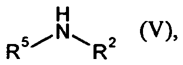 in welcher
R² und R⁵ die in Anspruch 1 angegebene Bedeutung aufweisen,
zu einer Verbindung der Formel 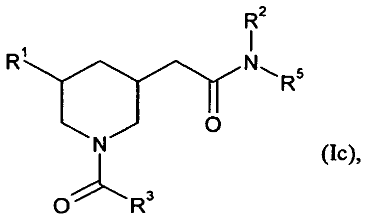 in welcher
R¹, R², R³ und R⁵ die in Anspruch 1 angegebene Bedeutung aufweisen,
umgesetzt wird
oder
[D] eine Verbindung der Formel 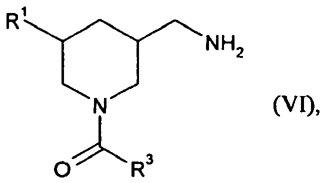 in welcher
R¹ und R³ die in Anspruch 1 angegebene Bedeutung aufweisen,
mit einer Verbindung der Formel 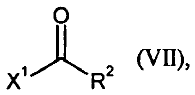 in welcher
R² die in Anspruch 1 angegebene Bedeutung aufweist, und
X¹ für Halogen, bevorzugt Brom oder Chlor, oder Hydroxy steht,
zu einer Verbindung der Formel 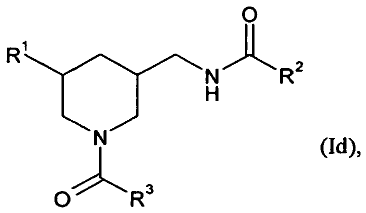 in welcher
R¹, R² und R³ die in Anspruch 1 angegebene Bedeutung aufweisen,
umgesetzt wird
oder
[E] eine Verbindung der Formel (VI) mit einer Verbindung der Formel 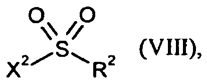 in welcher
R² die in Anspruch 1 angegebene Bedeutung aufweist, und
X² für Halogen, bevorzugt Brom oder Chlor, steht,
zu einer Verbindung der Formel 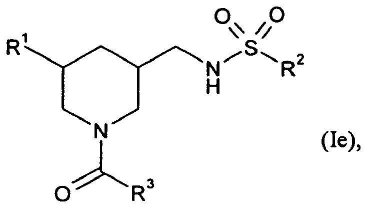 in welcher
R¹, R² und R³ die in Anspruch 1 angegebene Bedeutung aufweisen,
umgesetzt wird
oder
[F] eine Verbindung der Formel 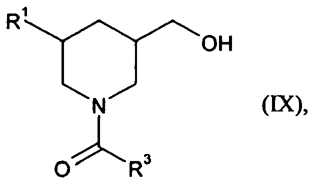 in welcher
R¹ und R³ die in Anspruch 1 angegebene Bedeutung aufweisen,
mit einer Verbindung der Formel 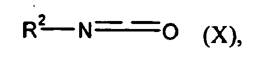 in welcher
R² die in Anspruch 1 angegebene Bedeutung aufweist,
zu einer Verbindung der Formel 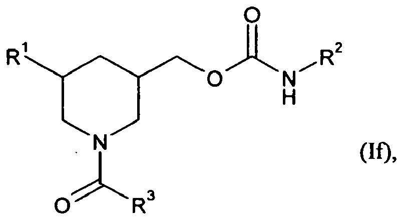 in welcher
R¹, R² und R³ die in Anspruch 1 angegebene Bedeutung aufweisen,
umgesetzt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von Krankheiten.

8. Verwendung einer Verbindung nach einem der Ansprüche 1 bis 4 zur Herstellung eines Arzneimittels zur Behandlung und/oder Prophylaxe von Herz-Kreislauf-Erkrankungen, thromboembolischen Erkrankungen und/oder von Tumorerkrankungen.

9. Verwendung einer Verbindung nach einem der Ansprüche 1 bis 4 zur Verhinderung der Blutkoagulation *in vitro.*

10. Arzneimittel enthaltend eine Verbindung nach einem der Ansprüche 1 bis 4 in Kombination mit einem inerten, nichttoxischen, pharmazeutisch geeigneten Hilfsstoff.

11. Arzneimittel enthaltend eine Verbindung nach einem der Ansprüche 1 bis 4 in Kombination mit einem weiteren Wirkstoff.

12. Arzneimittel nach Anspruch 10 oder 11 zur Behandlung und/oder Prophylaxe von Herz-Kreislauf-Erkrankungen, thromboembolischen Erkrankungen und/oder von Tumorerkrankungen.

13. Verfahren zur Verhinderung der Blutkoagulation *in vitro,* **dadurch gekennzeichnet, dass** eine antikoagulatorisch wirksame Menge einer Verbindung nach einem der Ansprüche 1 bis 4 zugegeben wird.

## Claims

1. Compound of the formula 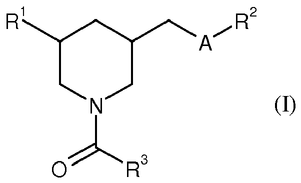 in which
A is an oxygen atom, a sulphur atom, -NR⁴-, -S(O=)- or -S(=O)₂-,
where
R⁴ is hydrogen or C₁-C₃-alkyl,
or
R² and R⁴ together with the nitrogen atom to which they are bonded form a 5- or 6-membered heterocycle,
in which the heterocycle may be substituted by 1 to 3 substituents selected independently from the group consisting of halogen, cyano, hydroxyl, amino, C₁-C₄-alkyl, C₁-C₄-alkoxy and C₁-C₄-alkylamino,
or
A is a group of the formula 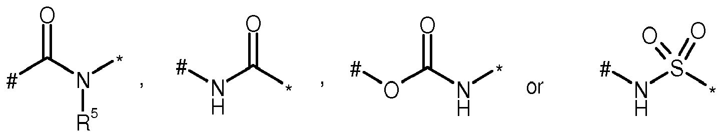 where
# is the attachment site to the piperidine ring,
* is the attachment site of R²,
and
R⁵ is hydrogen or C₁-C₃-alkyl,
R¹ is phenyl,
where phenyl may be substituted by 1 to 3 substituents selected independently from the group consisting of monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, methylsulphonyl, C₁-C₄-alkyl, C₁-C₄-alkoxy and C₁-C₄-alkoxycarbonyl,
R² is C₁-C₆-alkyl, C₃-C₆-cycloalkyl, 4- to 6-membered heterocyclyl, phenyl or 5- or 6-membered heteroaryl,
where cycloalkyl, heterocyclyl, phenyl and heteroaryl may be substituted by 1 to 3 substituents selected independently from the group consisting of halogen, cyano, hydroxyl, amino, monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, C₁-C₄-alkyl, C₁-C₄-alkoxy, C₁-C₆-alkylamino and phenyl,
in which phenyl may be substituted by 1 to 3 substituents selected independently from the group consisting of halogen and trifluoromethyl,
and
where C₁-C₆-alkyl may be substituted by one substituent selected from the group consisting of C₁-C₄-alkoxy, C₁-C₄-alkylsulphonyl, C₃-C₆-cycloalkyl and phenyl, in which cycloalkyl and phenyl may be substituted by 1 to 3 substituents selected independently from the group consisting of halogen, cyano, monofluoromethyl, difluoromethyl, trifluoromethyl, monofluoromethoxy, difluoromethoxy, trifluoromethoxy, C₁-C₄-alkyl and C₁-C₄-alkoxy,
R³ is C₁-C₆-alkyl, C₁-C₆-alkoxy, C₁-C₆-alkylamino, C₃-C₇-cycloalkyl, 4- to 7-membered heterocyclyl, phenyl, 5- or 6-membered heteroaryl, C₃-C₇-cycloalkyloxy, C₃-C₇-cycloalkylamino, 4- to 7-membered heterocyclylamino, phenylamino or 5- or 6-membered heteroarylamino,
where alkyl, C₂-C₆-alkoxy and alkylamino may be substituted by one substituent selected from the group consisting of halogen, hydroxyl, amino, cyano, C₁-C₄-alkoxy, C₁-C₄-alkoxycarbonyl, C₃-C₇-cycloalkyl, 4- to 6-membered heterocyclyl, phenyl and 5- or 6- membered heteroaryl,
and
where cycloalkyl, heterocyclyl, phenyl, heteroaryl, cycloalkyloxy, cycloalkylamino, heterocyclylamino, phenylamino and heteroarylamino may be substituted by 1 to 3 substituents selected independently from the group consisting of halogen, cyano, oxo, hydroxyl, amino, monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, hydroxycarbonyl, aminocarbonyl, C₁-C₄-alkyl, C₁-C₄-alkoxy, C₁-C₆-alkylamino, C₁-C₄-alkoxycarbonyl, C₁-C₄-alkylaminocarbonyl and cyclopropyl,
in which alkyl may be substituted by one hydroxyl substituent,
or one of its salts, its solvates or the solvates of its salts.

2. Compound according to Claim 1, **characterized in that**
A is an oxygen atom, a sulphur atom, -NR⁴-, -S(O=)- or -S(=O)₂-,
where
R⁴ is hydrogen, methyl or ethyl,
or
R² and R⁴ together with the nitrogen atom to which they are bonded form a 3-hydroxypyrrolidin-1-yl,
or
A is a group of the formula 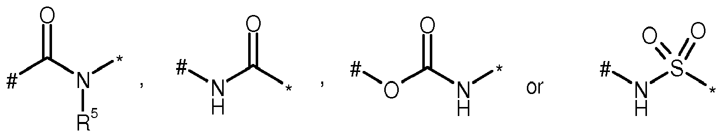 where
# is the attachment site to the piperidine ring,
* is the attachment site of R²,
and
R⁵ is hydrogen, methyl or ethyl,
R¹ is phenyl,
where phenyl is substituted by 1 to 2 substituents selected independently from the group consisting of trifluoromethyl, trifluoromethoxy, methyl, ethyl and methoxy,
R² is methyl, ethyl, propyl, isopropyl, 2-methylprop-1-yl, tert-butyl, cyclopropyl, cyclobutyl, cyclopentyl, cyclohexyl, tetrahydro-2H-pyran-4-yl or phenyl,
where phenyl may be substituted by 1 to 2 substituents selected independently from the group consisting of halogen, cyano, methyl, ethyl, methoxy and ethoxy,
and
where methyl and ethyl may be substituted by one substituent selected from the group consisting of methoxy, methylsulphonyl and phenyl,
R³ is morpholin-4-yl, 1,1-dioxidothiomorpholin-4-yl, 3-hydroxyazetidin-1-yl, 3-hydroxypyrrolidin-1-yl, 4-cyanopiperidin-2-yl or 4-hydroxypiperidin-1-yl,
or one of its salts, its solvates or the solvates of its salts.

3. Compound according to Claim 1 or 2, **characterized in that**
A is a group of the formula 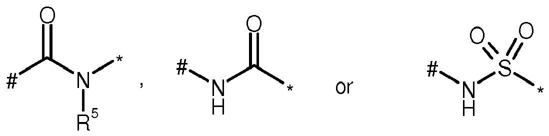 where
# is the attachment site to the piperidine ring,
* is the attachment site to R²,
and
R⁵ is hydrogen, methyl or ethyl,
R¹ is phenyl,
where phenyl is substituted by 1 to 2 substituents selected independently from the group consisting of trifluoromethyl, trifluoromethoxy, methyl, ethyl and methoxy,
R² is methyl, ethyl, propyl, isopropyl, 2-methylprop-1-yl, tert-butyl, cyclopropyl, cyclobutyl, cyclopentyl, cyclohexyl, tetrahydro-2H-pyran-4-yl or phenyl,
where phenyl may be substituted by 1 to 2 substituents selected independently from the group consisting of halogen, cyano, methyl, ethyl, methoxy and ethoxy,
and
where methyl and ethyl may be substituted by one substituent selected from the group consisting of methoxy, methylsulphonyl and phenyl,
R³ is morpholin-4-yl, 1,1-dioxidothiomorpholin-4-yl, 3-hydroxyazetidin-1-yl, 3-hydroxypyrrolidin-1-yl, 4-cyanopiperidin-1-yl or 4-hydroxypiperidin-1-yl,
or one of its salts, its solvates or the solvates of its salts.

4. Compound according to any of Claims 1 to 3, **characterized in that** the -R¹ and -CH₂-A-R² substituents are in cis-positions to one another.

5. Process for preparing a compound of the formula (I) or one of its salts, its solvates or the solvates of its salts according to Claim 1, **characterized in that** either
[A] a compound of the formula 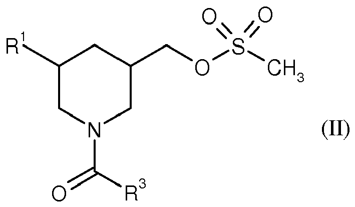 in which
R¹ and R³ are each as defined in Claim 1
is reacted with a compound of the formula
H—A—R² (III),
in which
R² is as defined in Claim 1
A is an oxygen atom, an sulphur atom or -NR⁴-, and
R⁴ is as defined in Claim 1
to give a compound of the formula 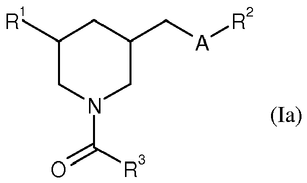 in which
A is an oxygen atom, a sulphur atom or -NR⁴-, and
R¹, R², R³ and R⁴ are each as defined in Claim 1,
or
[B] a compound of the formula (I) in which A is a sulphur atom and R¹, R² and R³ are each as defined in Claim 1 is reacted with an oxidizing agent
to give a compound of the formula 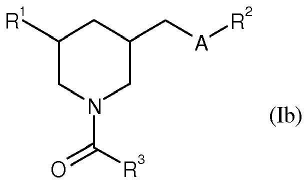 in which
A is -S(=O)- or -S(=O)₂-, and
R¹, R² and R³ are each as defined in Claim 1,
or
[C] a compound of the formula 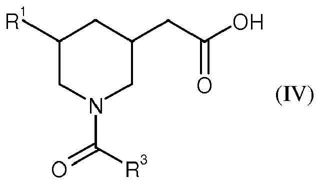 in which
R¹ and R³ are each as defined in Claim 1,
is reacted with a compound of the formula 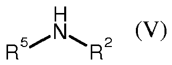 in which
R² and R⁵ are each as defined in Claim 1 to give a compound of the formula 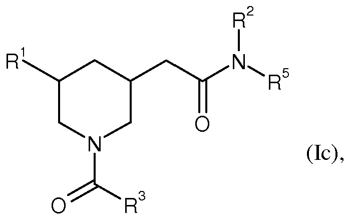 in which
R¹, R², R³ and R⁵ are each as defined in Claim 1,
or
[D] a compound of the formula 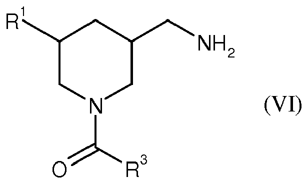 in which
R¹ and R³ are each as defined in Claim 1
is reacted with a compound of the formula 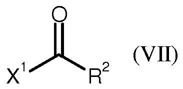 in which
R² is as defined in Claim 1 and
X¹ is halogen, preferably bromine or chlorine, or hydroxyl to give a compound of the formula 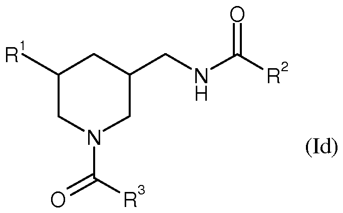 in which
R¹, R² and R³ are each as defined in Claim 1,
or
[E] a compound of the formula (VI) is reacted with a compound of the formula 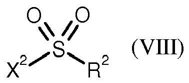 in which
R² is as defined in Claim 1 and
X² is halogen, preferably bromine or chlorine,
to give a compound of the formula 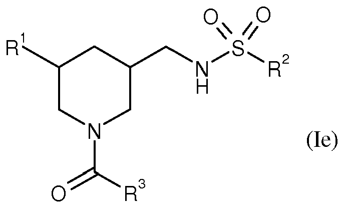 in which
R¹, R² and R³ are each as defined in Claim 1,
or
[F] a compound of the formula 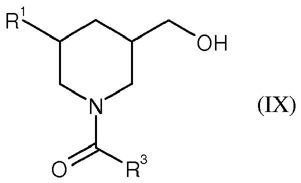 in which
R¹ and R³ are each as defined in Claim 1
is reacted with a compound of the formula 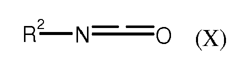 in which
R² is as defined in Claim 1,
to give a compound of the formula 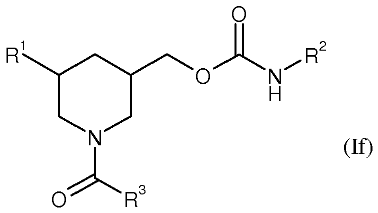 in which
R¹, R² and R³ are as defined in Claim 1.

6. Compound according to any of Claims 1 to 4 for treatment and/or prophylaxis of diseases.

7. Use of a compound according to any of Claims 1 to 4 for production of a medicament for treatment and/or prophylaxis of diseases.

8. Use of a compound according to any of Claims 1 to 4 for production of a medicament for treatment and/or prophylaxis of cardiovascular disorders, thromboembolic disorders and/or tumour disorders.

9. Use of a compound according to any of Claims 1 to 4 for prevention of blood coagulation *in vitro.*

10. Medicament comprising a compound according to any of Claims 1 to 4 in combination with an inert, non-toxic, pharmaceutically acceptable excipient.

11. Medicament comprising a compound according to any of Claims 1 to 4 in combination with a further active ingredient.

12. Medicament according to Claim 10 or 11 for treatment and/or prophylaxis of cardiovascular disorders, thromboembolic disorders and/or tumour disorders.

13. Method for prevention of blood coagulation *in vitro,* **characterized in that** an anticoagulatory amount of a compound according to any of Claims 1 to 4 is added.

## Revendications

1. Composé de formule 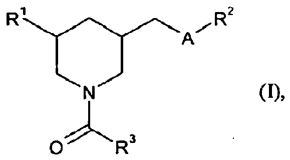 dans laquelle
A représente un atome d'oxygène, un atome de soufre, -NR⁴-, -S (=O) - ou -S (=O) ₂-,
R⁴ représentant hydrogène ou C₁-C₃-alkyle, ou
R² et R⁴ formant ensemble avec l'atome d'azote auquel ils sont liés un hétérocycle de 5 ou 6 chaînons,
l'hétérocycle pouvant être substitué par 1 à 3 substituants, choisis indépendamment les uns des autres dans le groupe constitué par halogène, cyano, hydroxy, amino, C₁-C₄-alkyle, C₁-C₄-alcoxy et C₁-C₄-alkylamino,
ou
A représente un groupe de formule 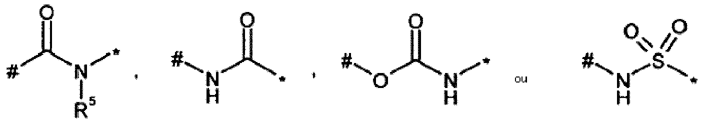 où
# est le site de liaison au cycle pipéridine,
* est le site de liaison à R², et
R⁵ représente hydrogène ou C₁-C₃-alkyle,
R¹ représente phényle,
phényle pouvant être substitué par 1 à 3 substituants, choisis indépendamment les uns des autres dans le groupe constitué par monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, méthylsulfonyle, C₁-C₄-alkyle, C₁-C₄-alcoxy et C₁-C₄-alcoxycarbonyle,
R² représente C₁-C₆-alkyle, C₃-C₆-cycloalkyle, hétérocyclyle de 4 à 6 chaînons, phényle ou hétéroaryle de 5 ou 6 chaînons, cycloalkyle, hétérocyclyle, phényle et hétéroaryle pouvant être substitués par 1 à 3 substituants, choisis indépendamment les uns des autres dans le groupe constitué par halogène, cyano, hydroxy, amino, monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, C₁-C₄-alkyle, C₁-C₄-alcoxy, C₁-C₆-alkylamino et phényle,
phényle pouvant être substitué par 1 à 3 substituants, choisis indépendamment les uns des autres dans le groupe constitué par halogène et trifluorométhyle,
et
C₁-C₆-alkyle pouvant être substitué par un substituant choisi dans le groupe constitué par C₁-C₄-alcoxy, C₁-C₄-alkylsulfonyle, C₃-C₆-cycloalkyle et phényle,
cycloalkyle et phényle pouvant être substitués par 1 à 3 substituants, choisis indépendamment les uns des autres dans le groupe constitué par halogène, cyano, monofluorométhyle, difluorométhyle, trifluorométhyle, monofluorométhoxy, difluorométhoxy, trifluorométhoxy, C₁-C₄-alkyle et C₁-C₄-alcoxy,
R³ représente C₁-C₆-alkyle, C₁-C₆-alcoxy, C₁-C₆-alkylamino, C₃-C₇-cycloalkyle, hétérocyclyle de 4 à 7 chaînons, phényle, hétéroaryle de 5 ou 6 chaînons, C₃-C₇-cycloalkyloxy, C₃-C₇-cycloalkylamino, hétérocyclylamino de 4 à 7 chaînons, phénylamino ou hétéroarylamino de 5 ou 6 chaînons,
alkyle, C₂-C₆-alcoxy et alkylamino pouvant être substitués par un substituant choisi dans le groupe constitué par halogène, hydroxy, amino, cyano, C₁-C₄-alcoxy, C₁-C₄-alcoxycarbonyle, C₃-C₇-cycloalkyle, hétérocyclyle de 4 à 6 chaînons, phényle et hétéroaryle de 5 ou 6 chaînons, et cycloalkyle, hétérocyclyle, phényle, hétéroaryle, cycloalkyloxy, cycloalkylamino, hétérocyclylamino, phénylamino et hétéroarylamino pouvant être substitués par 1 à 3 substituants, choisis indépendamment les uns des autres dans le groupe constitué par halogène, cyano, oxo, hydroxy, amino, monofluorométhyle, difluorométhyle, trifluorométhyle, monofluorométhoxy, difluorométhoxy, trifluorométhoxy, monofluorométhylsulfanyle,
difluorométhylsulfanyle, trifluorométhylsulfanyle, hydroxycarbonyle, aminocarbonyle, C₁-C₄-alkyle, C₁-C₄-alcoxy, C₁-C₆-alkylamino, C₁-C₄-alcoxycarbonyle, C₁-C₄-alkylaminocarbonyle et cyclopropyle,
alkyle pouvant être substitué par un substituant hydroxy,
ou un de ses sels, de ses solvates ou des solvates de ses sels.

2. Composé selon la revendication 1, **caractérisé en ce que**
A représente un atome d'oxygène, un atome de soufre, -NR⁴-, -S (=O) - ou -S (=O)₂-, où
R⁴ représente hydrogène, méthyle ou éthyle, ou
R² et R⁴ forment ensemble avec l'atome d'azote auquel ils sont liés 3-hydroxypyrrolidin-1-yle,
ou
A représente un groupe de formule 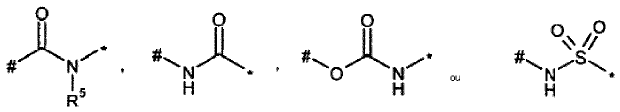 où
# est le site de liaison au cycle pipéridine,
* est le site de liaison à R², et
R⁵ représente hydrogène, méthyle ou éthyle,
R¹ représente phényle, phényle étant substitué par 1 à 2 substituants, choisis indépendamment l'un de l'autre dans le groupe constitué par trifluorométhyle, trifluorométhoxy, méthyle, éthyle et méthoxy,
R² représente méthyle, éthyle, propyle, isopropyle, 2-méthylprop-1-yle, tert-butyle, cyclopropyle, cyclobutyle, cyclopentyle, cyclohexyle, tétrahydro-2H-pyran-4-yle ou phényle, phényle pouvant être substitué par 1 à 2 substituants, choisis indépendamment l'un de l'autre dans le groupe constitué par halogène, cyano, méthyle, éthyle, méthoxy et éthoxy, et méthyle et éthyle pouvant être substitués par un substituant choisi dans le groupe constitué par méthoxy, méthylsulfonyle et phényle,
R³ représente morpholin-4-yle, 1,1-dioxydothiomorpholin-4-yle, 3-hydroxyazétidin-1-yle, 3-hydroxypyrrolidin-1-yle, 4-cyanopipéridin-1-yle ou 4-hydroxypipéridin-1-yle,
ou un de ses sels, de ses solvates ou des solvates de ses sels.

3. Composé selon la revendication 1 ou 2, **caractérisé en ce que**
A représente un groupe de formule 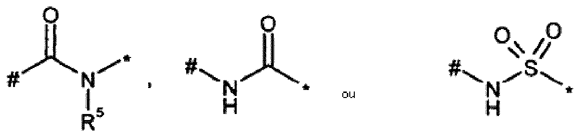 où
# est le site de liaison au cycle pipéridine,
* est le site de liaison à R², et
R⁵ représente hydrogène, méthyle ou éthyle,
R¹ représente phényle, phényle étant substitué par 1 à 2 substituants, choisis indépendamment l'un de l'autre dans le groupe constitué par trifluorométhyle, trifluorométhoxy, méthyle, éthyle et méthoxy,
R² représente méthyle, éthyle, propyle, isopropyle, 2-méthylprop-1-yle, tert-butyle, cyclopropyle, cyclobutyle, cyclopentyle, cyclohexyle, tétrahydro-2H-pyran-4-yle ou phényle, phényle pouvant être substitué par 1 à 2 substituants, choisis indépendamment l'un de l'autre dans le groupe constitué par halogène, cyano, méthyle, éthyle, méthoxy et éthoxy, et méthyle et éthyle pouvant être substitués par un substituant choisi dans le groupe constitué par méthoxy, méthylsulfonyle et phényle,
R³ représente morpholin-4-yle, 1,1-dioxydothiomorpholin-4-yle, 3-hydroxyazétidin-1-yle, 3-hydroxypyrrolidin-1-yle, 4-cyanopipéridin-1-yle ou 4-hydroxypipéridin-1-yle,
ou un de ses sels, de ses solvates ou des solvates de ses sels.

4. Composé selon l'une quelconque des revendications 1 à 3, **caractérisé en ce que** les substituants -R¹ et -CH₂-A-R² se trouvent en position cis l'un par rapport à l'autre.

5. Procédé pour la préparation d'un composé de formule (I) ou d'un de ses sels, de ses solvates ou des solvates de ses sels selon la revendication 1, **caractérisé en ce qu'**on transforme soit
[A] un composé de formule 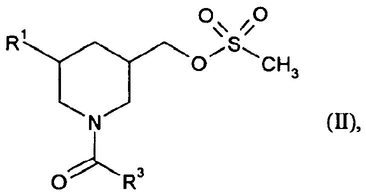 dans laquelle
R¹ et R³ présentent la signification indiquée dans la revendication 1, avec un composé de formule
H—A—R² (III),
dans laquelle
R² présente la signification indiquée dans la revendication 1,
A représente un atome d'oxygène, un atome de soufre ou -NR⁴-, et
R⁴ présente la signification indiquée dans la revendication 1, en un composé de formule 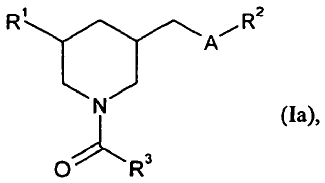
dans laquelle
A représente un atome d'oxygène, un atome de soufre ou -NR⁴-, et
R¹, R², R³ et R⁴ présentent la signification indiquée dans la revendication 1,
soit
[B] un composé de formule (I) dans laquelle A représente un atome de soufre et R¹, R² et R³ présentent la signification indiquée dans la revendication 1, avec un oxydant en un composé de formule 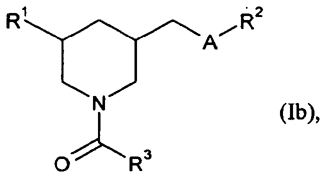 dans laquelle
A représente -S(=O)- ou -S(=O)₂-, et
R¹, R² et R³ présentent la signification indiquée dans la revendication 1, soit
[C] un composé de formule 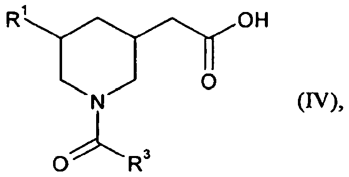 dans laquelle
R¹ et R³ présentent la signification indiquée dans la revendication 1, avec un composé de formule 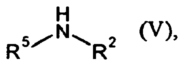 dans laquelle
R² et R⁵ présentent la signification indiquée dans la revendication 1, en un composé de formule 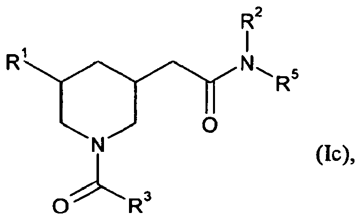
dans laquelle
R¹, R², R³ et R⁵ présentent la signification indiquée dans la revendication 1, soit
[D] un composé de formule 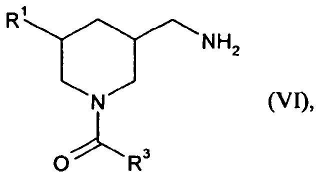 dans laquelle
R¹ et R³ présentent la signification indiquée dans la revendication 1, avec un composé de formule 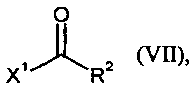 dans laquelle
R² présente la signification indiquée dans la revendication 1, et
X¹ représente halogène, de préférence brome ou chlore, ou hydroxy,
en un composé de formule 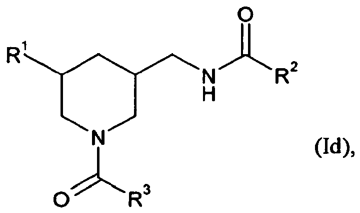 dans laquelle
R¹, R² et R³ présentent la signification indiquée dans la revendication 1, soit
[E] un composé de formule (VI) avec un composé de formule 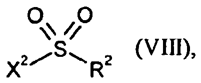 dans laquelle
R² présente la signification indiquée dans la revendication 1, et
X² représente halogène, de préférence brome ou chlore,
en un composé de formule 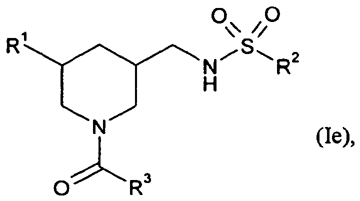 dans laquelle
R¹, R² et R³ présentent la signification indiquée dans la revendication 1, soit
[F] un composé de formule 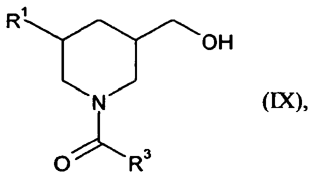 dans laquelle
R¹ et R³ présentent la signification indiquée dans la revendication 1, avec un composé de formule 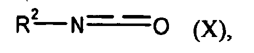
dans laquelle
R² présente la signification indiquée dans la revendication 1, en un composé de formule 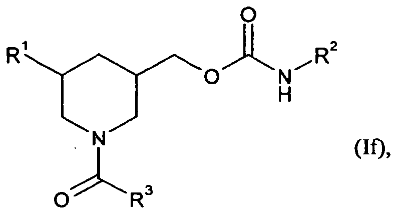
dans laquelle
R¹, R² et R³ présentent la signification indiquée dans la revendication 1.

6. Composé selon l'une quelconque des revendications 1 à 4 destiné au traitement et/ou à la prophylaxie de maladies.

7. Utilisation d'un composé selon l'une quelconque des revendications 1 à 4 pour la préparation d'un médicament destiné au traitement et/ou à la prophylaxie de maladies.

8. Utilisation d'un composé selon l'une quelconque des revendications 1 à 4 pour la préparation d'un médicament destiné au traitement et/ou à la prophylaxie de maladies cardio-vasculaires, thromboemboliques et/ou tumorales.

9. Utilisation d'un composé selon l'une quelconque des revendications 1 à 4, destinée à empêcher la coagulation du sang in vitro.

10. Médicament contenant un composé selon l'une quelconque des revendications 1 à 4 en combinaison avec un adjuvant inerte, non toxique, pharmaceutiquement approprié.

11. Médicament contenant un composé selon l'une quelconque des revendications 1 à 4 en combinaison avec une autre substance active.

12. Médicament selon la revendication 10 ou 11 destiné au traitement et/ou à la prophylaxie de maladies cardio-vasculaires, thromboemboliques et/ou tumorales.

13. Procédé pour empêcher la coagulation du sang in vitro, **caractérisé en ce qu'**on ajoute une quantité active en anticoagulation d'un composé selon l'une quelconque des revendications 1 à 4.
